# EUROPEAN PATENT APPLICATION

(11) **EP 2 018 872 A1**
(43) Date of publication of application: **28.01.2009**
(21) Application number: 07741998.4
(22) Date of filing: 19.04.2007
(51) Int. Cl.: A61K 45/00, A61K 31/216, A61K 31/41, A61K 31/4192, A61K 31/428, A61K 45/06, A61P 9/00, A61P 25/00, A61P 29/00, A61P 31/00, A61P 37/00, A61P 43/00, C07D 249/06, C07D 249/08, C07D 257/04, C07D 275/06

(54) **PHARMACEUTICAL PRODUCT**

(30) Priority: 20.04.2006 JP 2006117270
(71) Applicant: Takeda Pharmaceutical Company Limited, Osaka-shi, Osaka 541-0045 (JP)
(72) Inventor: IIZAWA, Yuji, Osaka-shi, Osaka 532-8686 (JP); II, Masayuki, Deerfield, Illinois 60015 (US); KAWAMOTO, Tomohiro, Osaka-shi, Osaka 532-8686 (JP)
(74) Representative: Kalhammer, Georg
(86) International application number: PCT/JP2007/058562
(87) International publication number: WO 2007/123186

(57) **Abstract**

An agent for suppressing the production of various cytokines (IL-8 and the like) and inflammatory mediators, an agent for suppressing the expression of COX-II and the like, or an inhibitor of various phosphorylation enzymes (ATF2 and the like), which contains a TLR signaling inhibitory substance, preferably a compound represented by the formula (I) or the formula (II) 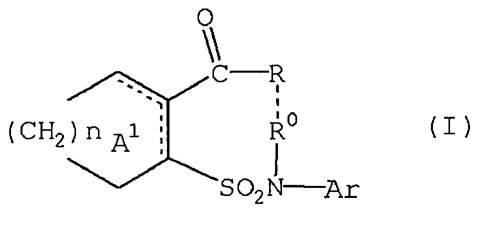 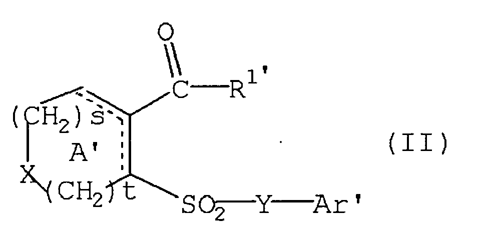 wherein each symbol is as defined in the specification, or a salt thereof or a prodrug thereof.

## Description

### Technical Field

The present invention relates to an agent suppressing production of various cytokines such as IL-10 and the like and inflammatory mediators, and an agent suppressing COX-II and the like, and the like, each of which comprises a TLR signaling inhibitory substance.

### Background of the Invention

Patent reference 1 describes that (i) a compound represented by the formula:

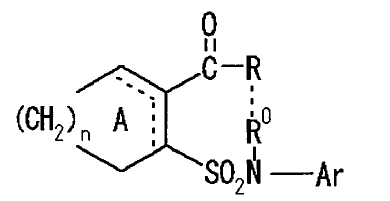

wherein R represents an aliphatic hydrocarbon group optionally having substituent(s), an aromatic hydrocarbon group optionally having substituent(s), a heterocyclic group optionally having substituent(s), a group represented by the formula: -OR¹ (wherein R¹ represents a hydrogen atom or an aliphatic hydrocarbon group optionally having substituent(s)) or a group represented by the formula:

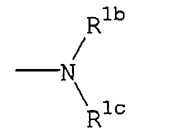

(wherein R^{1b} represents a hydrogen atom or an aliphatic hydrocarbon group optionally having substituent(s), and R^{1c} is, the same as or different from R^{1b}, a hydrogen atom or an aliphatic hydrocarbon group optionally having substituent(s)),
R⁰ represents a hydrogen atom or an aliphatic hydrocarbon group, or R¹ and R⁰ represent a bond with each other,
ring A is a cycloalkene substituted by 1 to 4 substituent(s) selected from (1) an aliphatic hydrocarbon group optionally having substituent(s), (2) an aromatic hydrocarbon group optionally having substituent(s), (3) a group represented by the formula: -OR¹¹ (wherein R¹¹ represents a hydrogen atom or an aliphatic hydrocarbon group optionally having substituent(s)) and (4) a halogen atom,
Ar represents an aromatic hydrocarbon group optionally having substituent(s),
a group represented by the formula:

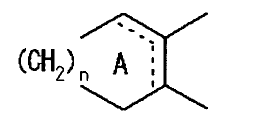

represents a group represented by the formula:

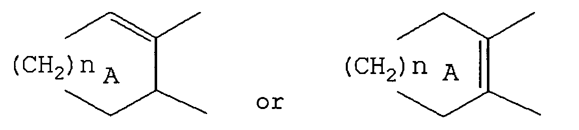

and n represents an integer of 1 to 4, and
(ii) a compound represented by the formula:

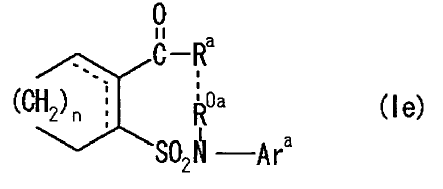

wherein R^{a} represents an aliphatic hydrocarbon group optionally having substituent(s), an aromatic hydrocarbon group optionally having substituent(s), a heterocyclic group optionally having substituent(s), a group represented by the formula: -OR^{1a} (wherein R^{1a} represents a hydrogen atom or an aliphatic hydrocarbon group optionally having substituent(s)) or a group represented by the formula:

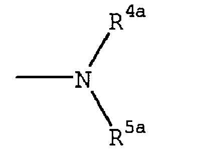

(wherein R^{4a} and R^{5a} are the same or different and each represents a hydrogen atom or an aliphatic hydrocarbon group optionally having substituent(s)),
R^{0a} represents a hydrogen atom or an aliphatic hydrocarbon group, or R^{a} and R^{0a} represent a bond with each other,
Ar^{a} represents an aromatic hydrocarbon group optionally having substituent(s),
a group represented by the formula:

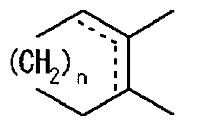

represents a group represented by the formula:

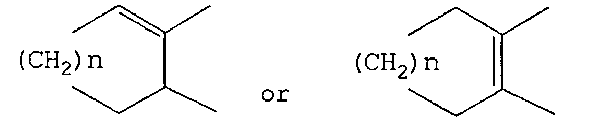

and n represents an integer of 1 to 4, a salt thereof and a prodrug thereof; and
in addition, patent reference 2 describes that a compound represented by the formula:

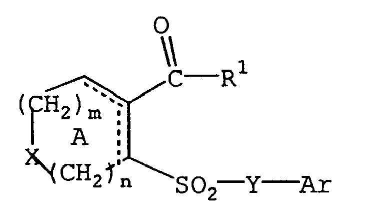

wherein R¹ represents an aliphatic hydrocarbon group optionally having substituent(s), an aromatic hydrocarbon group optionally having substituent(s), a heterocyclic group optionally having substituent(s), a group represented by the formula: -OR^{1a} (wherein R^{1a} represents a hydrogen atom or an aliphatic hydrocarbon group optionally having substituent(s)) or a group represented by the formula:

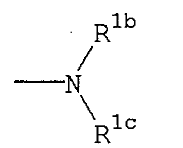

wherein R^{1b} and R^{1c} are the same or different and each represents a hydrogen atom or an aliphatic hydrocarbon group optionally having substituent(s),
X represents a methylene group, NH, a sulfur atom or an oxygen atom,
Y represents a methylene group optionally having substituent(s) or NH optionally having substituent(s), ring A represents a 5 to 8-membered ring optionally having 1 to 4 substituent(s) selected from the group consisting of (1) an aliphatic hydrocarbon group optionally having substituent(s), (2) an aromatic hydrocarbon group optionally having substituent(s), (3) a group represented by the formula: -OR² (wherein R² represents a hydrogen atom or an aliphatic hydrocarbon group optionally having substituent(s)) and (4) a halogen atom,
Ar represents an aromatic hydrocarbon group optionally having substituent(s),
a group represented by the formula:

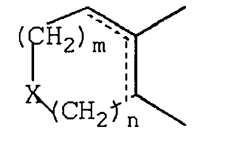

represents a group represented by the formula:

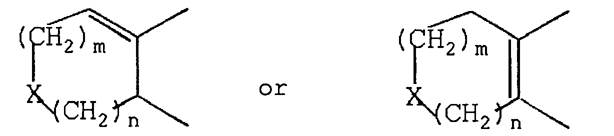

m represents an integer of 0 to 2,
n represents an integer of 1 to 3, and
the total of m and n is not more than 4;
provided that when X is a methylene group, Y represents a methylene group optionally having substituent(s), a salt thereof and a prodrug thereof have a nitric oxide (NO) production-inhibiting effect and an inhibitory effect on the production of inflammatory cytokines, such as TNF-α, IL-1, IL-6 and the like, and are useful as an agent for the prophylaxis or treatment of diseases including cardiac diseases, autoimmune diseases, inflammatory diseases, central nervous system diseases, infectious diseases, sepsis, septic shock and the like.
Patent reference 3 describes that the above-mentioned compound is useful as a TLR signaling inhibitor or an agent for the prophylaxis or treatment of severe sepsis. patent reference 1: WO99/46242
patent reference 2: WO01/10826
patent reference 3: WO03/84527

### Disclosure of the Invention

### Problems to be Solved by the Invention

The present invention aims at provision of a pharmaceutical agent effective for suppressing the production of various cytokines such as IL-10 and the like and inflammatory mediators, or effective for suppressing the expression of COX-II and the like.

### Means of Solving the Problems

The present inventors have conducted intensive studies in an attempt to solve the above-mentioned problem, and found that a TLR signaling inhibitory substance unexpectedly and efficiently suppresses production of various cytokines such as IL-10 and the like and inflammatory mediators, as well as expression of COX-II and the like. Further studies made by the present inventors based on said finding resulted in the completion of the present invention.

Accordingly, the present invention relates to
[1] an agent for suppressing the production of at least one kind of factor selected from IL-2, IL-3, IL-8, IL-10, IL-12, IL-17, MIP-2, KC, GM-CSF, IFN-γ and prostaglandin E2, which comprises a TLR signaling inhibitory substance;
[2] an agent for suppressing the expression of at least one kind selected from COX-II, IL-10, IL-18, MCP-1/3, MIP-2, RANTES, P2X4, plasminogen activator inhibitor, G-CSF, Fas antigen, TNF receptor, Fc receptor, galectin-9, histidine decarboxylase, IL-1 receptor antagonist and MMP-9, which comprises a TLR signaling inhibitory substance;
[3] an inhibitor of at least one kind of phosphorylation enzyme selected from ATF2, JNK, p38MAP kinase, IκBα, ERK1/2, p90RSK, STAT2 and p70 S6 kinase, which comprises a TLR signaling inhibitory substance;
[4] the agent of any of the above-mentioned [1] to [3], wherein the TLR signaling inhibitory substance is a compound represented by the formula (I):

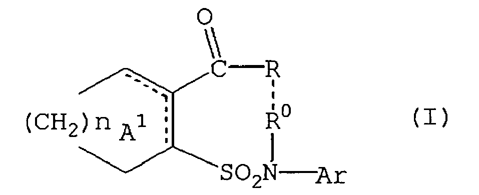

wherein R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula:

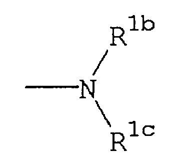

wherein R^{1b} and R^{1c} are the same or different and each is a hydrogen atom or an aliphatic hydrocarbon group optionally having substituent(s),
R⁰ is a hydrogen atom or an aliphatic hydrocarbon group, or R and R⁰ in combination show a bond,
ring A¹ is a cycloalkene optionally substituted by 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom, Ar is an aromatic hydrocarbon group optionally having substituent(s),
a group represented by the formula:

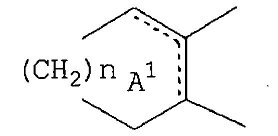

is a group represented by the formula:

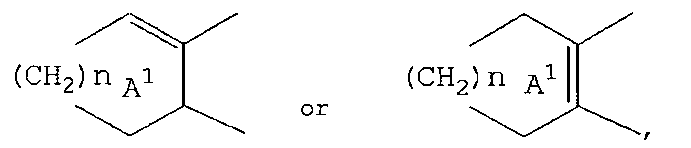

wherein n is an integer of 1 - 4, or a salt thereof or a prodrug thereof, or,
a compound represented by the formula (II):

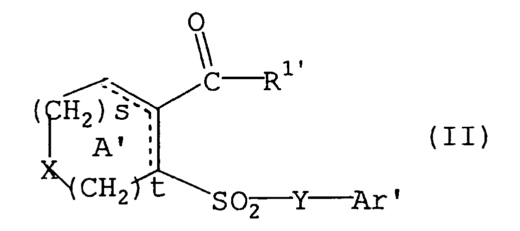

wherein R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula:

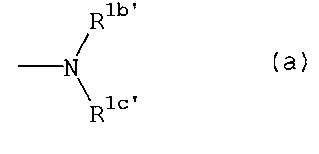

wherein R^{1b'} and R^{1c'} are the same or different and each is a hydrogen atom or an aliphatic hydrocarbon group optionally having substituent(s),
X is a methylene group, NH, a sulfur atom or an oxygen atom,
Y is a methylene group optionally having substituent(s) or NH optionally having substituent(s), ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR^{2'} wherein R^{2'} is a hydrogen atom or an aliphatic hydrocarbon group optionally having substituent(s) and (4) a halogen atom, Ar' is an aromatic hydrocarbon group optionally having substituent(s),
a group represented by the formula:

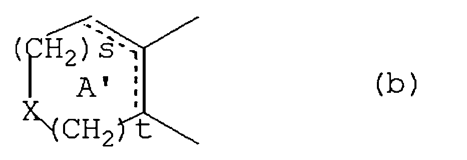

is a group represented by the formula:

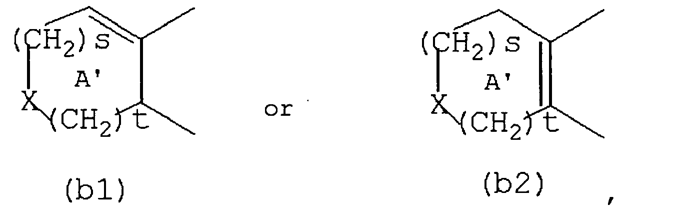

wherein s is an integer of 0 to 2,
t is an integer of 1 to 3,
the total of s and t is not more than 4;
provided that when X is a methylene group, then Y is a methylene group optionally having substituent(s), or a salt thereof or a prodrug thereof;
[5] the agent of the above-mentioned [4], wherein the formula (I) is the formula (Ia):

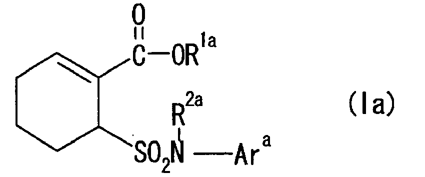

wherein R^{1a} is C₁₋₆ alkyl, R^{2a} is a hydrogen atom or C₁₋₆ alkyl, Ar^{a} is a phenyl group substituted by 1 or 2 halogen atoms, the formula (II) is the formula (IIa):

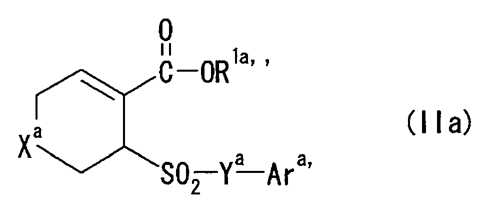

wherein R^{1a''} is C₁₋₆ alkyl, X^{a} is a methylene group or an oxygen atom, Y^{a} is a methylene group or -NH-, Ar^{a'} is a phenyl group optionally having 1 or 2 substituents selected from a halogen atom and a C₁₋₆ alkoxy group;
[6] the agent of any of the above-mentioned [1] - [3], which is used in combination with at least one kind of drug selected from the group consisting of antibacterial agents, antifungal agents, non-steroidal anti-inflammatory agent, steroid drugs, anticoagulant drugs and antisepsis agents;

[7] a method of suppressing the production of at least one kind of factor selected from IL-2, IL-3, IL-8, IL-10, IL-12, IL-17, MIP-2, KC, GM-CSF, IFN-γ and prostaglandin E2, comprising administering an effective amount of a TLR signaling inhibitory substance to a mammal;

[8] use of a TLR signaling inhibitory substance for the production of an agent for suppressing the production of at least one kind of factor selected from IL-2, IL-3, IL-8, IL-10, IL-12, IL-17, MIP-2, KC, GM-CSF, IFN-γ and prostaglandin E2;
[9] a method of suppressing the expression of at least one kind selected from COX-II, IL-10, IL-18, MCP-1/3, MIP-2, RANTES, P2X4, plasminogen activator inhibitor, G-CSF, Fas antigen, TNF receptor, Fc receptor, galectin-9, histidine decarboxylase, IL-1 receptor antagonist and MMP-9, comprising administering an effective of a TLR signaling inhibitory substance to a mammal;

[10] use of a TLR signaling inhibitory substance for the production of an agent for suppressing the expression of at least one kind selected from COX-II, IL-10, IL-18, MCP-1/3, MIP-2, RANTES, P2X4, plasminogen activator inhibitor, G-CSF, Fas antigen, TNF receptor, Fc receptor, galectin-9, histidine decarboxylase, IL-1 receptor antagonist and MMP-9;
[11] a method of inhibiting at least one kind of phosphorylation enzyme selected from ATF2, JNK, p38MAP kinase, IκBα, ERK1/2, p90RSK, STAT2 and p70 S6 kinase, comprising administering an effective amount of a TLR signaling inhibitory substance to a mammal;
[12] use of a TLR signaling inhibitory substance for the production of an inhibitor of at least one kind of phosphorylation enzyme selected from ATF2, JNK, p38MAP kinase, IκBα, ERK1/2, p90RSK, STAT2 and p70 S6 kinase;

[13] a method for the prophylaxis and/or treatment of at least one kind of disease or condition selected from infectious disease, cardiac disease, autoimmune diseases, inflammatory disease, central nervous system diseases, immune hypofunction, sepsis including severe sepsis, septic shock, sepsis, endotoxin shock, exotoxin shock, systemic inflammation reaction syndrome (SIRS), compensatory anti-inflammatory reaction syndrome (CARS), burn injury, trauma, postoperative complications, cardiac failure, shock, hypotension, rheumatism arthritis, osteoarthritis, gastritis, ulcerative colitis, peptic ulcer, stress-related gastric ulcer, Crohn's disease, autoimmune diseases, tissue disorder and rejection after organ transplantation, ischemia-reperfusion injury, acute coronary microvascular embolus, vascular embolus resulting from shock, which includes disseminated intravascular coagulation (DIC), ischemic brain disorder, arteriosclerosis, pernicious anemia, Fanconi anemia, sickle cell anemia, pancreatitis, nephrosis syndrome, nephritis, renal failure, insulin-dependent diabetes mellitus, insulin-independent diabetes mellitus, hepatic porphyria, alcohol poisoning, Parkinson's disease, chronic leukemia, acute leukemia, tumor, myeloma, infant and adult respiratory distress syndrome, emphysema, dementia, Alzheimer's disease, multiple sclerosis, vitamin E deficiency, aging, sunburn, muscular dystrophy, myocarditis, cardiomyopathy, myocardial infarction, myocardial infarction sequelae, osteoporosis, pneumonia, hepatitis, psoriasis, pain, cataract, influenza infections, malaria, human immunodeficiency virus (HIV) infections, radiation disorder, burn, hypercalcemia, ankylosing spondylitis, osteopenia, bone Paget's disease, osteomalacia, bone fracture, acute bacterial meningitis, Helicobacter pylori infections, Invasive Staphylococcus aureus infections, tuberculosis, systemic fungal infectious diseases, simple herpes virus infections, varicella-zoster virus infections, human papilloma virus infections, acute virus encephalitis, encephalitis, meningitis, immune hypofunction due to infections, asthma, atopic dermatitis, allergic rhinitis, erosive esophagitis, fever, hypercholesterolemia, hyperglyceridemia, hyperlipidemia, diabetic complications, diabetic nephropathy, diabetic neuropathy, diabetic retinopathy, gout, stomach atony, hemorrhoid, systemic lupus erythematosus, spinal trauma, insomnia, schizophrenia, epilepsy, cirrhosis, hepatic failure, unstable angina pectoris, cardiac valvular disease, thrombocytopenia or hypotension due to dialysis, acute ischemic cerebral apoplexy, acute brain thrombosis, cancer metastasis, urinary bladder cancer, breast cancer, cervical cancer, colorectal cancer, gastric cancer, ovarian cancer, prostate cancer, small cell lung cancer, non-small cell lung cancer, malignant melanoma, Hodgkin's disease, non-Hodgkin's lymphoma and side effects caused by the administration of an anti-cancer agent or an immunosuppressant, which comprises administering the agent of any of the above-mentioned [1] to [3] to a mammal; and

[14] a method for the prophylaxis and/or treatment of at least one kind of disease or condition selected from chronic obliterative pulmonary diseases, cystic fibrosis, lung fibrosis, autoimmune hemolytic anemia, meningitis, inflammatory pulmonary disease (including silicosis, lung sarcoidosis and pulmonary tuberculosis), endometriosis, cachexia (including cachexia derived from infection, carcinocachexia and cachexia induced by acquired immunodeficiency syndrome), cancer pain, Addison's disease, acute pain caused by inflammation, pain due to chronic inflammation, postoperative pain (including incision pain, deep pain, visceral pain and postoperative chronic pain), muscular pain (including muscular pain associated with chronic pain disease and stiff neck), arthritic pain, toothache, pain of temporomandibular joint, headache (including migraine, tension headache, headache caused by fever and headache associated with hypertension), visceral pain (cardiac pain, anginal pain, abdominal pain, kidney pain, ureteral pain, cystalgia, obstetric and gynecologic pain (including intermenstrual pain, dysmenorrheal and labor pain), neuralgia (including disc hernia, radicular pain, post herpetic neuralgia pain and trigeminal neuralgia pain), reflex sympathetic atrophy, complex topalgia syndrome, pituitary gland abscess, thyroiditis, peritonitis and erythema nodosum), allergic conjunctivitis, pollinosis, metal allergy, exudative otitis media, Meniere's disease, contact dermatitis, anaphylaxis, urticaria, myasthenia gravis, Sjogren's syndrome, Basedow's disease, leukocyte abnormality, renal tubulointerstitial injury (including fibrotic pathology), acute coronary artery syndrome, atherosclerotic aortic aneurysm, heart anaphylaxis, deep-vein thrombosis, ophthalmic disorder (including pterygium, spring catarrh and dry eye), food allergy, NUD (Non Ulcer Dyspepsia), stomach MALT lymphoma, ulcer caused by non-steroidal anti-inflammatory agent, hyperacidity, hyperacidity and ulcer due to postoperative stress, obesity, edema, granuloma, atopic myelitis, neurofibromatosis and nasal mucosal sensitivity, which comprises administering the agent of any of the above-mentioned [1] to [3] to a mammal.

### Effect of the Invention

A pharmaceutical composition comprising a TLR signaling inhibitory substance of the present invention is useful as an agent for suppressing the production of various cytokines such as IL-10 and the like and inflammatory mediators, an agent for suppressing the expression of COX-II and the like, and the like.

### Brief description of the Drawings

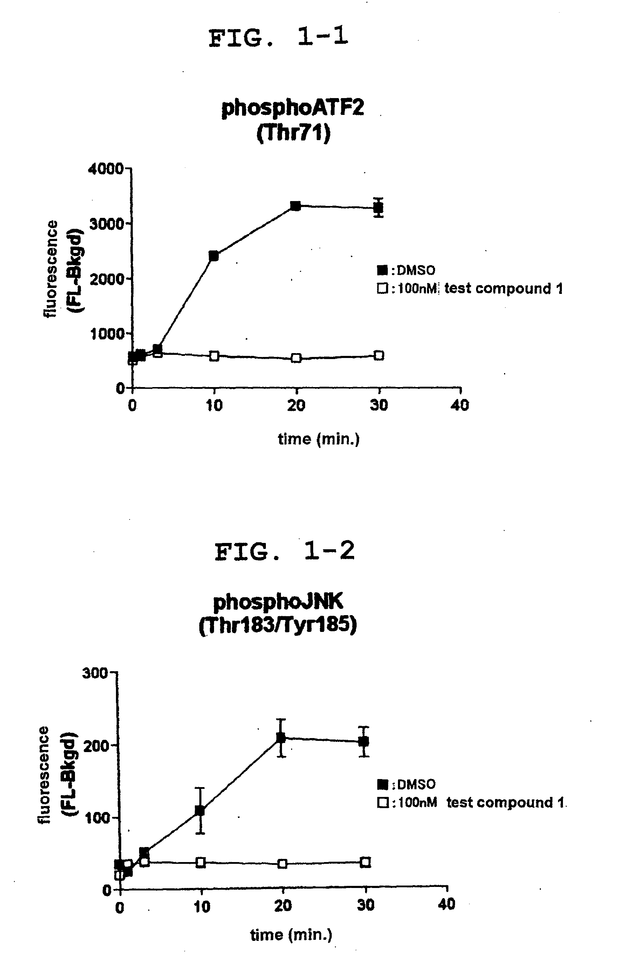

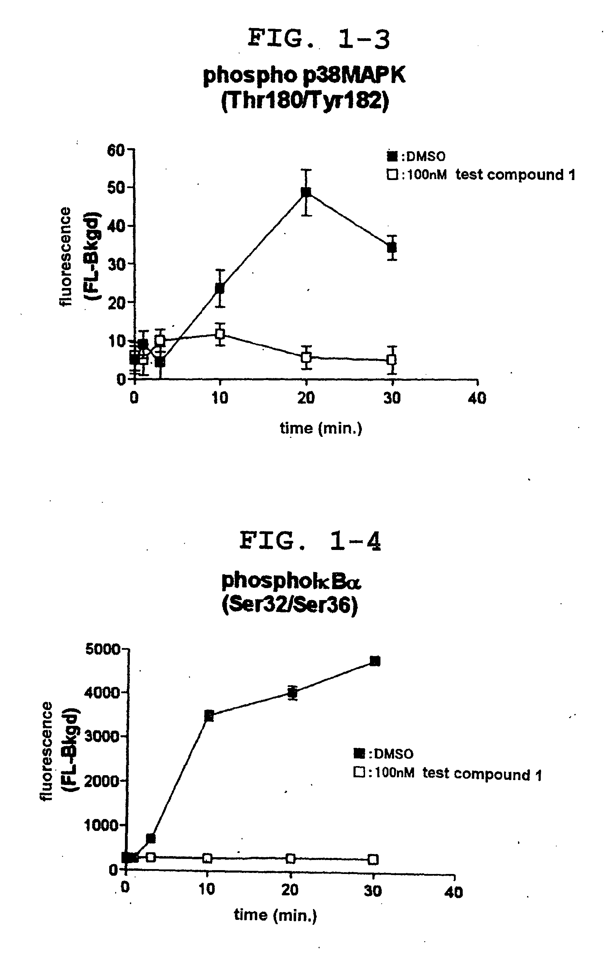

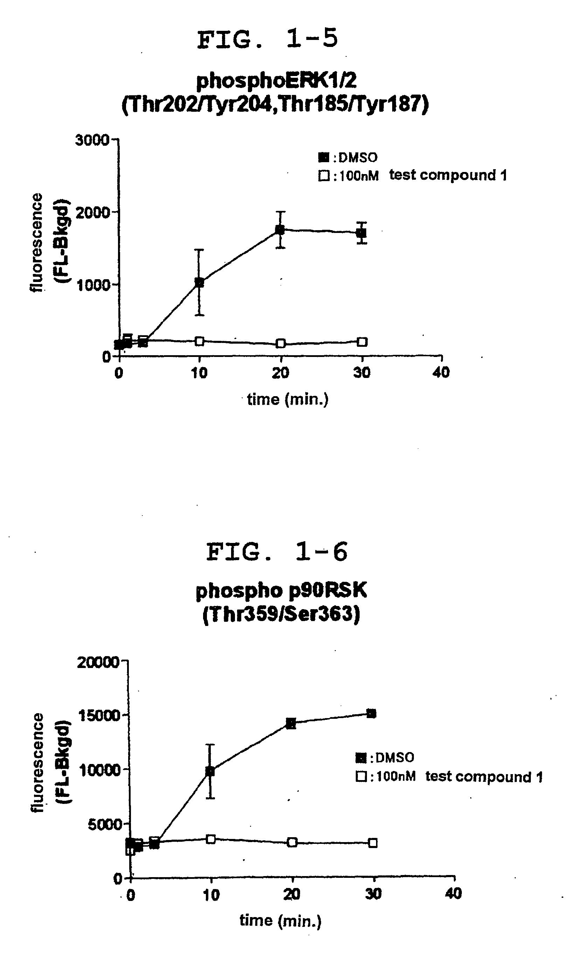

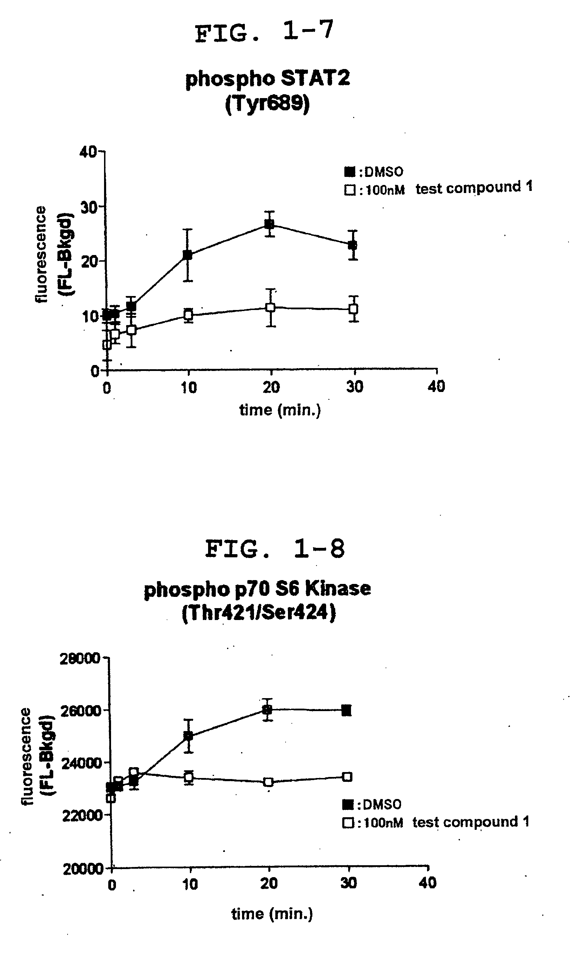

Fig. 1-1 is a graph showing the effect of test compound 1 on phosphorylated protein (ATF2) after LPS addition in mouse macrophage cells. An average of triplicate measurement values is shown, mean±standard error (Experimental Example 5, n=3).
Fig. 1-2 is a graph showing the effect of test compound 1 on phosphorylated protein (JNK) after LPS addition in mouse macrophage cells. An average of triplicate measurement values is shown, mean±standard error (Experimental Example 5, n=3).
Fig. 1-3 is a graph showing the effect of test compound 1 on phosphorylated protein (p38MAPK) after LPS addition in mouse macrophage cells. An average of triplicate measurement values is shown, mean±standard error (Experimental Example 5, n=3).
Fig. 1-4 is a graph showing the effect of test compound 1 on phosphorylated protein (IκBα) after LPS addition in mouse macrophage cells. An average of triplicate measurement values is shown, mean±standard error (Experimental Example 5, n=3).
Fig. 1-5 is a graph showing the effect of test compound 1 on phosphorylated protein (ERK1/2) after LPS addition in mouse macrophage cells. An average of triplicate measurement values is shown, mean±standard error (Experimental Example 5, n=3).
Fig. 1-6 is a graph showing the effect of test compound 1 on phosphorylated protein (p90RSK) after LPS addition in mouse macrophage cells. An average of triplicate measurement values is shown, mean±standard error (Experimental Example 5, n=3).
Fig. 1-7 is a graph showing the effect of test compound 1 on phosphorylated protein (STAT2) after LPS addition in mouse macrophage cells. An average of triplicate measurement values is shown, mean±standard error (Experimental Example 5, n=3).
Fig. 1-8 is a graph showing the effect of test compound 1 on phosphorylated protein (p70 S6 Kinase) after LPS addition in mouse macrophage cells. An average of triplicate measurement values is shown, mean±standard error (Experimental Example 5, n=3).

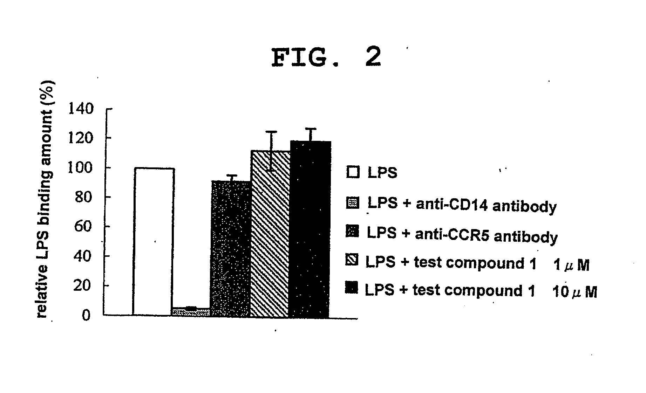

Fig. 2 is a graph showing the effects of test compound 1, anti-CD14 antibody and anti-CCR5 antibody on the binding of LPS to PBMC. With an average of triplicate measurement values of LPS alone as 100%, % averages of triplicate measurement values of other groups are shown, mean±standard error (Experimental Example 6, results of 4 independent tests using PBMC obtained from the blood of different 4 subjects).

In the present invention, TLR signaling means signal transduction for any Toll-like receptor to recognize a bacterial component and the like of microorganisms and induce a biological defense reaction. For example, signal transduction via known TLR1 - TLR10 can be mentioned. Of these, signal transduction via TLR4 is preferable.

In the present invention, as the TLR signaling inhibitory substance, for example, peptidic compound (e.g., anti-TLR antibody, TLR inhibitory peptide, anti-MIF (migration inhibitory factor) antibody, MIF inhibitory peptide and the like), or nonpeptidic compound and the like can be used.

The nonpeptidic compound is not particularly limited as long as it can inhibit signal transduction via any of the above-mentioned TLRs. While a nonpeptidic compound capable of specifically inhibiting the signal transduction via TLR4 is preferable, one capable of specifically inhibiting other TLR signaling and one capable of specifically inhibiting plural kinds of TLR are also preferable. For example, a low molecule weight nonpeptidic compound having a molecular weight of not more than about 1000, preferably not more than about 500, is used. Particularly, a compound having the below-mentioned cycloalkene skeleton or compound A is preferably used.

As the compound having a cycloalkene skeleton to be used in the present invention (hereinafter to be abbreviated as cycloalkene compound), compounds represented by the following formulas (I) and (II) (hereinafter to be collectively abbreviated as compound A), or a salt thereof or a prodrug thereof and the like is preferable.

The above-mentioned compounds are explained in detail in the following.
In the specification, R is an aliphatic hydrocarbon group optionally having substituent(s), an aromatic hydrocarbon group optionally having substituent(s), a heterocyclic group optionally having substituent(s), a group represented by the formula: -OR¹ (wherein R¹ is a hydrogen atom or an aliphatic hydrocarbon group optionally having substituent(s)) or a group represented by the formula:

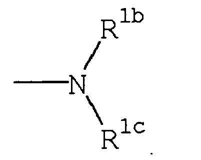

wherein R^{1b} and R^{1c} are the same or different and each is a hydrogen atom or an aliphatic hydrocarbon group optionally having substituent(s), or R and R⁰ in combination form a bond, with particular preference given to the group represented by the formula: -OR¹ (wherein R¹ is as defined above).
When R and R⁰ in combination form a bond, the compound represented by the formula (I) can be represented by the formula:

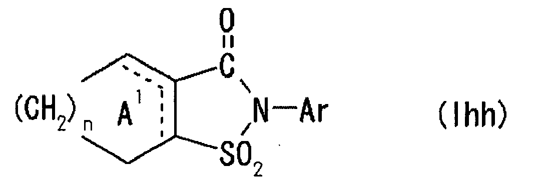

wherein each symbol is as defined above, and specifically can be represented by the formula:

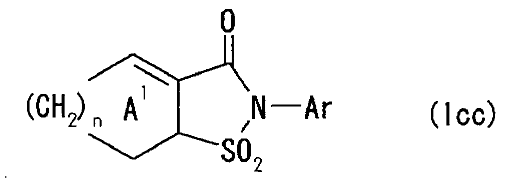

wherein each symbol is as defined above, or

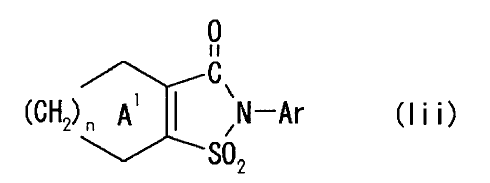

wherein each symbol is as defined above.
When R is a group represented by the formula: -OR¹ (wherein R¹ is as defined above), the compound represented by the formula (I) can be represented by the formula:

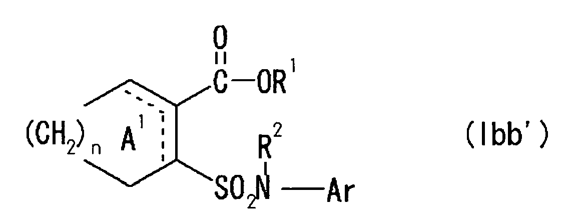

wherein R² is a hydrogen atom or an aliphatic hydrocarbon group, and other symbols are as defined above, and specifically can be represented by the formula:

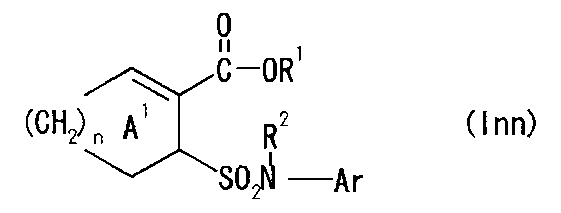

wherein each symbol is as defined above, or the formula:

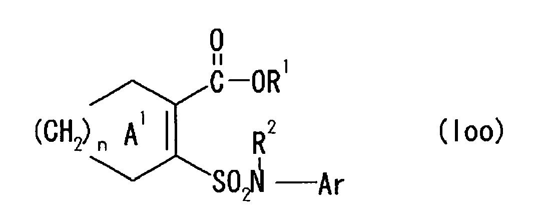

wherein each symbol is as defined above.
As the compound represented by the formula (I), a compound represented by the formula (Icc) or the formula (Inn) is preferable.

As the "aliphatic hydrocarbon group" of the "aliphatic hydrocarbon group optionally having substituent(s)" represented by R, R¹, R¹¹, R^{1b} and R^{1c} and the "aliphatic hydrocarbon group" represented by R⁰ and R², for example, an alkyl group, a cycloalkyl group, a cycloalkylalkyl group, an alkenyl group, an alkynyl group, etc. are preferable.

As the alkyl group, for example, a linear or branched alkyl group having 1 to 20 carbon atoms (e.g., a methyl group, an ethyl group, an n-propyl group, an isopropyl group, an n-butyl group, an isobutyl group, a sec-butyl group, a tert-butyl group, a pentyl group, a hexyl group, a heptyl group, an octyl group, a nonyl group, a decyl group, a dodecyl group, etc.) and the like are preferable, and particularly, for example, a lower alkyl group having 1 to 6 carbon atoms (e.g., a methyl group, an ethyl group, an n-propyl group, an isopropyl group, an n-butyl group, an isobutyl group, a sec-butyl group, a tert-butyl group, etc.) and the like are preferable.

As the cycloalkyl group, for example, a cycloalkyl group having 3 to 10 carbon atoms (e.g., a cyclopropyl group, a cyclobutyl group, a cyclopentyl group, a cyclohexyl group, a cycloheptyl group, a cyclooctyl group, etc.) and the like are preferable, and particularly, for example, a cycloalkyl group having 3 to 6 carbon atoms (e.g., a cyclopropyl group, a cyclobutyl group, a cyclopentyl group, a cyclohexyl group, etc.) and the like are preferable.

As the cycloalkylalkyl group, for example, a cycloalkylalkyl group having 4 to 12 carbon atoms (e.g., a cyclopropylmethyl group, a cyclopentylmethyl group, a cyclohexylmethyl group, a cycloheptylmethyl group, etc.) and the like are preferable, and particularly, for example, a cycloalkylalkyl group having 4 to 8 (particularly 4 to 7) carbon atoms (e.g., a cyclopropylmethyl group, a cyclopentylmethyl group, a cyclohexylmethyl group, etc.) and the like are preferable.

As the alkenyl group, for example, a lower alkenyl group having 3 to 6 carbon atoms (e.g., a propenyl group, a butenyl group, a pentenyl group, etc.) and the like are preferable, and particularly, for example, a lower alkenyl group having 3 or 4 carbon atoms (e.g., a propenyl group, a butenyl group, etc.) and the like are preferable.

As the alkynyl group, for example, a lower alkynyl group having 3 to 6 carbon atoms (e.g., a propynyl group, a butynyl group, a pentynyl group, etc.) and the like are preferable, and particularly, for example, a lower alkynyl group having 3 or 4 carbon atoms (e.g., a propynyl group, a butynyl group, etc.) and the like are preferable.

As the "substituent" of the above-mentioned "aliphatic hydrocarbon group optionally having substituent(s)", for example, a heterocyclic group, an oxo group, a hydroxy group, a C₁₋₆ alkoxy group, a C₃₋₁₀ (particularly C₃-₆) cycloalkyloxy group, a C₆₋₁₀ aryloxy group, a C₇₋₁₉ (particularly C₇₋₁₂) aralkyloxy group, a heterocyclic-oxy group, a C₁₋₆ alkylthio group (sulfur atom may be oxidized), a C₃₋₁₀ (particularly C₃₋₆) cycloalkylthio group (sulfur atom may be oxidized), a C₆₋₁₀ arylthio group (sulfur atom may be oxidized), a C₇₋₁₉ (particularly C₇₋₁₂) aralkylthio group (sulfur atom may be oxidized), a heterocyclic-thio group, a heterocyclic-sulfinyl group, a heterocyclic-sulfonyl group, a nitro group, a halogen atom, a cyano group, a carboxyl group, a C₁₋₁₀ (particularly C₁₋₆) alkoxy-carbonyl group, a C₃₋₆ cycloalkyloxy-carbonyl group, a C₆₋₁₀ aryloxy-carbonyl group, a C₇₋₁₉ (particularly C₇₋₁₂) aralkyloxy-carbonyl group, a heterocyclic-oxy-carbonyl group, a C₆₋₁₀ aryl-carbonyl group, C₁₋₆ alkanoyl group, C₃₋₅ alkenoyl group, a C₆₋₁₀ aryl-carbonyloxy group, a C₂₋₆ alkanoyloxy group, a C₃₋₅ alkenoyloxy group, a carbamoyl group optionally having substituent(s), a thiocarbamoyl group optionally having substituent(s), a carbamoyloxy group optionally having substituent(s), a C₁₋₆ alkanoylamino group, a C₆₋₁₀ aryl-carbonylamino group, a C₁₋₁₀ (particularly C₁₋₆) alkoxy-carboxamido group, a C₆₋₁₀ aryloxy-carboxamido group, a C₇₋₁₉ (particularly C₇₋₁₂) aralkyloxy-carboxamido group, a C₁₋₁₀ (particularly C₁₋₆) alkoxy-carbonyloxy group, a C₆₋₁₀ aryloxy-carbonyloxy group, a C₇₋₁₉ (particularly C₇₋₁₂) aralkyloxy-carbonyloxy group, a C₃₋₁₀ (particularly C₃₋₆) cycloalkyloxy-carbonyloxy group, a ureido group optionally having substituent(s), a C₆₋₁₀ aryl group optionally having substituent(s), etc. are used.
These substituents substitute at substitutable positions in the above-mentioned "aliphatic hydrocarbon group", wherein the substituents are not limited to a single substituent but may be the same or different plural (preferably 2 to 4) substituents.

As the "C₁₋₆ alkoxy group", for example, a methoxy group, an ethoxy group, an n-propoxy group, an isopropoxy group, an n-butoxy group, a tert-butoxy group, an n-pentyloxy group, an n-hexyloxy group, etc. are used, as the "C₃₋₁₀ cycloalkyloxy group", for example, a cyclopropyloxy group, a cyclohexyloxy group, etc. are used, as the "C₆₋₁₀ aryloxy group", for example, a phenoxy group, a naphthyloxy group, etc. are used, as the "C₇₋₁₉ aralkyloxy group", for example, a benzyloxy group, a 1-phenylethyloxy group, a 2-phenylethyloxy group, a benzhydryloxy group, a 1-naphthylmethyloxy group, etc. are used, as the "C₁₋₆ alkylthio group (sulfur atom may be oxidized)", for example, a methylthio group, an ethylthio group, an n-propylthio group, an n-butylthio group, a methylsulfinyl group, a methylsulfonyl group, etc. are used, as the "C₃₋₁₀ cycloalkylthio group (sulfur atom may be oxidized)", for example, a cyclopropylthio group, a cyclohexylthio group, a cyclopentylsulfinyl group, a cyclohexylsulfonyl group, etc. are used, as the "C₆₋₁₀ arylthio group (sulfur atom may be oxidized)", for example, a phenylthio group, a naphthylthio group, a phenylsulfinyl group, a phenylsulfonyl group, etc. are used, as the "C₇₋₁₉ aralkylthio group (sulfur atom may be oxidized)", for example, a benzylthio group, a phenylethylthio group, a benzhydrylthio group, a benzylsulfinyl group, a benzylsulfonyl group, etc. are used, as the "halogen atom", a fluorine atom, a chlorine atom, a bromine atom and an iodine atom are used, as the "C₁₋₁₀ alkoxy-carbonyl group", for example, a methoxycarbonyl group, an ethoxycarbonyl group, an n-propoxycarbonyl group, an isopropoxycarbonyl group, an n-butoxycarbonyl group, an isobutoxycarbonyl group, a tert-butoxycarbonyl group, etc. are used, as the "C₃₋₆ cycloalkyloxy-carbonyl group", for example, a cyclopropyloxycarbonyl group, a cyclopentyloxycarbonyl group, a cyclohexyloxycarbonyl group, etc. are used, as the "C₆₋₁₀ aryloxy-carbonyl group", for example, a phenoxycarbonyl group, a naphthyloxycarbonyl group, etc. are used, as the "C₇₋₁₉ aralkyloxy-carbonyl group", for example, a benzyloxycarbonyl group, a benzhydryloxycarbonyl group, a 2-phenethyloxycarbonyl group, etc. are used, as the "C₆₋₁₀ aryl-carbonyl group", for example, a benzoyl group, a naphthoyl group, etc. are used, as the "C₁₋₆ alkanoyl group", for example, a formyl group, an acetyl group, a propionyl group, a butyryl group, a valeryl group, a pivaloyl group, etc. are used, as the "C₃₋₅ alkenoyl group", for example, an acryloyl group, a crotonoyl group, etc. are used, as the "C₆₋₁₀ aryl-carbonyloxy group", for example, a benzoyloxy group, a naphthoyloxy group, etc. are used, as the "C₂₋₆ alkanoyloxy group", for example, an acetoxy group, a propionyloxy group, a butyryloxy group, a valeryloxy group, a pivaloyloxy group, etc. are used, and as the "C₃₋₅ alkenoyloxy group", for example, an acryloyloxy group, a crotonoyloxy group, etc. are used.

As the "carbamoyl group optionally having substituent(s)", for example, a carbamoyl group or a cyclic-amino (e.g., pyrrolidinyl, piperidinyl, piperazinyl, morpholinyl, etc.) carbonyl group, which may be substituted by 1 or 2 substituents selected from a C₁₋₄ alkyl (e.g., methyl, ethyl, etc.), a phenyl, a C₁₋₇ acyl (e.g., acetyl, propionyl, benzoyl, etc.), a C₁₋₄ alkoxy-phenyl (e.g., methoxyphenyl, etc.), etc., and the like are used, and specifically, for example, a carbamoyl group, an N-methylcarbamoyl group, an N-ethylcarbamoyl group, an N,N-dimethylcarbamoyl group, an N,N-diethylcarbamoyl group, an N-phenylcarbamoyl group, an N-acetylcarbamoyl group, an N-benzoylcarbamoyl group, an N-(p-methoxyphenyl)carbamoyl group, a 1-pyrrolidinylcarbonyl group, a piperidinocarbonyl group, a 1-piperazinylcarbonyl group, a morpholinocarbonyl group, etc. are used. As the "thiocarbamoyl group optionally having substituent(s)", for example, a thiocarbamoyl group which may be substituted by 1 or 2 substituents selected from C₁₋₄ alkyl (e.g., methyl, ethyl, etc.), phenyl, etc. are used, and specifically, for example, a thiocarbamoyl group, an N-methylthiocarbamoyl group, an N-phenylthiocarbamoyl group, etc. are used. As the "carbamoyloxy group optionally having substituent(s)", for example, a carbamoyloxy group which may be substituted by 1 or 2 substituents selected from C₁₋₄ alkyl (e.g., methyl, ethyl, etc.), phenyl, etc. are used, and specifically, for example, a carbamoyloxy group, an N-methylcarbamoyloxy group, an N,N-dimethylcarbamoyloxy group, an N-ethylcarbamoyloxy group, an N-phenylcarbamoyloxy group, etc. are used.

As the "C₁₋₆ alkanoylamino group", for example, an acetamido group, a propionamido group, a butyramido group, a valeramido group, a pivalamido group, etc. are used, as the "C₆₋₁₀ aryl-carbonylamino group", for example, a benzamido group, a naphthamido group, a phthalimido group, etc. are used, as the "C₁₋₁₀ alkoxy-carboxamido group", for example, a methoxy-carboxamido (CH₃OCONH-) group, an ethoxycarboxamido group, a tert-butoxycarboxamido group, etc. are used, as the "C₆₋₁₀ aryloxy-carboxamido group", for example, a phenoxycarboxamido (C₆H₅OCONH-) group, etc. are used, as the "C₇₋₁₉ aralkyloxy-carboxamido group", for example, a benzyloxycarboxamido (C₆H₅CH₂OCONH-) group, a benzhydryloxycarboxamido group, etc. are used, as the "C₁₋₁₀ alkoxy-carbonyloxy group", for example, a methoxycarbonyloxy group, an ethoxycarbonyloxy group, an n-propoxycarbonyloxy group, an isopropoxycarbonyloxy group, an n-butoxycarbonyloxy group, a tert-butoxycarbonyloxy group, an n-pentyloxycarbonyloxy group, an n-hexyloxycarbonyloxy group, etc. are used, as the "C₆₋₁₀ aryloxy-carbonyloxy group", for example, a phenoxycarbonyloxy group, a naphthyloxycarbonyloxy group, etc. are used, as the "C₇₋₁₉ aralkyloxy-carbonyloxy group", for example, a benzyloxycarbonyloxy group, a 1-phenylethyloxycarbonyloxy group, a 2-phenylethyloxycarbonyloxy group, a benzhydryloxycarbonyloxy group, etc. are used, and as the "C₃₋₁₀ cycloalkyloxy-carbonyloxy group", for example, a cyclopropyloxycarbonyloxy group, a cyclohexyloxycarbonyloxy group, etc. are used.

As the "ureido group optionally having substituent(s)", for example, a ureido group optionally substituted by 1 to 3 (preferably 1 or 2) substituents selected from a C₁₋₄ alkyl group (e.g., a methyl group, an ethyl group, etc.), a phenyl group, etc. are used, and, for example, a ureido group, a 1-methylureido group, a 3-methylureido group, a 3,3-dimethylureido group, a 1,3-dimethylureido group, a 3-phenylureido group, etc. are used.

When a heterocyclic group, a heterocyclic-oxy group, a heterocyclic-thio group, a heterocyclic-sulfinyl group, a heterocyclic-sulfonyl group or a heterocyclic-oxy-carbonyl group is used as the "substituent(s)" of the "aliphatic hydrocarbon group optionally having substituent(s)", the heterocyclic group represents a group formed by excluding one hydrogen atom that binds to the heterocycle. It represents, for example, a 5- to 8-membered ring (preferably 5- or 6-membered ring) group containing 1 to a few, preferably 1 to 4 hetero atoms such as a nitrogen atom (optionally oxidized), an oxygen atom, a sulfur atom, etc., or its condensed cyclic group. As these heterocyclic groups, for example, a pyrrolyl group, a pyrazolyl group, an imidazolyl group, a 1,2,3-triazolyl group, a 1,2,4-triazolyl group, a tetrazolyl group, a furyl group, a thienyl group, an oxazolyl group, an isoxazolyl group, a 1,2,3-oxadiazolyl group, a 1,2,4-oxadiazolyl group, a 1,2,5-oxadiazolyl group, a 1,3,4-oxadiazolyl group, a thiazolyl group, an isothiazolyl group, a 1,2,3-thiadiazolyl group, a 1,2,4-thiadiazolyl group, a 1,2,5-thiadiazolyl group, a 1,3,4-thiadiazolyl group, a pyridyl group, a pyridazinyl group, a pyrimidinyl group, a pyrazinyl group, an indolyl group, a pyranyl group, a thiopyranyl group, a dioxinyl group, a dioxolyl group, a quinolyl group, a pyrido[2,3-d]pyrimidyl group, a 1,5-, 1,6-, 1,7-, 1,8-, 2,6-or 2,7-naphthyridyl group, a thieno[2,3-d]pyridyl group, a benzopyranyl group, a tetrahydrofuryl group, a tetrahydropyranyl group, a dioxolanyl group, a dioxanyl group, etc. are used.
These heterocyclic groups may be substituted at substitutable positions by 1 to 3 substituents selected from a C₁₋₄ alkyl (e.g., methyl, ethyl, etc.), a hydroxy, an oxo, a C₁₋₄ alkoxy (e.g., methoxy, ethoxy, etc.), and the like.

As the "C₆₋₁₀ aryl group" of the "C₆₋₁₀ aryl group optionally having substituent(s)", for example, a phenyl group, a naphthyl group, etc. are used. The C₆₋₁₀ aryl group may be substituted at a substitutable position by a substituent selected from those exemplified as the "substituent" (except for a C₆₋₁₀ aryl group optionally having substituent(s)) of the "aliphatic hydrocarbon group optionally having substituent(s)" described above. Such substituent is not limited to a single substituent, but the same or different, more than one (preferably 2 to 4) substituents may be used.

In the "aliphatic hydrocarbon group optionally having substituent(s)", the substituent together with the aliphatic hydrocarbon group may form an optionally substituted fused ring group, and as such fused ring group, an indanyl group, a 1,2,3,4-tetrahydronaphthyl group, etc. are used. This fused ring group may be substituted at a substitutable position by a substituent selected from those exemplified as the "substituent" of the "aliphatic hydrocarbon group optionally having substituent(s)" described above. Such substituent substitutes at a substitutable position of the fused ring group, wherein the substituent is not limited to a single substituent, but the same or different, more than one (preferably 2 to 4) substituents may be used.

As preferable examples of the above-mentioned "aliphatic hydrocarbon group optionally having substituent(s)" for R, R¹, R¹¹, R^{1b} and R^{1c}, for example, a lower alkyl group having 1 to 6 carbon atoms (e.g., a methyl group, an ethyl group, an n-propyl group, an isopropyl group, an n-butyl group, an isobutyl group, a tert-butoxycarbonylmethyl group, a hydroxyethyl group etc.) optionally having substituent(s), etc., are used. Of these, a methyl group, an ethyl group, an n-propyl group, an isopropyl group, an n-butyl group, an isobutyl group, etc. are preferable. For example, a methyl group, an ethyl group, an n-propyl group and the like are more preferable, and particularly, an ethyl group, etc. are preferable.

As the "aromatic hydrocarbon group" of the "aromatic hydrocarbon group optionally having substituent(s)" represented by R, an aromatic hydrocarbon group having 6 to 14 carbon atoms (e.g., a phenyl group, a naphthyl group, an anthryl group, an indenyl group etc.) and the like are preferable, and particularly for example, an aryl group having 6 to 10 carbon atoms (e.g., phenyl, naphthyl groups etc.) and the like are preferable and, of these, a phenyl group and the like are particularly preferable.

As the "substituent" of the "aromatic hydrocarbon group optionally having substituent(s)" represented by R, for example, a halogen atom (fluorine atom, chlorine atom, bromine atom, iodine atom), a lower (C₁₋₄) alkyl group (e.g., a methyl group, an ethyl group, a propyl group, a butyl group etc.), a lower (C₁₋₄) alkoxy group (e.g., a methoxy group, an ethoxy group, a propoxy group, a butoxy group etc.), a lower C₁₋₄) alkoxy-carbonyl group (e.g., a methoxycarbonyl group, an ethoxycarbonyl group, a propoxycarbonyl group, a butoxycarbonyl group etc.), a carboxyl group, a nitro group, a cyano group, a hydroxyl group, an acylamino group (e.g., an alkanoylamino group having 1 to 4 carbon atoms such as an acetylamino group, a propionylamino group, a butyrylamino group and the like, etc.), a cycloalkyl group having 3 to 6 carbon atoms (e.g., a cyclopropyl group, a cyclopentyl group etc.), an aryl group having 6 to 10 carbon atoms (e.g., a phenyl group, a naphthyl group, an indenyl group etc.), a halogeno-lower (C₁₋₄) alkyl group (e.g., a trifluoromethyl group, a trifluoroethyl group etc.), a halogeno-lower (C₁₋₄) alkoxy group (e.g., a trifluoromethoxy group, a 1,1,2,2-tetrafluoroethoxy group, a 2,2,3,3,3-pentafluoropropoxy group etc.), a lower (C₁₋₄) alkylthio group (e.g., a methylthio group, an ethylthio group, a propylthio group etc.), a lower (C₁₋₄) alkanesulfonyl group (e.g., a methanesulfonyl group, an ethanesulfonyl group, a propanesulfonyl group etc.), a lower (C₁₋₄) alkanoyl group (e.g., a formyl group, an acetyl group, a propionyl group etc.), a 5-membered aromatic heterocyclic group (e.g., a 1,2,3-triazolyl group, a 1,2,4-triazolyl group, a tetrazolyl group, a thiazolyl group, an isothiazolyl group, an oxazolyl group, an isoxazolyl group, a thiadiazolyl group, a thienyl group, a furyl group etc.), a carbamoyl group, a lower (C₁₋₄) alkyl-carbamoyl group (e.g., a methylcarbamoyl group, a dimethylcarbamoyl group, a propylcarbamoyl group etc.), a lower (C₁₋₄) alkoxy-carbonyl-lower (C₁₋₄) alkyl-carbamoyl group (e.g., a butoxycarbonylmethylcarbamoyl group, an ethoxycarbonylmethylcarbamoyl group etc.), a 1,3-diacylguanidino-lower (C₁₋₄) alkyl group (e.g., 1,3-diacetylguanidinomethyl, 1,3-bis-(tert-butoxycarbonyl)guanidinomethyl etc.) and the like are used, and a halogen atom (fluorine, chlorine, bromine, iodine atoms), a lower (C₁₋₄) alkyl group (e.g., a methyl group, an ethyl group, a propyl group, a butyl group etc.) and the like are preferably used, and a fluorine atom, a chlorine atom and a methyl group are more preferably used.
These substituents substitute at substitutable positions of the aromatic hydrocarbon group, and the number of the substituents is preferably 1 to 5, more preferably 1 to 3, most preferably 1 or 2. When two or more of such substituents are present, they may be the same or different.

The "heterocyclic group" of the "heterocyclic group optionally having substituent(s)" represented by R is, for example, a 5 to 8-membered ring (particularly 5 or 6-membered ring) group containing 1 to several, preferably 1 to 4, hetero atoms such as nitrogen atom (optionally oxidized), oxygen atom, sulfur atom and the like, and a fused ring group thereof. As such heterocyclic group, for example, pyrrolyl group, pyrazolyl group, imidazolyl group, 1,2,3-triazolyl group, 1,2,4-triazolyl group, tetrazolyl group, furyl group, thienyl group, oxazolyl group, isoxazolyl group, 1,2,3-oxadiazolyl group, 1,2,4-oxadiazolyl group, 1,2,5-oxadiazolyl group, 1,3,4-oxadiazolyl group, thiazolyl group, isothiazolyl group, 1,2,3-thiadiazolyl group, 1,2,4-thiadiazolyl group, 1,2,5-thiadiazolyl group, 1,3,4-thiadiazolyl group, pyridyl group, pyridazinyl group, pyrimidinyl group, pyrazinyl group, indolyl group, pyranyl group, thiopyranyl group, dioxinyl group, dioxolyl group, quinolyl group, pyrido[2,3-d]pyrimidyl group, 1,5-, 1,6-, 1,7-, 1,8-, 2,6- or 2,7-naphthyridinyl group, thieno[2,3-d]pyridyl group, benzopyranyl group, tetrahydrofuryl group, tetrahydropyranyl group, dioxolanyl group, dioxanyl group and the like are used.
These heterocyclic groups are optionally substituted by 1 to 3 substituents selected from C₁₋₄ alkyl (e.g., methyl, ethyl etc.), hydroxy, oxo, C₁₋₄ alkoxy (e.g., methoxy, ethoxy etc.) and the like at substitutable positions.

As the "aromatic hydrocarbon group" of the "aromatic hydrocarbon group optionally having substituent(s)" represented by Ar, an aromatic hydrocarbon group having 6 to 14 carbon atoms (e.g., a phenyl group, a naphthyl group, an anthryl group, an indenyl group etc.) and the like are preferable, and particularly for example, an aryl group having 6 to 10 carbon atoms (e.g., phenyl, naphthyl groups etc.) and the like are preferable and, of these, a phenyl group and the like are particularly preferable.

As the "substituent" of the "aromatic hydrocarbon group optionally having substituent(s)" represented by Ar and Ar^{a}, for example, a halogen atom (fluorine, chlorine, bromine, iodine atoms), a lower (C₁₋₄) alkyl group (e.g., a methyl group, an ethyl group, a propyl group, an isopropyl group, a butyl group etc.), a lower (C₁₋₄) alkoxy group (e.g., a methoxy group, an ethoxy group, a propoxy group, a butoxy group etc.), a lower (C₁₋₄) alkoxy-carbonyl group (e.g., a methoxycarbonyl group, an ethoxycarbonyl group, a propoxycarbonyl group, a butoxycarbonyl group etc.), a carboxyl group, a nitro group, a cyano group, a hydroxyl group, an acylamino group (e.g., an alkanoylamino group having 1 to 4 carbon atoms such as an acetylamino group, a propionylamino group, a butyrylamino group and the like, etc.), a cycloalkyl group having 3 to 6 carbon atoms (e.g., a cyclopropyl group, a cyclopentyl group etc.), an aryl group having 6 to 10 carbon atoms (e.g., a phenyl group, a naphthyl group, an indenyl group etc.), a halogeno-lower (C₁₋₄) alkyl group (e.g., a trifluoromethyl group, a trifluoroethyl group etc.), a halogeno-lower (C₁₋₄) alkoxy group (e.g., a trifluoromethoxy group, a 1,1,2,2-tetrafluoroethoxy group, a 2,2,3,3,3-pentafluoropropoxy group etc.), a lower (C₁₋₄) alkylthio group (e.g., a methylthio group, an ethylthio group, a propylthio group etc.), a lower (C₁₋₄) alkanesulfonyl group (e.g., a methanesulfonyl group, an ethanesulfonyl group, a propanesulfonyl group etc.), a lower (C₁₋₄) alkanoyl group (e.g., a formyl group, an acetyl group, a propionyl group etc.), a 5-membered aromatic heterocyclic group (e.g., a 1,2,3-triazolyl group, a 1,2,4-triazolyl group, a tetrazolyl group, a thiazolyl group, an isothiazolyl group, an oxazolyl group, an isoxazolyl group, a thiadiazolyl group, a thienyl group, a furyl group etc.), a carbamoyl group, a lower (C₁₋₄) alkyl-carbamoyl group (e.g., a methylcarbamoyl group, a dimethylcarbamoyl group, a propionylcarbamoyl group etc.), a lower (C₁₋₄) alkoxy-carbonyl-lower (C₁₋₄) alkyl-carbamoyl group (e.g., a butoxycarbonylmethylcarbamoyl group, a tert-butoxycarbonylmethylcarbamoyl group, an ethoxycarbonylmethylcarbamoyl group etc.), a 1,3-diacylguanidino-lower (C₁₋₄) alkyl group (e.g., 1,3-diacetylguanidinomethyl, 1,3-bis-(tert-butoxycarbonyl)guanidinomethyl etc.) and the like are used, and a halogen atom (fluorine, chlorine, bromine, iodine atoms), a lower (C₁₋₄) alkyl group (e.g., a methyl group, an ethyl group, a propyl group, a butyl group etc.) and the like are preferably used, and a fluorine atom, a chlorine atom and a methyl group are more preferably used.
These substituents substitute at substitutable positions of the aromatic hydrocarbon group, and the number of the substituents is preferably 1 to 5, more preferably 1 to 3, most preferably 1 or 2. When two or more of such substituents are present, they may be the same or different.

Typically, as Ar, for example, a phenyl group, a halogenophenyl group, a lower (C₁₋₄) alkylphenyl group, a lower (C₁₋₄) alkoxyphenyl group, a lower (C₁₋₄) alkoxy-carbonylphenyl group, a carboxylphenyl group, a nitrophenyl group, a cyanophenyl group, a halogeno-lower (C₁₋₄) alkylphenyl group, a halogeno-lower (C₁₋₄) alkoxyphenyl group, a lower (C₁₋₄) alkanoyl phenyl group, a 5-membered aromatic heterocyclyl-substituted phenyl group, a lower (C₁₋₄) alkoxy-carbonyl-lower (C₁₋₄) alkyl-carbamoylphenyl group, 1,3-diacylguanidino-lower (C₁₋₄) alkylphenyl group, a phenyl group substituted by halogen atom and a lower (C₁₋₄) alkyl group, a phenyl group substituted by halogen atom and lower (C₁₋₄) alkoxycarbonyl group, a phenyl group substituted by halogen atom and cyano group, a phenyl group substituted by halogen atom and 5-membered aromatic heterocyclic group, a phenyl group substituted by halogen atom and lower (C₁₋₄) alkoxy-carbonyl-lower (C₁₋₄) alkyl-carbamoyl group and the like are used.

As Ar, a phenyl group optionally having substituent(s) is preferable. Of these, a halogenophenyl group, a lower (C₁₋₄) alkylphenyl group, a halogen- and lower (C₁₋₄) alkoxycarbonyl-substituted phenyl group, a halogen- and lower (C₁₋₄) alkylsubstituted phenyl group and the like are preferably used.
As Ar, a group represented by the formula:

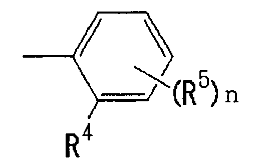

wherein R⁴ and R⁵ are the same or different and each represents a halogen atom or a lower (C₁₋₄) alkyl group, and n is an integer of 0 to 2, is more preferable, in which a group wherein at least one of R⁴ and R⁵ is a halogen atom is still more preferable.
As the halogen atom represented by R⁴ and R⁵, a fluorine atom or a chlorine atom is preferable.

As the halogenophenyl group, for example, a 2,3-difluorophenyl group, a 2,3-dichlorophenyl group, a 2,4-difluorophenyl group, a 2,4-dichlorophenyl group, a 2,5-difluorophenyl group, a 2,5-dichlorophenyl group, a 2,6-difluorophenyl group, a 2,6-dichlorophenyl group, a 3,4-difluorophenyl group, a 3,4-dichlorophenyl group, a 3,5-difluorophenyl group, a 3,5-dichlorophenyl group, a 2-fluorophenyl group, a 2-chlorophenyl group, a 3-fluorophenyl group, a 3-chlorophenyl group, a 4-fluorophenyl group, a 4-chlorophenyl group, a 4-chloro-2-fluorophenyl group, a 2-chloro-4-fluorophenyl group, a 4-bromo-2-fluorophenyl group, a 2,3,4-trifluorophenyl group, a 2,4,5-trifluorophenyl group, a 2,4,6-trifluorophenyl and the like are used.

As the lower (C₁₋₄) alkylphenyl group, for example, a 2-ethylphenyl group, a 2,6-diisopropylphenyl group and the like are preferably used, and as the lower (C₁₋₄) alkoxyphenyl group, for example, a 4-methoxyphenyl and the like are preferably used.

As the lower (C₁₋₄) alkoxy-carbonylphenyl group, for example, a 2-ethoxycarbonylphenyl group, a 2-methoxycarbonylphenyl group, a 4-methoxycarbonylphenyl group and the like are preferably used, and as the halogeno-lower (C₁₋₄) alkylphenyl group, for example, a 2-trifluoromethylphenyl group and the like are preferably used, and as the halogeno-lower (C₁₋₄) alkoxyphenyl group, for example, a 2-trifluoromethoxyphenyl group, a 4-(2,2,3,3,3-pentafluoropropoxy)phenyl group and the like are preferably used.

As the lower (C₁₋₄) alkanoylphenyl group, for example, a 2-acetylphenyl group and the like are preferably used, and as the 5-membered aromatic heterocyclyl-substituted phenyl group, for example, a 4-(2H-1,2,3-triazol-2-yl)phenyl group, a 4-(2H-tetrazol-2-yl)phenyl group, a 4-(1H-tetrazol-1-yl)phenyl group, a 4-(1H-1,2,3-triazol-1-yl)phenyl group and the like are preferably used, and as the lower (C₁₋₄) alkoxy-carbonyl-lower (C₁₋₄) alkyl-carbamoylphenyl group, for example, a 4-(N-ethoxycarbonylmethylcarbamoyl)phenyl group and the like are preferably used, and as the 1,3-diacylguanidino-lower (C₁₋₄) alkylphenyl group, for example, a 4-(1,3-bis-tert-butoxycarbonylguanidinomethyl)phenyl group and the like are preferably used.

As the phenyl group substituted by halogen atom and lower (C₁₋₄) alkyl group, for example, a 2-fluoro-4-methylphenyl group, a 2-chloro-4-methylphenyl group, a 4-fluoro-2-methylphenyl group and the like are preferably used, and as the phenyl group substituted by halogen atom and lower (C₁₋₄) alkoxy-carbonyl group, for example, a 2-chloro-4-methoxycarbonylphenyl group and the like are preferably used, and as the phenyl group substituted by halogen atom and cyano group, a 2-chloro-4-cyanophenyl group and the like are preferably used, and as the phenyl group substituted by halogen atom and 5-membered aromatic heterocyclic group, for example, a 2-fluoro-4-(1H-1,2,4-triazol-1-yl)phenyl group and the like are preferably used, and as the phenyl group substituted by halogen atom and lower (C₁₋₄) alkoxy-carbonyl-lower (C₁₋₄) alkyl-carbamoyl group, for example, a 2-chloro-4-(N-tert-butoxycarbonylmethylcarbamoyl)phenyl group, a 2-chloro-4-(N-ethoxycarbonylmethylcarbamoyl)phenyl group and the like are preferably used.

More specifically, as Ar, a phenyl group, a phenyl group substituted by 1 to 3 (particularly 1 or 2) halogen atoms (e.g., a 2,3-difluorophenyl group, a 2,3-dichlorophenyl group, a 2,4-difluorophenyl group, a 2,4-dichlorophenyl group, a 2,5-difluorophenyl group, a 2,5-dichlorophenyl group, a 2,6-difluorophenyl group, a 2,6-dichlorophenyl group, a 3,4-difluorophenyl group, a 3,4-dichlorophenyl group, a 3,5-difluorophenyl group, a 3,5-dichlorophenyl group, a 4-bromo-2-fluorophenyl group, a 2-fluorophenyl group, a 2-chlorophenyl group, a 3-fluorophenyl group, a 3-chlorophenyl group, a 4-fluorophenyl group, a 4-chlorophenyl group, a 2-fluoro-4-chlorophenyl group, a 2-chloro-4-fluorophenyl group, a 2,3,4-trifluorophenyl group, a 2,4,5-trifluorophenyl group etc.), a phenyl group substituted by halogen atom and lower (C₁₋₄) alkyl group (e.g., a 2-chloro-4-methylphenyl group, a 4-fluoro-2-methylphenyl group etc.), etc. are particularly preferable. Of these, a phenyl group substituted by 1 to 3 (particularly 1 or 2) halogen atoms (e.g., a 2,3-dichlorophenyl group, a 2,4-difluorophenyl group, a 2,4-dichlorophenyl group, a 2,6-dichlorophenyl group, a 2-fluorophenyl group, a 2-chlorophenyl group, a 3-chlorophenyl group, a 2-chloro-4-fluorophenyl group, a 2,4,5-trifluorophenyl group etc.), a phenyl group substituted by halogen atom and lower (C₁₋₄) alkyl group (e.g., a 2-chloro-4-methylphenyl group, a 4-fluoro-2-methylphenyl group etc.), etc. are preferable. Particularly, a 2,4-difluorophenyl group, a 2-chlorophenyl group, a 2-chloro-4-fluorophenyl group, a 2-chloro-4-methylphenyl group and the like are preferable, and a 2,4-difluorophenyl group, a 2-chloro-4-fluorophenyl group and the like are preferable.

In this specification, the ring A¹ represents a cycloalkene optionally substituted by 1 to 4 substituents selected from (i) an aliphatic hydrocarbon group optionally having substituent(s), (ii) an aromatic hydrocarbon group optionally having substituent(s), (iii) a group represented by the formula -OR¹¹ (wherein R¹¹ is a hydrogen atom or an aliphatic hydrocarbon group optionally having substituent(s)) and (iv) a halogen atom, and a cycloalkene optionally substituted by 1 to 4 substituents selected from (i) an aliphatic hydrocarbon group optionally having substituent(s), (ii) an aromatic hydrocarbon group optionally having substituent(s) and (iv) a halogen atom is preferable.

These substituents (i) - (iv) substitute on substitutable carbon atoms in the ring A¹, and when the ring A¹ is substituted by two or more of such substituents, the substituents may be the same or different. A single carbon atom may be substituted by two substituents, and different carbon atoms may be substituted by two or more substituents.

As the "aliphatic hydrocarbon group optionally having substituent(s)" as the substituent on the ring A¹, for example, those similar to the "aliphatic hydrocarbon group optionally having substituent(s)" represented by R and the like described above may be used.

As the "aromatic hydrocarbon group optionally having substituent(s)" as the substituent on the ring A¹, for example, those similar to the "aromatic hydrocarbon group optionally having substituent(s)" represented by Ar described above may be used.

As the "heterocyclic group optionally having substituent(s)" as the substituent on the ring A¹, for example, those similar to the "heterocyclic group" as the "substituent" of the aforementioned "aliphatic hydrocarbon group optionally having substituent(s)" represented by R and the like, may be used.

As the substituents for the ring A¹, 1 or 2 C₁₋₆ alkyl groups (e.g., a C₁₋₄ alkyl group such as a methyl group, a tert-butyl group, etc.), a phenyl group, a halogen atom (e.g., fluorine, chlorine, bromine, iodine atoms), etc. are preferably used.
As the integer of 1 to 4 represented by n, 1 to 3 is preferable, and 2 is particularly preferable.

As the compound represented by the formula (I), the compound represented by the formula (Ibb') is preferable, and the compound represented by the formula (Inn) is more preferable.
As the compound represented by the formula (Ibb') or the formula (Inn), a compound wherein R¹ is a lower alkyl group (more preferably R¹ is a C₁₋₆ alkyl group) optionally having substituent(s), R² is a hydrogen atom or a lower (C₁₋₆) alkyl group, Ar is a phenyl group optionally having substituent(s) (more preferably Ar is a phenyl group substituted by 1 or 2 halogen atoms) and n is 1, 2 or 3 (more preferably n is 2) is preferable.
As the compound represented by the formula (I), the compound represented by the formula (Ia):

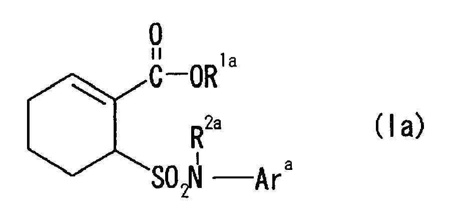

wherein R^{1a} represents a C₁₋₆ alkyl, R^{2a} represents a hydrogen atom or a C₁₋₆ alkyl and Ar^{a} represents a phenyl group substituted by 1 or 2 halogen atoms is preferable.

Specifically, as the compound represented by the formula (I), a compound obtained in Reference Example B to be mentioned below and the like is used. Among others,
(1) d-ethyl 6-[N-(2,4-difluorophenyl)sulfamoyl]-1-cyclohexene-1-carboxylate,
(2) ethyl 6-[N-(2-chlorophenyl)sulfamoyl]-1-cyclohexene-1-carboxylate,
(3) ethyl 6-[N-(2-chloro-4-methylphenyl)sulfamoyl]-1-cyclohexene-1-carboxylate, and
(4) ethyl (6R)-6-[(2-chloro-4-fluoroanilino)sulfonyl]-1-cyclohexene-1-carboxylate and salts thereof and the like are preferable.

The compound of the formula (II) is explained in detail.
As the "aliphatic hydrocarbon group optionally having substituent(s)" "aromatic hydrocarbon group optionally having substituent(s)" and "heterocyclic group optionally having substituent(s)" represented by R^{1'}, those similar to these substituents for R can be used.

As the "aliphatic hydrocarbon group optionally having substituent(s)" represented by R^{1a'}, for example, those similar to the aforementioned "aliphatic hydrocarbon group optionally having substituent(s)" represented by R can be used. As R^{1a'}, for example, lower alkyl group having 1 to 6 carbon atoms optionally having substituent(s) (e.g., methyl group, ethyl group, n-propyl group, isopropyl group, n-butyl group, isobutyl group, tert-butoxycarbonylmethyl group, hydroxyethyl group etc.) and the like are preferably used. Of these, for example, methyl group, ethyl group, n-propyl group, isopropyl group, n-butyl group, isobutyl group and the like are preferably used. Particularly, for example, methyl group, ethyl group, n-propyl group and the like are preferable, and ethyl group and the like are specifically preferable.

As the "aliphatic hydrocarbon group optionally having substituent(s)" represented by R^{1b'} and R^{1c'}, for example, those similar to the aforementioned "aliphatic hydrocarbon group optionally having substituent(s)" represented by R can be used. As R^{1b'} and R^{1c'}, for example, a lower alkyl group having 1 to 6 carbon atoms optionally having substituent(s) (e.g., methyl group, ethyl group, n-propyl group, isopropyl group, n-butyl group, isobutyl group, tert-butoxycarbonylmethyl group, hydroxyethyl group etc.) and the like are preferably used. Of these, for example, methyl group, ethyl group, n-propyl group, isopropyl group, n-butyl group, isobutyl group and the like are preferably used. Particularly, for example, methyl group, ethyl group, n-propyl group and the like are preferable, and ethyl group and the like are specifically preferable.

As R^{1'}, for example, a lower alkyl group having 1 to 6 carbon atoms optionally having substituent(s) (e.g., methyl group, ethyl group, n-propyl group, isopropyl group, n-butyl group, isobutyl group, tert-butoxycarbonylmethyl group, hydroxyethyl group etc.) and the like are preferably used. Of these, for example, methyl group, ethyl group, n-propyl group, isopropyl group, n-butyl group, isobutyl group and the like are preferably used. Particularly, for example, methyl group, ethyl group, n-propyl group and the like are preferable, and ethyl group and the like are specifically preferable.

As the "substituent" of the "methylene group optionally having substituent(s)" represented by Y, for example, C₁₋₆ alkyl group (e.g., methyl group, ethyl group, n-propyl group, isopropyl group, n-butyl group, isobutyl group etc.), hydroxy-substituted-C₁₋₆ alkyl group (e.g., hydroxymethyl group, hydroxyethyl group etc.), C₁₋₄ alkoxy-carbonyl-C₁₋₄ alkyl group (e.g., methoxycarbonylmethyl group, ethoxycarbonylmethyl group, tert-butoxycarbonylmethyl group, methoxycarbonylethyl group, ethoxycarbonylethyl group, tert-butoxycarbonylethyl group etc.) and the like can be mentioned. Of these, methyl group is preferable. Unsubstituted methylene is particularly preferable.

As the "substituent" of the "NH optionally having substituent(s)" represented by Y, C₁₋₆ alkyl group (e.g., methyl group, ethyl group, n-propyl group, isopropyl group, n-butyl group, isobutyl group etc.), hydroxy-substituted-C₁₋₆ alkyl group (e.g., hydroxymethyl group, hydroxyethyl group etc.), C₁₋₄ alkoxy-carbonyl-C₁₋₄ alkyl group (e.g., methoxycarbonylmethyl group, ethoxycarbonylmethyl group, tert-butoxycarbonylmethyl group, methoxycarbonylethyl group, ethoxycarbonylethyl group, tert-butoxycarbonylethyl group etc.) and the like can be mentioned. Of these, methyl group is preferable. Unsubstituted NH is particularly preferable.

In the "aromatic hydrocarbon group optionally having substituent(s)" represented by Ar', those similar to the "aromatic hydrocarbon group optionally having substituent(s)" for Ar can be used.
Particularly, as Ar', those similar to Ar are preferable. Among others, a group represented by the formula (c):

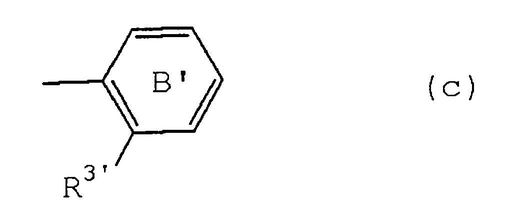

wherein R^{3'} represents a halogen atom or a lower alkyl group, and ring B' is optionally further substituted by 1 to 4 halogen atoms is preferable, and a group represented by the formula (c1):

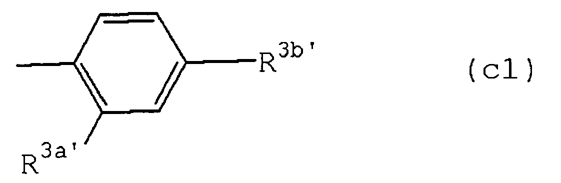

wherein R^{3a'} and R^{3b'} are the same or different and each represents a halogen atom is more preferable.

As the halogen atom represented by R^{3'} in the formula (c), halogen atom which is a substituent of ring B' in the formula (c) and the halogen atom represented by R^{3a'} and R^{3b'} in the formula (c1), fluorine atom and chlorine atom are preferable. As the lower alkyl group represented by R^{3'} in the formula (c), for example, C₁₋₄ alkyl group such as methyl, ethyl, propyl and the like can be mentioned. Of the groups represented by the formula (c), a 2,4-difluorophenyl group, a 2-chloro-4-fluorophenyl group, a 2-methyl-4-chlorophenyl group and the like are preferable. Of the groups represented by the formula (c1), a 2,4-difluorophenyl group, a 2-chloro-4-fluorophenyl group and the like are preferable.

X represents a methylene group, NH, a sulfur atom or an oxygen atom, wherein a methylene group and an oxygen atom are preferable.

Ring A' is a 5 to 8-membered ring substituted by a group represented by the formula: -CO-R^{1'} wherein R^{1'} is as defined above and a group represented by the formula: -SO₂-Y-Ar' wherein Y and Ar' are as defined above, and optionally further substituted by 1 to 4 substituents selected from the group consisting of (i) an aliphatic hydrocarbon group optionally having substituent(s), (ii) an aromatic hydrocarbon group optionally having substituent(s), (iii) a group represented by the formula: -OR^{2'} wherein R^{2'} is as defined above and (iv) a halogen atom, with preference given to a 5 to 8-membered ring optionally substituted by 1 to 4 substituents selected from (i) an aliphatic hydrocarbon group optionally having substituent(s), (ii) an aromatic hydrocarbon group optionally having substituent(s) and (iv) a halogen atom.

These substituents substitute at substitutable positions on the ring A'. When X constituting the ring is NH or a methylene group, they can substitute the NH and methylene group. When ring A' is substituted by plural substituents, the kinds of such substituents may be the same or different. In addition, two substituents may substitute on the same carbon atom.

As the "aliphatic hydrocarbon group optionally having substituent(s)" and "aromatic hydrocarbon group optionally having substituent(s)", which are substituents of ring A', for example, those similar to the aforementioned groups for R can be mentioned.

As the "aliphatic hydrocarbon group optionally having substituent(s)" for R^{2'}, for example, those similar to the aforementioned groups for R can be mentioned.

As the substituent for ring A', 1 or 2 C₁₋₆ alkyl groups (e.g., C₁₋₄ alkyl group such as methyl group, tert-butyl group etc.), phenyl groups, halogen atoms (e.g., fluorine, chlorine, bromine, iodine etc.) and the like are preferably used.

The "s" is an integer of 0 to 2, "t" is an integer of 1 to 3, and the total of "s" and "t" is not more than 4, with preference given to "s" being 1 and "t" being 1.
A group represented by the formula:

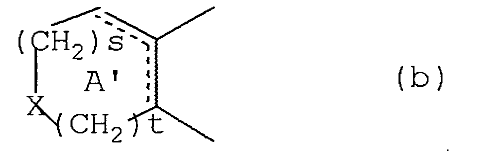

is a group represented by the formula:

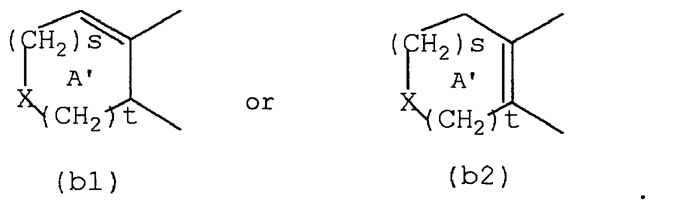

As the compound represented by the formula (II), for example, the following compounds and the like are preferable. (1) compound (II) wherein R^{1'} is a group represented by the formula: -OR^{1a'} (R^{1a'} represents a C₁₋₆ alkyl group), the group represented by the formula:

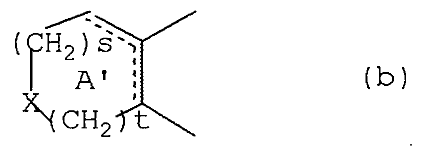

is a group represented by the formula:

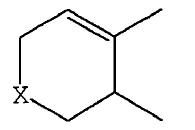

X is a methylene or an oxygen atom,
Y is a methylene or -NH-, and
Ar' is a phenyl group optionally having 1 or 2 substituents selected from a halogen atom and a C₁₋₆ alkoxy, thus, a compound represented by the formula (IIa):

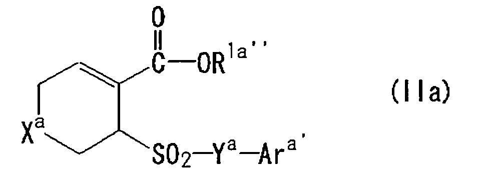

wherein R^{1a''} represents a C₁₋₆ alkyl, X^{a} represents a methylene group or an oxygen atom, Y^{a} represents a methylene group or - NH-, and Ar^{a'} represents a phenyl group optionally having 1 or 2 substituents selected from a halogen atom and a C₁₋₆ alkoxy.

(2) Compound (II) wherein R^{1'} is a group represented by the formula: -OR^{1a'} (R^{1a'} is a C₁₋₆ alkyl group),
   a group represented by the formula:

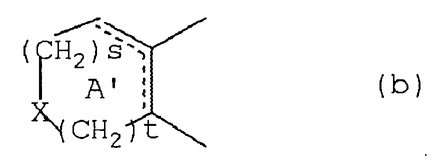

is a group represented by the formula:

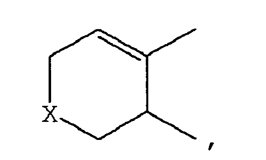

wherein X and Y are each methylene, or X is an oxygen atom and
Y is -NH-, and
Ar' is a phenyl group optionally having two halogen atoms (e.g., 2-chloro-4-fluorophenyl group etc.)

(3) Ethyl 6-(benzylsulfonyl)-1-cyclohexene-1-carboxylate (compound 1),
   ethyl 6-[(4-methoxybenzyl)sulfonyl]-1-cyclohexene-1-carboxylate (compound 2), ethyl 6-[(2,4-difluorobenzyl)sulfonyl]-1-cyclohexene-1-carboxylate (compound 3),
   ethyl 6-[(2-chloro-4-fluorobenzyl)sulfonyl]-1-cyclohexene-1-carboxylate (compound 4),
   ethyl (-)-6-[(2-chloro-4-fluorobenzyl)sulfonyl]-1-cyclohexene-1-carboxylate (compound 5),
   ethyl (+)-6-[(2-chloro-4-fluorobenzyl)sulfonyl]-1-cyclohexene-1-carboxylate (compound 6),
   ethyl 3-[(2,4-difluorophenyl)sulfamoyl]-3,6-dihydro-2H-pyran-4-carboxylate (compound 7), and
   ethyl 3-[(2-chloro-4-fluorophenyl)sulfamoyl]-3,6-dihydro-2H-pyran-4-carboxylate (compound 8).

(4) Ethyl 6-[(2-chloro-4-fluorobenzyl)sulfonyl]-1-cyclohexene-1-carboxylate (compound 4),
   ethyl (+)-6-[(2-chloro-4-fluorobenzyl)sulfonyl]-1-cyclohexene-1-carboxylate (compound 6), and
   ethyl 3-[(2-chloro-4-fluorophenyl)sulfamoyl]-3,6-dihydro-2H-pyran-4-carboxylate (compound 8).

When the compounds represented by the formulas (I) and (II) have stereoisomers, each stereoisomer and a mixture of these stereoisomers are encompassed in the present invention.
Furthermore, when the compound represented by the formula (I) is a compound represented by the formula (Icc) or (Inn), and the formula (b) of the compound represented by the formula (II) is the formula (b1) and s and t are 1, each has an optical isomer based on the asymmetric carbon in cycloalkene or cyclohexene ring. Such optical isomer and a mixture of such optical isomers are both encompassed in the present invention.

compound A used for pharmaceutical agent of the present invention, may be converted into a salt with an inorganic base, organic base, inorganic acid, organic acid, basic or acidic amino acid, and the like. As the salt with an inorganic base, for example, an alkaline metal salt such as sodium and potassium salts, etc.; an alkaline earth metal salt such as calcium and magnesium salts, etc.; aluminum salt; ammonium salt; and the like are used. As the salt with an organic base, for example, a salt with trimethylamine, triethylamine, pyridine, picoline, ethanolamine, diethanolamine, triethanolamine, dicyclohexylamine, N,N'-dibenzylethylenediamine, etc are used. As the salt with an inorganic acid, for example, a salt with hydrochloric acid, hydrobromic acid, nitric acid, sulfuric acid, phosphoric acid, etc are used. As the salt with an organic acid, for example, a salt with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, and the like are used. As the salt with a basic amino acid, for example, a salt with arginine, lysine, ornithine, etc are used. As the salt with acidic amino acid, for example, a salt with aspartic acid, glutamic acid, and the like are used.

A prodrug of compound A or a salt thereof is a compound which is converted into compound A as a result of a reaction with an enzyme, gastric acid etc. under physiological conditions in vivo. Thus, the compound is converted into compound A by enzymatical oxidation, reduction, hydrolysis etc., by hydrolysis due to gastric acid etc. A prodrug of compound A may be a compound obtained by subjecting an amino group of compound A to an acylation, alkylation or phosphorylation (e.g., a compound obtained by subjecting an amino group of compound A to an eicosanoylation, alanylation, pentylaminocarbonylation, 2-hydroxypropionylation, 2-acetoxypropionylation, (5-methyl-2-oxo-1,3-dioxolen-4-yl)methoxycarbonylation, tetrahydrofuranylation, pyrrolidylmethylation, pivaloyloxymethylation, tert-butylation, etc.); a compound obtained by subjecting a hydroxy group of compound A to an acylation, alkylation, phosphorylation and boration (e.g., a compound obtained by subjecting a hydroxy group of compound A to an acetylation, palmitoylation, propanoylation, pivaloylation, succinylation, fumarylation, alanylation, dimethylaminomethylcarbonylation, etc.); a compound obtained by subjecting a carboxyl group of compound A to an esterification or amidation (e.g., a compound obtained by subjecting a carboxyl group of compound A to an ethyl-esterification, phenyl-esterification, carboxymethyl-esterification, dimethylaminomethyl-esterification, pivaloyloxymethyl-esterification, ethoxycarbonyloxyethyl-esterification, phthalidyl-esterification, (5-methyl-2-oxo-1,3-dioxolen-4-yl)methyl-esterification, cyclohexyloxycarbonylethyl-esterification and methylamidation, etc.) and the like. Any of these compounds can be produced from compound A by a method known per se.
A prodrug of compound A may also be one which is converted into compound A under a physiological condition, such as those described in "IYAKUHIN no KAIHATSU (Development of Pharmaceuticals)", Vol.7, Design of Molecules, p. 163-198, Published by HIROKAWA SHOTEN (1990).

The compound (I), a salt thereof and a prodrug thereof can be produced according to a method known *per se,* for example, a production method described in WO99/46242 or a method analogous thereto. The compound (II), a salt thereof and a prodrug thereof can be produced according to a production method described in WO01/10826 or a method analogous thereto.

When the optically active compound or a salt thereof contains an enantiomer, general separation means may be applied such as diastereomeric salt methods wherein a salt with an optically active acid (e.g., camphor sulfonic acid etc.) or optically active base (e.g., 1-methylbenzylamine etc.) is formed, inclusion compound methods using an optically active host molecule (e.g., 1,6-bis(2-chlorophenyl)-1,6-diphenylhexa-2,4-diyn-1,6-diol), various chromatographies (e.g., liquid chromatography using a chiral column etc.), fractional recrystallization and the like, whereby an optically pure compound can be obtained.

The compound A or a salt thereof or a prodrug thereof (hereinafter to be comprehensively referred to as compound A) may be a solvate or a non-solvate.
In addition, compound A may be labeled with an isotope (e.g., ³H, ¹⁴C, ³⁵S, ¹²⁵I etc.) and the like.

A TLR signaling inhibitory substance containing a cycloalkene compound and compound A in the present invention is highly safe for the human body, and used for mammals (e.g., rat, mouse, guinea pig, monkey, bovine, dog, swine, human etc.) as a pharmaceutical agent (e.g., an agent for the prophylaxis or treatment of various diseases), animal drug and the like.

A TLR signaling inhibitory substance in the present invention is low toxic, and useful as an agent for suppressing the production or expression and the like of a factor selected from IL(Interleukin)-2, IL-3, IL-8, IL-10, IL-12, IL-17, MIP(macrophage inflammatory protein)-2, KC (keratinocyte derived-chemokine), GM-CSF (granulocyte-macrophage colony-stimulating factor), IFN(interferon)-γ and prostaglandin E2 and the like. A TLR signaling inhibitory substance in the present invention may suppress production or expression of one kind of these factors, or may suppress two or more kinds thereof.

In addition, a TLR signaling inhibitory substance in the present invention is low toxic, and useful as an phosphorylation enzyme inhibitor (e.g., activity inhibitor, production suppressive agent, expression suppressive agent) and the like of phosphorylation enzyme selected from ATF2 (Activated transcription factor 2), JNK (c-Jun N-terminal kinase), p38MAP kinase (mitogen-activated protein kinase), IκBα, ERK1/2, p90RSK, STAT2 (Signal transducer and activator of transcription 2) and p70 S6 kinase and the like. A TLR signaling inhibitory substance in the present invention may inhibit one kind of these phosphorylation enzymes, or two or more kinds thereof.

Furthermore, a TLR signaling inhibitory substance in the present invention is low toxic, and useful as an agent for suppressing the expression of COX-II (cyclooxygenase-II), IL-10, MCP (monocyte chemoattractant protein)-1/3, MIP-1α, RANTES (regulated on activation normal T cell expressed and secreted), plasminogen activator inhibitor, G-CSF (granulocyte colony-stimulating factor), Fas antigen, MIF(migration inhibition factor), MMP (matrix metalloproteinase)-9, A20, Adenisine A2b receptor, argininosuccinate synthetase, bcl-3, Caspase-11, CCR1(chemokine receptor 1), Cytokine inducible SH-2-containing protein, Fc receptor, Galectin-9, Guanylate binding protein-2, histidine decarboxylase, IL-1β, IL-1 receptor antagonist, IL-18, IL-4 receptor, IL-6, inducible NO synthase (inducible nitric oxide synthase), Interferon regulatory factor, IP-10(interferon-γ inducible protein-10), JAK2 (Janus kinase 2), MHC (major histocompatibility complex), MCP-3, MIP-2, P2X4, phosphorylation enzyme PAC1 (phosphatase PAC1), serum amyloid A, TNF receptor, TNF receptor associated factor and the like, particularly COX-II, IL-10, MCP-1/3, MIP-2, RANTES, P2X4, plasminogen activator inhibitor, G-CSF, Fas antigen, TNF receptor, Fc receptor, galectin-9, histidine decarboxylase, IL-1 receptor antagonist, MMP-9 or the like. A TLR signaling inhibitory substance in the present invention may suppress expression of one kind of these, or two or more kinds thereof.

Moreover, a TLR signaling inhibitory substance in the present invention is low toxic, and useful as an inhibitor or drug for suppressing the induction of, for example, apoptosis, cell death receptor (Death receptor), Akt/PKB (Protein kinase B), MAP kinase (Mitogen-activated protein kinase), G-protein, Cell cycle control, transcription control (Translation control), Protein acetylation, Proteasome, Wnt/β-Catenin, Cytoskeletal, Insulin receptor, Erb/Her, B-cell receptor, T-cell receptor, NF-κB (nuclear factor-κ B), TGF-β (transforming growth factor-beta), Jak/Stat (Janus kinase/signal transducer and activator of transcription), Cyclooxygenase, Lipoxygenase, eIF4E binding protein, Ableson protein tyrosine kinase, Adenylate cyclase A (Adenylate cyclase), histone acetyltransferase (A histone acetyltransferase), ADAP (Adhesion and degranulation adaptor protein), Antigen, Programmed cell death protein 8, AKT8 virus oncogene cellular homolog, AMPK (AMP-activated protein kinase), Adenine nucleotide translocation channel, AP-1 (Activator protein 1) (c-jun and c-fos), APAF-1 (Apoptotic protease activating factor 1), APC (Adenomatous polyposis coli), β-APP (β-Amyloid precursor protein), APP (Acute phase proteins), APS (Adapter protein with a PH and SH2 domain), ASP (Agouti switch protein), ASK(Apoptosis signal-regulating kinase) (e.g., ASK1), Atf2 (Activating transcription factor 2), ATM (Ataxia telangiectasia-mutated protein kinase), ATR (ATM and Rad3-related protein kinase), BAFF/ BLyS (B cell activating factor of TNF family/ BLyS), BAM32 (B-cell adaptor molecule 32 kDa), BCAP (B-cell adaptor for PI3K), Bc110 (B-cell leukemia 10 protein), Bcl-2-related protein A1, BID (A BH3 domain-only death agonist protein), Blimp-1 (B-lymphocyte-induced maturation protein 1), BLyS/BAFF (B lymphocyte
stimulator/BAFF), BR3/BAFF-R (BLyS receptor 3/BAFF-R), BRCA (Breast cancer growth suppressor protein), Btk (Bruton's tyrosine kinase), β-TrCP (β-transducin repeat-containing protein), GRF2 (Guanine nucleotide-releasing factor 2), CAD (Caspase-activated deoxyribonuclease), Calmodulin, CaMK (Calcium/calmodulin-dependent kinase), cAMP (Cyclic adenosine monophosphate), CAP (c-Cbl-associated protein), p130CAS (Crk-associated substrate), Caspases (Cysteine proteases with aspartate specificity), Caveolin, Cellular homologue of the v-Cbl oncogene, CBP (CREB binding protein), CDC (Cell division control protein), CDK1 (cyclin-dependent kinase 1), CDK1 (Cyclin-dependent kinase 1), ubiquinone conjugated (E2) enzyme (ubiquitin conjugating (E2) enzyme), A kinase-binding chaperone, Small GTPase of Rho family, CDK (Cyclin-dependent kinase), CHK (Checkpoint kinase), CHOP-10 (C/EBP homologous protein 10), c-IAP (Cellular inhibitor of apoptosis protein), Cip (CDK-interacting protein), CIS (Cytokine inducible SH2-containing protein), Casein kinase, cellular homologue of v-myB (Cellular homologue of avian myeloblastosis virus oncogene), cellular homologue of c-myc (Cellular homologue of avian myelocytomatosis virus oncogene), COT (Cancer osaka thyroid), a S/T kinase, cPLA2 (Cytoplasmic phospholipase A2), CREB (cAMP response element-binding protein), CT10 sarcoma oncogene cellular homolog, CTMP (Carboxyl-terminal modulator protein), Diacylglycerol, DAP kinase (Death-associated protein kinase), Daxx (Fas death domain-associated protein), DIABLO (Direct IAP binding protein with low pI), DAP kinase (DNA-activated protein kinase), Member of the E2F transcription factor family, DCP4 (Deleted in pancreatic cancer locus 4)(Smad4), cell death receptor (Death receptor), Dishevelled, Transcription factor family including E2F- and DP-like subunits, eEF (Eukaryotic elongation factor), cell death receptor 3 (Death receptor 3), Double-stranded RNA, EF (Eukaryotic elongation factor), EGR1 (Early growth response protein 1), eIF (Eukaryotic initiation factor), Ets domain protein, ENaC (Epithelial sodium channel), Epac (Exchange protein activated by cAMP), Endoplasmic reticulum, Estrogen receptor, ERK (Extracellular signal-regulated kinase), C-ets-1 protein, a transcription factor, FADD (Fas-associated protein with death domain), FAK (Focal adhesion kinase), Fc-γ RII-b (Immunoglobulin γ Fc region receptor II-B), FKHR (Forkhead in rhabdomyosarcoma), FLIP (FLICE (Caspase-8) inhibitory protein), FRAP (FKBP12-rapamycin-associated protein), FRS2 (Lipid anchored Grb2 binding protein activated by FGF receptor), A Src family proto-oncogene tyrosine-protein kinase, Gab1 (GRB2-associated binder-1), GADD34 (Growth arrest and DNA damage protein 34), GADD45 (Growth arrest and DNA damage protein 45), GAPs (GTPase activating proteins), IFNγ-activated sequences, gas gene 2 (Growth arrest-specific gene 2), GCK (Germinal center kinase), GCS2 (General control of amino acid biosynthesis protein 2), an S/T kinase, GCS5 (General control of amino acid biosynthesis protein 5), GEF (Guanine nucleotide exchange factor), Glucose transporter type 4, GPCRs (G-protein coupled receptor), Grb2 (Growth factor receptor-bound protein 2), Grb10 (Growth factor receptor-bound protein 10), GRIP (Glucocorticoid receptor interacting protein), GRK (G-protein coupled receptor kinase), GSK-3β (Glycogen synthase kinase-3β), Histone deacetylase, HMG (High mobility group), HPK (Hematopoietic progenitor kinase), HRI (Hemin-regulated inhibitor), HRK protein (Harakiri protein), Hrk (an activator of apoptosis), HSP27 (Heat shock protein 27), IAP (Inhibitor of apoptosis), ICAD (Inhibitor of caspase-activated deoxyribonuclease), NF-κB inhibitory factor (Inhibitor of NF-κB), IκB kinase, CDK4 inhibitory factor (Inhibitor of CDK4), IRS (Insulin receptor substrate) (e.g., IRS-1), ISRE (Interferon-stimulated response element), JAK (Janus-family tyrosine kinase), JIP1 (JNK interacting protein 1), JNK (Jun N-terminal kinase), KSR (Kinase suppressor of Ras), DLC (Dynein light chain, cytoplasmic), DSP (Dual-specificity phosphatase), MALT1 (Mucosa-associated lymphoma translocation protein 1), MAPKAPK-2 (MAP kinase activated protein kinase 2), MDM2 (Murine double minute 2), a p53-associated oncogene, MEF-2 (Myocyte enhancer factor 2), MAPK/Erk kinase, MAPK/Erk kinase kinase, mIg (Membrane immunoglobulin), MKP (MAP kinase phosphatase), MLK (Mixed lineage kinase), MNK (MAP kinase interacting kinase), MP1 (MEK partner 1), MSK1 (Mitogen and stress activated kinase 1), mTOR (Mammalian target of rapamycin), MDF (Myogenic determination factor), DYRK (A dual-specificity protein kinase), Nck adaptor protein, NCoR (Nuclear receptor corepressor), NF-AT (Nuclear factor of activated T-cells), NFκB (Nuclear factor κ B), NIK (Nuclear factor κ B induced kinase), Damagei protein, apoptosis inducible BH3 containing protein (a proapoptotic BH3-containing protein), Oct-2 (Octamer-binding transcription factor 2), p19ARF (p19 alternative reading frame protein), TS (a tumor suppressor), A histone acetyltransferase, cancer suppressor protein (Tumor suppressor protein that protects from DNA damage), RSK (90 kDa ribosomal S6 kinase), PABP (Poly(A) tail-binding protein), PAIP1 and 2 (Polyadenylate binding protein interacting proteins 1 and 2), PARP (Poly(ADP-ribose)polymerase), Paxillin, PCAF (p300/CBP-associated factor), p/CIP (p300/CBP-interacting protein), CGI-PDE B (cGMP-inhibited 3',5'-cyclic phosphodiesterase B), PDK (3-phosphoinositide-dependent protein kinase), PEK (Pancreatic eukaryotic initiation factor 2α-subunit kinase), PERK (Type I transmembrane ER-resident protein kinase), PI3K (Phosphoinositide 3 kinase), PIAS (Protein inhibitors of activated Stats), PtdIns(3,4)P2 (Phosphatidylinositol 3,4-bisphosphate), PtdIns(3,4,5)P3 (Phosphatidylinositol 3,4,5-trisphosphate), PKA (Protein kinase A), PKC (Protein kinase C), PKR (dsRNA-dependent serine/threonine protein kinase), PLC-β (Phospholipase C β), PLC-γ (Phospholipase C γ), Plk-1 (Polo-like kinase 1), PLA2 (Phospholipase 2A), Phospho-protein phosphatase 1, Phospho-protein phosphatase 2A, PPARγ (Peroxisome proliferator-activated receptor γ), PR (Progesterone receptor), PRAK (p38 regulated activated kinase), PRK2 (Protein kinase C-related kinase 2), PTEN (Phosphatase and tensin homologue deleted on chromosome 10), PUMA (p53 upregulated modulator of apoptosis), PYK2 (Proline-rich tyrosine kinase 2), RAIDD (RIP-associated ICH/CED-3-homologous protein with a death domain), Ras-related protein RAP-1A, RasGRP (Ras guanyl nucleotide releasing protein), pRB (Retinoblastoma protein), TS (a tumor suppressor), RGS (Regulator of G-protein signaling), RIP (Receptor-interacting protein), ROCK (Rho-associated, coiled-coil-containing protein kinase), RTK (Receptor tyrosine kinase), RXR (Retinoid X receptor), SSU ribosomal protein S6 (Small subunit ribosomal protein S6), SAPK (Stress-activated protein kinase), SCF (Skp-cdc53-F-box ubiquitin ligase complex), SGK (Serum/glucocorticoid-regulated kinase), Shc (SH2-containing collagen-related proteins), SHP-1 (SH2-containing phosphatase 1), SHP-2 (SH2-containing phosphatase 2), A transcriptional corepressor, SKP2 (S-phase kinase associated protein 2), SMAC (Second mitochondria-derived activator of caspase), Smad (Contraction of Sma and Mad (Mothers against decapentaplegic)), SMRT (Silencing mediator of retinoic acid and thyroid hormone receptor), SNAREs (Soluble N-ethylmaleimide attachment protein receptors), SOCS (Suppressor of cytokine signalling), Sos (Son of sevenless) GEF (guanine nucleotide exchange factor), SRC-1 (Steroid receptor coactivator 1), SRF (Serum response factor), STAT (Signal transducer and activator of transcription), Tyrosine-protein kinase Syk, Synip (Syntaxin 4 interacting protein), TAB (TAK1 binding protein), TAFs (TBP-associated factors), TAK (TGF β activated kinase), Talin, tBid (Truncated Bid), TBP (TATA-binding protein), GTP-binding protein TC10, ThF (T-cell factor), TF (Transcription factor), TH (Tyrosine hydroxylase), Tip (HIV-1-Tat interactive protein), TNF (Tumor necrosis factor), TR (Thyroid hormone receptor), TRADD (TNF receptor-1-associated death domain protein), TRAF (TNF receptor-associated factor 2), The onc F proto-oncogene, ABL, ACTR2B, AKT1, AKT2, ALK, ALK1 (ACVRL1), ANPα (NPR1), ARG (ABL2), ATM, ATR, AurA, AurB, Axl, BCR, BMPR1A, BMPR2, B-RAF, BRD4, BRK, BTK, BUB1, BUBR1 (BUB1B), CDC2 (CDK1), CDK2, CDK4, CDK6, CDK9, CHK1, CHK2, CK1δ and CK1α, CK1ε (CSNK1ε), CK2α1 and CK2α2 (CSNK2α1/2), COT/TPL2, CTK/MATK, CYGD (GUCY2D), CYGF (GUCY2F), DAPK1, DMPK1, DNAPK, Dyrk1A, eEF2K (CaMKIII), EGFR, Eph family, EphA1, EphA2, EphA3 (HEK), EphB2, EphB4 (HTK), Erk5 (BMK1), FAK (PTK2), FER, FES, FGFR1, FGFR2, FGFR3, FGFR4, FGR, FLT1 (VEGFR1), FLT3, FLT4 (VEGFR3), FMS (CSF1R), FRAP (mTOR), FYN, GSK3α and GSK3β, Her2 (ErbB2), Her3 (ErbB3), Her4 (ErbB4), HGK (ZC1), HIPK1, HIPK2, IGF1R, IKKα, IKKβ, ILK, INSR, IRAK2, IRAK4, JAK1, JAK2, JAK3, JNK1, JNK3, KDR (FLK1, VEGFR2), KIT, LATS1, LATS2, LCK, LIMK1, LKB1 (STK11), LYN, MEK1,2, MER, MET, MISR2 (AMHR2), MKK3 (MAP2K3), MKK4 (MAP2K4), skMLCK (MYLK2), MLK4, MST4, MYO3A, NEK1, NEK2, NEK8, p38 (α,β,γ,δ), PAK3, PAK4, PDGFRα, PDGFRβ, PEK (PERK), PHKγ2, PIM1, PIM2, PIM3, PINK1, PKCα, PKCβ, PKCδ, PKCε, PKCη, PKCγ, PKCθ, PKR (PRKR), PLK1, PRKX, PRKY, RAF1 (c-Raf), RET, RHOK, RNAseL, ROCK1, ROCK2, RON, ROR2, ROS, p70S6K (RPS6KB1), RSK2 (RPS6KA3), SGK1, SRC, SYK, TGFβR1 (ALK5), TGFβR2, TIE2 (TEK), TRKA (NTRK1), TRKB (NTRK2), TRKC (NTRK3), TYK2, TYRO3 (SKY), WNK1, WNK4, YES, ZAP70, CCL5, CCL15/Leukotactin, CCL22, CCL28, CINC-1, CXCL 11, Eotaxin, Fractalkine, Gro α-γ, Gro-β and -γ, Gro-1, ICOS, IFN-γ, IL-1a, IL-1b, IL-1 receptor antagonist, IL-2, IL-6, IL-8, IL-9, IL-10, IL-11, IL-12 (p40), IL-13, IL-15, β-Interferon" KC, LIX (mouse), ENA-78 (CXCL5) and GCP-2 (CXCL6) (human), LTA (Lymphotoxin α), LTB (Lymphotoxinβ), MCP-1/JE, MIP-1α,β, aka (LAG-1), MIP-2, MIP-3α/CCL20, mob-1, ENA-78(Neutrophil activating peptide-78), RANTES, TCA3, T-cell activation gene 3, TNFα, TNFβ, TRAIL (aka Apo2 ligand), TFF3 (Treefoil factor), VEGI, Immunoreceptors, B7.1 (CD80), BRL-1, CCR5, CCR7, CD137, CD154, CD3γ, CD86, CD23 (Fc epsilon receptor II), ICOS, IL-2 receptor α-chain, Immunoglobulin Cγ1, Immunoglobulin γ1, Immunoglobulin γ4, Immunoglobulin heavy chain, Immunoglobulin k light chain, Invariant Chain II, Kinin B1 Receptor, MHC class I (H-2Kb), MHC Class I HLA-B7, β2 Microglobulin, Nod2, pIgR (Polymeric Ig receptor), T-cell receptor β chain, T-cell receptor/CD3γ, TLR-2, TLR9, TNF-Receptor, p75/80 (CD120B), Proteins involved in antigen presentation Complement B, Complement component 3, Complement Receptor 2, Proteasome Subunit LMP2, Peptide Transporter TAP1, Tapasin, Cell adhesion molecules, DC-SIGN, ELAM-1 (CD62E, E-selectin), Endoglin, Fibronectin, ICAM-1, MadCAM-1, P-selectin, Tenascin-C, VCAM-1, APP (Acute phase proteins), Angiotensinogen, β-defensin-2, C4b binding protein, Complement factor B, Complement factor C4, CRP (C-reactive protein), LBP (Lipopolysaccharide binding protein), Pentraxin PTX3, SAAs (Serum amyloid A proteins) (SAA1, SAA2, SAA3), Tissue factor-1, u-PA(Urokinase-type plasminogen activator), Stress response genes, Angiotensin II, CYP2E1, CYP2C11, COX-2, Ferritin H chain, 5-Lipoxygenase (guinea pig), 12-Lipoxygenase, iNOS (Inducible NO-Synthase), MAP4K1, Cu/Zn SOD, Mn SOD, NQO1(NAD(P)H quinone oxidoreductase (DT-diaphorase)), Phospholipase A2, A1 adenosine receptor, ADAM19, Amiloride-sensitive sodium channel, α2B-adrenergic receptor, Bradykinin B1-Receptor, CD23, CD69, EGFR (Epidermal Growth Factor Receptor), ErbB2, Gall Receptor, Lox-1, Mdr1, µ-opioid receptor, Neuropeptide Y-Y1 receptor, NMDA receptor subunit 2A (NMDA receptor subunit 2A (rat)), NMDA receptor subunit NR-1 (GRIN1 gene), PAF receptor 1, RAGE (receptor for advanced glycation end products), Regulators of apoptosis, Bax, Bf11/A1, Bcl-xL, Bcl-2, B7-H1, Caspase-11, Nr13, c-FLIP, CD95 (Fas), FAP-1(Fas-associated phosphatase-1), Fas-Ligand, IAPs, IEX-1L, TRAF-1, TRAF-2, T2BP(TRAF-2 binding protein) (Carp), Growth factors, ligands, and their modulators, Angiopoietin, BMP-2, G-CSF, GM-CSF, HGF/SF, EPO, IGFBP-1, IGFBP-2, M-CSF (CSF-1), NK-1R, NK4, PDGF B chain, PlGF, Proenkephalin, Prolactin, Stem Cell Factor, Thrombospondin-1 (TSP-1), Thrombospondin-2 (THBS2), VEGF C, ERGs(Early response genes), B94, Egr-1, p22/PRG1, p62, TIEG, Transcription factors, A20, Androgen receptor, CDX1, c-fos (fish gene), c-myb, c-myc, c-rel, E2F3a, Elf3, ELYS, ETR101, Gata-3, Glucocorticoid receptor, IRF-1, IRF-2, IRF-4, IRF-7, IkB-α, IkB-ε, junB, Mail, nfkb2, nfkb1, NURR1, p53, relb, Snail, Sox9, Stat5a, Tfec, WT1, Adenovirus (E3 region), Avian Leukosis Virus, Bovine Leukemia Virus, CMV, EBV (Wp promoter), HBV (pregenomic promoter), HIV-1, HSV, JC Virus, HPV type 16, SIV, SV-40, ABC Transporters, N-acetylglucosaminyltransferase I (rat gene), ADH, ARP1 (ARF-related protein-1), Argininosuccinate synthetase, Aromatase (promoter II), α1ACT, BACE, Cathepsin B, Cathepsin L, Ceramide glycosyltransferase, Chitinase 3-like protein, CRAD1 (cis-retinoid/androgen dehydrogenase type 1), CRAD2 (cis-retinoid/androgen dehydrogenase type 2), Collagenase 1, Cu/Zn-SOD, Dihydrodiol dehydrogenase, DYPD, DNASIL2, EL, ENO2, GAD67, GD3-synthase, Gelatinase B, GSTP1-1, GCL (Glutamate-cysteine ligase), GCLC, GCLM (Glutamate-cysteine ligase modifier), γ glutamylcysteine synthetase, Glucose 1-6-phosphate dehydrogenase, sGCα(1) (Soluble Guanylyl cyclaseα(1)), Heparanase, HO-1, Hyaluronan synthase, 11bHSD2, H⁺-K⁺ATPase α2, Iodothyronine deiodinase (type 2), L-PGDS (Lipocalin-type prostaglandin D synthase), Lysozyme, Mthfr, MKP-1, MMP-3(matrix metalloproteinaase-3), MMP-9 (matrix metalloproteinase-9), iNOS, PDE7A1, PIM-1, Plk3, PP5, PKC delta, PLC delta 1, PTGIS, prostaglandin synthase, PGES, prostaglandin E synthase, RACK1, REV3, Serpin 2A, SIAT1, SNARK, SUV3, TERT (mouse), Transglutaminase, type II-sPLA2 (Type II-secreted phospholipase A2), Xanthine Dehydrogenase, AβH-J-J, α-1-AGP (α-1 acid glycoprotein), α-fetoprotein, AMH, β-amyloid, Apolipoprotein C III, Apolipoprotein E, Biglycan, BRCA2, Caveolin-1, Clone 330, Clone 156, Clone 68, p21-CIP1, Claudin-2, α2(I) collagen, Connexin32, Cyclin D1, Cyclin D2, Cyclin D3, DMT1, Endothelin 1, Ephrin-A1, Factor VIII, Ferritin Heavy Chain, Gadd45β, Galpha i2, GIF, Galectin 3, GBP-1, ε-Globin, GS3686, HMG14, K3 Keratin, K6 Keratin, K15 Keratin, Lactoferrin, Laminin B2 Chain, Mts1, MNE1, Mucin (MUC-2), Mx1 (bovine), Naf1, NGAL (Neutrophil gelatinase-associated lipocalin), NLF1, p11, PA28, PAI-1, Pax8, PCBD, Perforin, PGK1, POMC, rnCGM3 (Pregnancy-specific glycoprotein), Prodynorphin, PSA (Prostate-specific antigen), RAG-1, RAG-2, RICK, S100A6 (calcyclin), SH3BGRL, Spergen-1, SWS1, Syndecan-4, TAUT, TASK-2, TFPI-2 (Tissue factor pathway inhibitor-2), transferrin (Transferrin (mosquito)), TRIF, UBE2M, UCP-2, Uroplakin Ib, CYP27B1 (25-hydroxyvitamin D3 1α hydroxylase), Vimentin, α1-antitrypsin, radicals (e.g., oxide radicals, NO (nitric oxide), carbon oxide), Lipid mediators (e.g., Prostaglandins, Tromboxanes, Leukotrienes, Ceramides) and the like.

Therefore, a TLR signaling inhibitory substance in the present invention can also be used as an agent for the prophylaxis or treatment of, for example, diseases such as infectious disease, cardiac disease, autoimmune diseases, inflammatory disease, central nervous system diseases, immune hypofunction and the like, sepsis including severe sepsis, septic shock, sepsis, endotoxin shock, exotoxin shock, systemic inflammation reaction syndrome (SIRS), compensatory anti-inflammatory reaction syndrome (CARS), burn injury, trauma, postoperative complications, cardiac failure, shock, hypotension, rheumatism arthritis, osteoarthritis, gastritis, ulcerative colitis, peptic ulcer, stress-related gastric ulcer, Crohn's disease, autoimmune diseases, tissue disorder and rejection after organ transplantation, ischemia-reperfusion injury, acute coronary microvascular embolus, vascular embolus resulting from shock (disseminated intravascular coagulation (DIC) and the like), ischemic brain disorder, arteriosclerosis, pernicious anemia, Fanconi anemia, sickle cell anemia, pancreatitis, nephrosis syndrome, nephritis, renal failure, insulin-dependent diabetes mellitus, insulin-independent diabetes mellitus, hepatic porphyria, alcohol poisoning, Parkinson's disease, chronic leukemia, acute leukemia, tumor, myeloma, infant and adult respiratory distress syndrome, emphysema, dementia, Alzheimer's disease, multiple sclerosis, vitamin E deficiency, aging, sunburn, muscular dystrophy, myocarditis, cardiomyopathy, myocardial infarction, myocardial infarction sequelae, osteoporosis, pneumonia, hepatitis, psoriasis, pain, cataract, influenza infections, malaria, human immunodeficiency virus (HIV) infections, radiation disorder, burn, hypercalcemia, ankylosing spondylitis, osteopenia, bone Paget's disease, osteomalacia, bone fracture, acute bacterial meningitis, Helicobacter pylori infections, Invasive Staphylococcus aureus infections, tuberculosis, systemic fungal infectious diseases, simple herpes virus infections, varicella-zoster virus infections, human papilloma virus infections, acute virus encephalitis, encephalitis, meningitis, immune hypofunction due to infections, asthma, atopic dermatitis, allergic rhinitis, erosive esophagitis, fever, hypercholesterolemia, hyperglyceridemia, hyperlipidemia, diabetic complications, diabetic nephropathy, diabetic neuropathy, diabetic retinopathy, gout, stomach atony, hemorrhoid, systemic lupus erythematosus, spinal trauma, insomnia, schizophrenia, epilepsy, cirrhosis, hepatic failure, unstable angina pectoris, cardiac valvular disease, thrombocytopenia or hypotension due to dialysis, acute ischemic cerebral apoplexy, acute brain thrombosis, cancer metastasis, urinary bladder cancer, breast cancer, cervical cancer, colorectal cancer, gastric cancer, ovarian cancer, prostate cancer, small cell lung cancer, non-small cell lung cancer, malignant melanoma, Hodgkin's disease, non-Hodgkin's lymphoma, side effect caused by administration of anti-cancer agent or immunosuppressant, chronic obliterative pulmonary diseases, cystic fibrosis, lung fibrosis, autoimmune hemolytic anemia, meningitis, inflammatory pulmonary disease (e.g., silicosis, lung sarcoidosis, pulmonary tuberculosis), endometriosis, cachexia (e.g., cachexia derived from infection, carcinocachexia, cachexia induced by acquired immunodeficiency syndrome), cancer pain, Addison's disease, acute pain caused by inflammation, pain due to chronic inflammation, postoperative pain (incision pain, deep pain, visceral pain, postoperative chronic pain and the like), muscular pain (muscular pain associated with chronic pain disease, stiff neck and the like), arthritic pain, toothache, pain of temporomandibular joint, headache (migraine, tension headache, headache caused by fever, headache associated with hypertension), visceral pain (cardiac pain, anginal pain, abdominal pain, kidney pain, ureteral pain, cystalgia, obstetric and gynecologic pain(intermenstrual pain, dysmenorrheal, labor pain), neuralgia (disc hernia, radicular pain, post herpetic neuralgia pain, trigeminal neuralgia pain), reflex sympathetic atrophy, complex topalgia syndrome, pituitary gland abscess, thyroiditis, peritonitis, erythema nodosum), allergic conjunctivitis, pollinosis, metal allergy, exudative otitis media, Meniere's disease, contact dermatitis, anaphylaxis, urticaria, myasthenia gravis, Sjogren's syndrome, Basedow's disease, leukocyte abnormality, renal tubulointerstitial injury (including fibrotic pathology), acute coronary artery syndrome, atherosclerotic aortic aneurysm, heart anaphylaxis, deep-vein thrombosis, ophthalmic disorder (e.g., pterygium, spring catarrh, dry eye and the like), food allergy, NUD (Non Ulcer Dyspepsia), stomach MALT lymphoma, ulcer due to non-steroidal anti-inflammatory agent, hyperacidity, hyperacidity and ulcer caused by postoperative stress, obesity, edema, granuloma, atopic myelitis, neurofibromatosis, nasal mucosal sensitivity and the like. In addition, the TLR signaling inhibitory substance in the present invention can also be used for efficient external fertilization.

A TLR signaling inhibitory substance can be used in combination with other drugs. Examples of the combination drugs include antibacterial agents, antifungal agents, non-steroidal antiinflammatory drugs, steroids, anticoagulants, antithrombotic drugs, thrombolytic drugs, immunomodulators, antiprotozoals, antitussive and expectorant drugs, sedatives, anesthetics, antinarcotics, antiulcer drugs, hyperlipidemia treating agents, therapeutic agents for arteriosclerosis, HDL increasing agents, unstable plaque stabilizing agents, myocardial protecting agent, hypothyroidism treating agent, nephrotic syndrome treating agent, chronic renal failure treating agent, diuretics, hypertension treating agents, cardiac failure treating agents, muscle relaxants, anticonvulsants, cardiacs, vasodilators, vasoconstrictors, antiarrhythmics, antidiabetic drugs, hypertensors, tranquilizers, antipsychotics, therapeutic agents for Alzheimer's diseases, anti-Parkinson drugs, therapeutic agents for amyotrophic spinal lateral sclerosis, neurotrophic factors, antidepressants, therapeutic agents for schizophrenia, antitumor drugs, vitamins, vitamin derivatives, therapeutic agents for arthritis, antirheumatics, antiallergic drugs, antiasthmatics, therapeutic agents for atopic dermatitis, therapeutic agents for allergic rhinitis, therapeutic agents for pollakisuria/anischuria, protease drugs, protease inhibitors, anti-SIDS drugs, anti-sepsis drugs, anti-septic shock drugs, endotoxin-antagonists or -antibodies, signal transduction inhibitors, inhibitors of inflammatory mediator activity, antibodies to inhibit inflammatory mediator activity, inhibitors of inflammatory mediator production, inhibitors of anti-inflammatory mediator activity, antibodies to inhibit anti-inflammatory mediator activity, inhibitors of anti-inflammatory mediator production, α1-adrenergic agonists and the like. Of these, antibacterial agents, antifungal agents, non-steroidal antiinflammatory drugs, steroids, anticoagulants, antiemetic, inhibitor of methemoglobin increase and the like are preferable. Specific examples thereof include the following.

(1) Antibacterial agents
   (i) Sulfa drugs
      sulfamethizole, sulfisoxazole, sulfamonomethoxine, sulfamethizole, salazosulfapyridine, silver sulfadiazine and the like.
   (ii) Quinoline antibacterial agents
      nalidixic acid, pipemidic acid trihydrate, enoxacin, norfloxacin, ofloxacin, tosufloxacin tosilate, ciprofloxacin hydrochloride, lomefloxacin hydrochloride, sparfloxacin, fleroxacin and the like.
   (iii) Antiphthisics
      isoniazid, ethambutol (ethambutol hydrochloride), p-aminosalicylic acid (calcium p-aminosalicylate), pyrazinamide, ethionamide, protionamide, rifampicin, streptomycin sulfate, kanamycin sulfate, cycloserine and the like.
   (iv) Antiacidfast bacterium drugs
      diaphenylsulfone, rifampicin and the like.
   (v) Antiviral drugs
      idoxuridine, acyclovir, vidarabine, ganciclovir and the like.
   (vi) Anti-HIV agents
      zidovudine, didanosine, zalcitabine, indinavir sulfate ethanolate, ritonavir and the like.
   (vii) Antispirocheteles
   (viii) Antibiotics
      tetracycline hydrochloride, ampicillin, piperacillin, gentamicin, dibekacin, kanendomycin, lividomycin, tobramycin, amikacin, fradiomycin, sisomycin, tetracycline, oxytetracycline, rolitetracycline, doxycycline, ampicillin, piperacillin, ticarcillin, cephalothin, cephapirin, cephaloridine, cefaclor, cephalexin, cefroxadine, cefadroxil, cefamandole, cefotoam, cefuroxime, cefotiam, cefotiam hexetil, cefuroxime axetil, cefdinir, cefditoren pivoxil, ceftazidime, cefpiramide, cefsulodin, cefmenoxime, cefpodoxime proxetil, cefpirome, cefozopran, cefepime, cefsulodin, cefmenoxime, cefmetazole, cefminox, cefoxitin, cefbuperazone, latamoxef, flomoxef, cefazolin, cefotaxime, cefoperazone, ceftizoxime, moxalactam, thienamycin, sulfazecin, aztreonam or a salt thereof, griseofulvin, lankacidin-group [Journal of Antibiotics (J. Antibiotics), 38, 877-885(1985)] and the like.

(2) Antifungal agents
   (i) polyethylene antibiotics (e.g., amphotericin B, nystatin, trichomycin)
   (ii) griseofulvin, pyrrolnitrin and the like.
   (iii) cytosine metabolism antagonists (e.g., flucytosine)
   (iv) imidazole derivatives (e.g., econazole, clotrimazole, miconazole nitrate, bifonazole, croconazole)
   (v) triazole derivatives (e.g. fluconazole, itraconazole, azole compound [2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone]
   (vi) thiocarbamic acid derivatives (e.g. trinaphthol)
   (vii) echinocandin derivatives (e.g., caspofungin, micafungin, anidulafungin) and the like.

(3) Non-steroidal antiinflammatory drugs
   acetaminophen, phenacetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, epirizole, tiaramide hydrochloride, zaltoprofen, gabexate mesylate, camostat mesylate, urinastatin, colchicine, probenecid, sulfinpyrazone, benzbromarone, allopurinol, gold sodium thiomalate, sodium hyaluronate, sodium salicylate, morphine hydrochloride, salicylic acid, atropine, scopolamine, morphine, pethidine, levorphanol, ketoprofen, naproxen, oxymorphone or a salt thereof, and the like.

(4) Steroids
   dexamethasone, hexestrol, methimazole, betamethasone, triamcinolone, triamcinolone acetonide, fluocinonide, fluocinolone acetonide, prednisolone, methylprednisolone, cortisone acetate, hydrocortisone, fluorometholone, beclometasone propionate, estriol and the like.

(5) Anticoagulants
   heparin sodium, sodium citrate, activated protein C, tissue factor pathway inhibitor, antithrombin III, dalteparin sodium, warfarin potassium, argatroban, gabexate, sodium citrate and the like.

(6) Antithrombotic drugs
   ozagrel sodium, ethyl icosapentate, beraprost sodium, alprostadil, ticlopidine hydrochloride, pentoxifylline, dipyridamole and the like.

(7) Thrombolytic drugs
   tisokinase, urokinase, streptokinase and the like.

(8) Immunomodulators
   cyclosporin, tacrolimus, gusperimus, azathioprine, antilymphocyte serum, dried sulfonated immunoglobulin, erythropoietin, colony-stimulating factor, interleukin, interferon and the like.

(9) Antiprotozoals
   metronidazole, tinidazole, diethylcarbamazine citrate, quinine hydrochloride, quinine sulfate and the like.

(10) Antitussive and expectorant drugs
   ephedrine hydrochloride, noscapine hydrochloride, codeine phosphate, dihydrocodeine phosphate, isoproterenol hydrochloride, ephedrine hydrochloride, methylephedrine hydrochloride, noscapine hydrochloride, alloclamide, chlophedianol, picoperidamine, chloperastine, protokylol, isoproterenol, salbutamol, terbutaline, oximetebanol, morphine hydrochloride, dextromethorphan hydrobromide, oxycodone hydrochloride, dimemorphan phosphate, tipepidine hibenzate, pentoxyverine citrate, clofedanol hydrochloride, benzonatate, guaifenesin, bromhexine hydrochloride, ambroxol hydrochloride, acetylcysteine, ethyl cysteine hydrochloride, carbocysteine and the like.

(11) Sedatives
   chlorpromazine hydrochloride, atropine sulfate, phenobarbital, barbital, amobarbital, pentobarbital, thiopental sodium, thiamylal sodium, nitrazepam, estazolam, flurazepam, haloxazolam, triazolam, flunitrazepam, bromovalerylurea, chloral hydrate, triclofos sodium and the like.

(12) Anesthetics
   (12-1) Local anesthetics
      cocaine hydrochloride, procaine hydrochloride, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine) and the like. (12-2) General anesthetics
      (i) inhalation anesthetics (e.g., ether, halothane, nitrous oxide, influrane, enflurane),
      (ii) intravenous anesthetics (e.g., ketamine hydrochloride, droperidol, thiopental sodium, thiamylal sodium, pentobarbital) and the like.

(13) Antinarcotics
   levallorphan, nalorphine, naloxone or a salt thereof and the like.

(14) Antiulcer drugs
   metoclopromide, histidine hydrochloride, lansoprazole, metoclopramide, pirenzepine, cimetidine, ranitidine, famotidine, urogastrone, oxethazaine, proglumide, omeprazole, sucralfate, sulpiride, cetraxate, gefarnate, aldioxa, teprenone, prostaglandin and the like.

(15) Hyperlipidemia treating agents HMG-CoA reductase inhibitors (e.g., fluvastatin, cerivastatin, atorvastatin etc.), fibrates (e.g., simfibrate, clofibrate aluminum, clinofibrate, fenofibrate etc.), adsorbents for bile acid (e.g., cholestyramine etc.), nicotinic acid formulations (e.g., nicomol, niceritrol, tocopherol nicotinate etc.), probucol and a derivative thereof, polyvalent unsaturated fatty acid derivatives (e.g., ethyl icosapentate, polyene phosphatidylcholine, melinamide etc.), vegetable sterols (e.g., gamma-oryzanol, soysterol etc.), elastases, sodium dextran sulfate, squalene synthetase inhibitor, squalene epoxidase inhibitor, CETP inhibitor, ethyl 2-chloro-3-[4-(2-methyl-2-phenylpropoxy)phenyl]propionate [chemical and pharmaceutical bulletin (Chem. Pharm. Bull.), 38, 2792, 2796 (1990)], LDL receptor enhancing drug, cholesterol absorption inhibitor (Ezetimibe etc.), MTP inhibitor, ileal bile acid transporter inhibitor, SCAP ligand, FXR ligand and the like.

(16) Therapeutic agents for arteriosclerosis
   MMP inhibitor, chymase inhibitor, ACAT inhibitor(Avasimibe, Eflucimibe etc.), apoAI Milano and an analogue thereof, scavenger receptor inhibitor, 15-lipoxygenase inhibitor, phospholipase A2 inhibitor, ABCA1 activator, LXR ligand, sphingomyelinase inhibitor, paraoxonase activator, estrogen receptor agonist and the like.

(17) HDL increasing agents
   squalene synthetase inhibitors, CETP inhibitors, LPL activators and the like.

(18) Unstable plaque stabilizing agents MMP inhibitors, chymasekinase inhibitors, ACAT inhibitors, lipid-rich plaque regressing agents and the like.

(19) Myocardial protecting agents
   cardiac ATP-K oral formulation, endoserine antagonist, urotensin antagonist and the like.

(20) Hypothyroidism treating agents
   dried thyroid gland (thyreoid), levothyroxine sodium (thyradin S), liothyronidin sodium (thyronine, thyromin) and the like.

(21) Nephrotic syndrome treating agents
   prednisolone (Predonine), prednisolone succinate sodium (Predonine), methylprednisolone succinate sodium (Solu medrol), betamethasone (rinderon) and the like.

(22) Chronic renal failure treating agents
   diuretics [e.g., furosemide (lasix), bumetamide (lunetron), azosemide (diart)], hypotensive agent (e.g., ACE inhibitor, (enalapril maleate (renivase)) and Ca antagonist (manidipine), α-receptor blocker, AII antagonist (candesartan)] and the like.

(23) Diuretics
   thiazide diuretics (benzylhydro-chlorothiazide, cyclopenthiazide, ethiazide, hydrochlorothiazide, hydroflumethiazide, methyclothiazide, penfluthiazide, polythiazide, trichloromethiazide etc.), loop diuretics (clortharidone, clofenamide, indapamide, mefruside, meticrane, sotolazone, tripamide, quinethazone, metolazone, furosemide etc.), potassium retaining diuretics (spironolacton, triamterene etc.).

(24) Hypertension Treating Agents
   (i) sympathetic nerve suppressants
      α₂ stimulants (e.g., clonidine, guanabenz, guanfacine, methyldopa etc.), ganglionic blocking agents (e.g., hexamethonium, trimethaphan etc.), presynaptic blockers (e.g., alseroxylon, dimethylaminoreserpinate, rescinamine, reserpine, syrosingopine etc.), neuron blockers (e.g., betanidine, guanethidine etc.), α₁ blockers (e.g., bunazosin, doxazocin, prazosin, terazosin, urapidil etc.), β blockers (e.g., propranolol, nadolol, timolol, nipradilol, bunitrolol, indenolol, penbutolol, carteolol, carvedilol, pindolol, acebutolol, atenolol, bisoprolol, metoprolol, labetalol, amosulalol, arotinolol etc.).
   (ii) vasodilators
      calcium channel antagonists (e.g., manidipine, nicardipine, nilvadipine, nisoldipine, nitrendipine, benidipine, amlodipine, aranidipine etc.), phthalazine derivatives (e.g., budralazine, cadralazine, ecarazine, hydralazine, todralazine etc.) and the like.
   (iii) ACE inhibitors
      alacepril, captopril, cilazapril, delapril, enalapril, lisinopril, temocapril, trandolapril, quinapril, imidapril, benazepril, perindopril and the like.
   (iv) AII antagonists
      losartan, candesartan, valsartan, termisartan, irbesartan, forasartan and the like.
   (v) diuretics (e.g., diuretics described above)

(25) Cardiac failure treating agents
   cardiotonic agents (e.g., digitoxin, digoxin, methyldigoxin, lanatoside C, proscillaridine etc.), α, β-stimulants (e.g., epinephrine, norepinephrine, isoproterenol, dopamine, docarpamine, dobutamine, denopamine etc.), phosphodiesterase inhibitors (e.g., amrinone, milrinone, olprinone hydrochloride etc.), calcium channel sensitivity promoters (e.g., pimobendan etc.), nitrate agents (e.g., nitroglycerin, isosorbide nitrate etc.), ACE inhibitors (e.g., ACE inhibitors described above), diuretics (e.g., diuretics described above), carperitide, ubidecarenone, vesnarinone, aminophylline and the like.

(26) Muscle relaxants
   pridinol, tubocurarine, pancuronium, tolperisone hydrochloride, chlorphenesin carbamate, baclofen, chlormezanone, mephenesin, chlorzoxazone, eperisone, tizanidine and the like.

(27) Anticonvulsants
   phenytoin, ethosuximide, acetazolamide, chlordiazepoxide, trimethadione, carbamazepine, phenobarbital, primidone, sulthiame, sodium valproate, clonazepam, diazepam, nitrazepam and the like.

(28) Cardiacs
   trans-pi-oxocamphor, terephyllol, aminophyllin, etilefrine, dopamine, dobutamine, denopamine, aminophyllin, becinarin, amrinone, pimobendan, ubidecarenone, digitoxin, digoxin, methyldigoxin, lanatoside C, G-strophanthin and the like.

(29) Vasodilators
   oxyfedrine, diltiazem, tolazoline, hexobendine, bamethan, clonidine, methyldopa, guanabenz and the like.

(30) Vasoconstrictors
   dopamine, dobutamine, denopamine and the like.

(31) Antiarrhythmics
   (i) Na channel blockers (e.g., quinidine, procainamide, disopyramide, ajmaline, cibenzoline, lidocaine, diphenylhydantoin, mexiletine, propafenone, flecainide, pilsicainide, phenitoin etc.),
   (ii) β-blockers (e.g., propranolol, alprenolol, bufetolol, oxprenolol, atenolol, acebutolol, metoprolol, bisoprolol, pindolol, carteolol, arotinolol etc.),
   (iii) K channel blockers (e.g., amiodarone etc.),
   (iv) Ca channel blockers (e.g., verapamil, diltiazem etc.) and the like.

(32) Hypertensors
   dopamine, dobutamine, denopamine, digitoxin, digoxin, methyldigoxin, lanatoside C, G-strophanthin and the like.

(33) Antidiabetic drugs
   sulfonylureas (e.g., tolbutamide, chlorpropamide, glyclopyramide, acetohexamide, tolazamide, glibenclamide, glybuzole etc.), biguanides (e.g., metformin hydrochloride, buformin hydrochloride etc.), α-glucosidase inhibitors (e.g., voglibose, acarbose etc.), insulin sensitizers (e.g., pioglitazone, rosiglitazone, troglitazone etc.), insulin, glucagon, agents for treating diabetic complications (e.g., epalrestat etc.) and the like.

(34) Tranquilizers
   diazepam, lorazepam, oxazepam, chlordiazepoxide, medazepam, oxazolam, cloxazolam, clotiazepam, bromazepam, etizolam, fludiazepam, hydroxyzine and the like.

(35) Antipsychotics
   chlorpromazine hydrochloride, prochlorperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, reserpine, clocapramine hydrochloride, sulpiride, zotepine and the like.

(36) Therapeutic agents for Alzheimer's diseases
   (i) choline esterase inhibitors such as donepezil, rivastigmine, galanthamine, TAK-147 and the like.
   (ii) cerebral function activators such as Idebenone, Memantine, vinpocetine and the like.

(37) Anti-Parkinson drugs
   L-dopa, Deprenyl, carbidopa+levodopa, Pergolide, Ropinirole, cabergoline, Pramipexol, Entacapone, Lazabemide and the like.

(38) Therapeutic agents for amyotrophic spinal lateral sclerosis
   riluzole, mecasermin, Gabapentin and the like.

(39) Antidepressants
   imipramine, clomipramine, noxiptiline, phenelzine, amitriptyline hydrochloride, nortriptyline hydrochloride, amoxapine, mianserin hydrochloride, maprotiline hydrochloride, sulpiride, fluvoxamine maleate, trazodone hydrochloride and the like.

(40) Therapeutic agents for schizophrenia
   Olanzapine, risperidone, Quetiapine, Iloperidone and the like.

(41) Antitumor drugs
   6-O-(N-chloroacetylcarbamoyl)fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, peplomycin sulfate, vincristine sulfate, vinblastine sulfate, irinotecan hydrochloride, cyclophosphamide, melphalan, busulphan, thiotepa, procarbazine hydrochloride, cisplatin, azathioprine, mercaptopurine, tegafur, carmofur, cytarabine, methyltestosterone, testosterone propionate, testosterone enanthate, mepitiostane, fosfestol, chlormadinone acetate, leuprorelin acetate, buserelin acetate and the like.

(42) Vitamins
   (i) vitamin A: vitamin A₁, vitamin A₂ and retinol palmitate
   (ii) vitamin D: vitamin D₁, D₂, D₃, D₄ and D₅
   (iii) vitamin E: α-tocopherol, β-tocopherol, γ-tocopherol, δ-tocopherol, dl-α-tocopherol nicotinate
   (iv) vitamin K: vitamin K₁, K₂, K₃ and K₄
   (v) folic acid (vitamin M)
   (vi) vitamin B: vitamin B₁, vitamin B₂, vitamin B₃, vitamin B₅, vitamin B₆ and vitamin B₁₂
   (vii) biotin (vitamin H) and the like.

(43) Vitamin derivatives
   various derivatives of vitamins, for example, ascorbic acid, vitamin D₃ derivatives such as 5,6-trans-cholecalciferol, 2,5-hydroxycholecalciferol, 1-α-hydroxycholecalciferol and the like, vitamin D₂ derivatives such as 5,6-trans-ergocalciferol and the like.

(44) Antiallergic drugs
   diphenhydramine, chlorpheniramine, tripelennamine, metodilamine, clemizole, diphenylpyraline, methoxyphenamine, sodium cromoglycate, tranilast, repirinast, amlexanox, ibudilast, ketotifen, terfenadine, mequitazine, azelastine, epinastine, ozagrel hydrochloride, pranlkast hydrate, seratrodast and the like.

(45) Antiasthmatics
   isoprenaline hydrochloride, salbutamol sulfate, procaterol hydrochloride, terbutaline sulfate, trimetoquinol hydrochloride, tulobuterol hydrochloride, orciprenaline sulfate, fenoterol hydrobromide, ephedrine hydrochloride, iprotropium bromide, oxitropium bromide, flutropium bromide, theophyline, aminophyllin, sodium cromoglycate, tranilast, repirinast, anrexanone, ibudilast, ketotifen, terfenadine, mequitazine, azelastine, epinastine, ozagrel hydrochloride, pranlkast hydrate, seratrodast, dexamethasone, prednisolone, hydrocortisone, beclopetasone propionate and the like.

(46) Therapeutic agents for atopic dermatitis
   sodium cromoglycate and the like.

(47) Therapeutic agents for allergic rhinitis
   sodium cromoglycate, chlorpheniramine maleate, alimemazine tartrate, clemastine fumarate, homochlorcyclizine hydrochloride, terfenadine, mequitazine and the like.

(48) Therapeutic agents for pollakisuria/anischuria
   flavoxate hydrochloride and the like.

(49) Anti-sepsis drugs
   peptidic compounds such as rBPI-21 (bactericidal permeability increasing protein), BI-51017 (antithrombin III), SC-59735 (rTFPI), r-PAF acetylhydrase, LY-203638 (r-activated protein C), anti-TNF-α antibody, anti-CD14 antibody, CytoFab, alkaline phosphatase (LPS inactivator) and the like, and non-peptidic compounds such as JTE-607, E-5531, E-5564, S-5920, FR-167653, ONO-1714, ONO-5046 (sivelestat), GW-273629, RWJ-67657, GR-270773, NOX-100, GR-270773, NOX-100, INO-1001 and the like.

(50) prognosis improving drug after coronary bypass operation E-5564 and the like.

(51) antiemetic
   phenothiazine derivative, 5-HT3 receptor antagonist and the like.

(52) inhibitor of methemoglobin increase
   Methylene blue, ascorbic acid and the like.

(53) Others
   hydroxycam, diaserine, megestrol acetate, nicerogolin, prostaglandins and the like.

When a TLR signaling inhibitory substance (particularly, cycloalkene compound or compound A) and other drug are combined, the following effects are afforded.
(1) The doses thereof can be reduced as compared to a single administration of each of a TLR signaling inhibitory substance and a concomitant drug.
(2) A synergistic treatment effect can be obtained.
(3) A broad treatment effect can be provided on various diseases developed by diseases such as bacterial infection and the like.
(4) The side effects of a TLR signaling inhibitory substance can be reduced.

With regard to the combined use, the timing of the administration of a TLR signaling inhibitory substance and a concomitant drug is not limited. A TLR signaling inhibitory substance or a pharmaceutical composition thereof and a concomitant drug or a pharmaceutical composition thereof may be simultaneously administered to the administration subject, or may be administered in a staggered manner. The dose of the concomitant drug follows a clinical dose and can be appropriately determined depending on the administration subject, administration route, disease, combination and the like.

The mode of combined administration is not particularly limited, and a TLR signaling inhibitory substance and a concomitant drug only need to be combined on administration. Examples of such administration mode include the following:
(1) administration of a single preparation obtained by simultaneously processing the TLR signaling inhibitory substance or a pharmaceutical composition and the concomitant drug, (2) simultaneous administration of two kinds of preparations of the TLR signaling inhibitory substance or a pharmaceutical composition and the concomitant drug or a pharmaceutical composition, which have been separately produced, by the same administration route, (3) administration of two kinds of preparations of the TLR signaling inhibitory substance or a pharmaceutical composition and the concomitant drug or a pharmaceutical composition, which have been separately produced, by the same administration route in a staggered manner, (4) simultaneous administration of two kinds of preparations of the TLR signaling inhibitory substance or a pharmaceutical composition and the concomitant drug or a pharmaceutical composition, which have been separately produced, by different administration routes, (5) administration of two kinds of preparations of the TLR signaling inhibitory substance or a pharmaceutical composition and the concomitant drug or a pharmaceutical composition, which have been separately produced, by different administration routes in a staggered manner (e.g., administration in the order of the TLR signaling inhibitory substance or a pharmaceutical composition and the concomitant drug or a pharmaceutical composition, or in the reverse order) and the like.

The mixing ratio of a TLR signaling inhibitory substance and a concomitant drug in the combination drug of the present invention can be appropriately determined according to the subject of administration, administration route, disease and the like.
For example, the content of the TLR signaling inhibitory substance of the present invention in the combination agent of the present invention differs depending on the form of a preparation, and usually from about 0.01 to 99.8% by weight, preferably from about 0.1 to 50% by weight, further preferably from about 0.5 to 20% by weight, based on the preparation.

The content of the concomitant drug in the combination agent of the present invention differs depending on the form of a preparation, and usually from about 0.01 to 99.8% by weight, preferably from about 0.1 to 50% by weight, further preferably from about 0.5 to 20% by weight, based on the preparation.
The content of additives such as carrier in the combination agent of the present invention differs depending on the form of a preparation, and usually from about 1 to 99.98% by weight, preferably from about 10 to 90% by weight, based on the preparation.
When the TLR signaling inhibitory substance and the concomitant drug are independently prepared, the contents thereof may be the same as those mentioned above.

When a TLR signaling inhibitory substance is administered to a human, it can be safely administered orally or parenterally as it is or in a mixture with an appropriate pharmacologically acceptable carrier, excipient and diluent, in a pharmaceutical composition such as an oral formulation (e.g., powder, granule, tablet, capsule etc.), a parenteral formulation (e.g., injection, external formulation (e.g., nasal formulation, percutaneous formulation etc.) and suppository (e.g., rectal suppository and vaginal suppository etc.).

Any of these formulations may be produced by any method known per se which is employed ordinarily for producing a pharmaceutical formulation. The amount of a TLR signaling inhibitory substance to be incorporated into a formulation may vary depending on the dosage forms, and is preferably about 10 to 95% by weight in an oral formulation described above and about 0.001 to about 95% by weight in a parenteral formulation described above.

For example, a TLR signaling inhibitory substance can be prepared into an aqueous injection together with a solubilizer (e.g., β-cyclodextrins etc.), a dispersant (e.g., Tween 80 (manufactured by ATLASPOWDER USA), HCO 60 (manufactured by NIKKO CHEMICALS), carboxymethylcellulose, sodium arginate etc.), a preservative (e.g., methyl paraben, propyl paraben, benzyl alcohol chlorobutanol etc.), an isotonic agent (e.g., sodium chloride, glycerine, sorbitol, glucose etc.) and the like, or into an oil-based injection by dissolving, suspending or emulsifying using a vegetable oil (e.g., olive oil, sesame oil, peanut oil, cottonseed oil, corn oil etc.) and propylene glycol and the like.

An oral formulation can be produced by, for example, compressing a TLR signaling inhibitory substance together with an excipient (e.g., lactose, sucrose, starch etc.), a disintegrant (e.g., starch, calcium carbonate etc.), a binder (e.g., starch, gum arabic, carboxymethyl cellulose, polyvinyl pyrrolidone, hydroxypropyl cellulose etc.), a lubricant (e.g., talc, magnesium stearate, polyethylene glycol 6000 etc.), and the like, followed by, where necessary, a coating process known per se for the purpose of masking a taste, forming an enteric coat, or achieving a sustained release. For such coating may be used, for example, hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (manufactured by ROHM, Germany, a copolymer of methacrylic acid and acrylic acid), a dye (e.g., titanium oxide, colcothar etc.) and the like.

A TLR signaling inhibitory substance can also be employed as an external formulation in the form of a solid or semi-solid or a liquid.
For example, a solid external formulation may be a TLR signaling inhibitory substance as it is or in a mixture with an excipient (e.g., glycol, mannitol, starch, crystalline cellulose etc.), a thickening agent (e.g., natural gums, cellulose derivatives, acrylic acid polymers etc.) which is then converted into a powder composition. A semi-solid external formulation may be produced by a standard method and preferably used in the form of an aqueous or oil-based gel or ointment. A liquid external formulation may be produced by a method employed for producing an injection formulation or an analogous method in the form of an oil-based or aqueous suspension.

The solid, semi-solid or liquid external formulation may be supplemented also with a pH modifier (e.g., carbonic acid, phosphoric acid, citric acid, hydrochloric acid, sodium hydroxide etc.), an antiseptic (e.g., p-oxybenzoates, chlorobutanol, benzalkonium chloride etc.) and the like, as appropriate. Typically, a vaseline or a lanolin is used as a formulation base, per 1 g of which about 0.1 to 100 mg of a TLR signaling inhibitory substance is contained to form an ointment.

A TLR signaling inhibitory substance may be also formulated as an oil or aqueous, solid or semi-solid or liquid suppository. As an oil base in preparing suppository, for example, a high fatty acid glyceride (e.g., cocoa butter, WITEPSOL (manufactured by DYNAMIT NOBEL) etc.), a middle fatty acid (e.g., MYGLYOL (manufactured by DYNAMIT NOBEL) etc.), a vegetable oil (e.g., sesame oil, soybean oil, cottonseed oil etc.) and the like are used as appropriate. An aqueous base may be, for example, polyethylene glycol or propylene glycol, and an aqueous gel base may be, for example, a natural gum, a cellulose derivative, a vinyl polymer, an acrylic polymer and the like.

In the present invention, a cycloalkene compound or compound A (particularly, compound 72 (ethyl (6R)-6-[(2-chloro-4-fluoroanilino)sulfonyl]-1-cyclohexene-1-carboxylate) of Reference Example B66 or a salt thereof is desirably used in the form of an emulsion composition containing the compound and a buffer adjusted to pH about 3.7 - about 5.5 (hereinafter the composition is abbreviated as emulsion composition A).

According to emulsion composition A, a cycloalkene compound or compound A (hereinafter the compound of the present invention) can be effectively used as a component of a composition constituted with an emulsifier.
The compound of the present invention may be present in an oil phase as a liquid or solid, and emulsion composition A is an oil-in-water (O/W) or S/O/W emulsion composition.

The emulsion composition A can be produced, for example, using an emulsifier.
The emulsion composition A is specifically constituted with an oil component, an emulsifier, dispersion phase particles containing the compound of the present invention, and water containing a buffer wherein the dispersion phase particles are dispersed. The dispersion phase particles mean a dispersion phase wherein one of immiscible two liquids is present as fine particles in the other.

As the oil component, any pharmaceutically acceptable fats and oils generally used for the preparation of fat emulsions in the pharmaceutical technical field can be used. Examples of fats and oils include vegetable oil, partially hydrogenated vegetable oil, fats and oils obtained by transesterification reaction (single acid group glyceride (simple glyceride) or mixed acid group glyceride (mixed glyceride)), and middle chain fatty acid glycerol ester and the like.

The aforementioned fats and oils include fatty acid glycerol ester having a carbon number of about 6 - 30 (preferably about 6 - 22). Examples of the aforementioned fatty acid include saturated fatty acid such as caproic acid, caprylic acid, capric acid, lauric acid, myristic acid, palmitic acid, stearic acid, behenic acid and the like, unsaturated fatty acid such as palmitoleic acid, oleic acid, linoleic acid, arachidonic acid, eicosapentanoic acid, docosahexaenoic acid and the like.

Among vegetable oils, a preferable oil component contains, for example, vegetable oil such as soybean oil, cottonseed oil, rape seed oil, peanut oil, safflower oil, sesame oil, rice bran oil, corn germ oil, sunflower oil, poppy oil, olive oil and the like, and the like. Among these vegetable oils, soybean oil and the like are preferably used.

As fats and oils, a middle chain fatty acid triglyceride having a carbon number of about 6 - 14 (preferably about 8 - 12) can also be used. Preferable middle chain fatty acid glycerol ester includes, for example, caprylic/capric triglycerides such as "miglyo1810", "miglyol 812" (both manufactured by Huls, available from Mitsuba Trading Co., Ltd.) and the like, caprylic acid triglycerides (glycerol tricaprylic acid ester) such as "Panacete 800" (manufactured by NOF Corporation) and the like, and the like.

The amount of the oil component in emulsion composition A to be used is, for example, about 1 - about 30 wt%, preferably about 2 - about 25 wt%, more preferably about 2.5 - about 22.5 wt%, of the whole composition.

As the aforementioned emulsifier, any pharmaceutically acceptable emulsifier can be used. Particularly, pharmaceutically acceptable phospholipids and non-ionic surfactants are preferable. The emulsifier can be used alone or as a mixture of two or more kinds thereof.

Phospholipid includes, for example, naturally occurring phospholipids (e.g., egg-yolk lecithin, soybean lecithin and the like), hydrogenated products thereof, or synthetically obtained phospholipids (e.g., phosphatidylcholine, phosphatidylethanolamines, phosphatidic acid, phosphatidylserine, phosphatidylinositol, phosphatidylglycerol and the like) and the like. Among these phospholipids, egg-yolk lecithin, soybean lecithin, and phosphatidylcholine derived from egg-yolk and soybean are preferable. A particularly preferable phospholipid is lecithin. Among synthetic phospholipids, anionic phospholipid is preferable. As the anionic synthetic phospholipid, anionic synthetic phospholipids such as dimyristoylphosphatidylglycerol, dipalmitoylphosphatidylglycerol, distearoylphosphatidylglycerol, dioleoylphosphatidylglycerol, oleoylpalmitoylphosphatidylglycerol, dioctanoylphosphatidic acid, didecanoylphosphatidic acid, dilauroylphosphatidic acid, dimyristoylphosphatidic acid, dipalmitoylphosphatidic acid, diheptadecanoylphosphatidic acid, distearoylphosphatidic acid, dioleoylphosphatidic acid, arachidonylstearoylphosphatidic acid, dipalmitoylphosphatidylserine, dioleoylphosphatidylserine, dimyristoylphosphatidylinositol, dipalmitoylphosphatidylinositol, distearoylphosphatidylinositol, dioleoylphosphatidylinositol, dimyristoylphosphatidylserine, distearoylphosphatidylserine and the like are specifically used, and dimyristoylphosphatidylglycerol is particularly preferable.
These anionic synthetic phospholipids can be chemically synthesized by a method known per se, or can also be obtained by purification.

As the non-ionic surfactant, a polymer surfactant having a molecular weight of about 800 - 20000, for example, polyoxyethylene-polyoxypropylene copolymer, polyoxyethylene alkyl ether, polyoxyethylenealkylarylether, hydrogenated castor oil polyoxyethylene derivative, sorbitan polyoxyethylene derivative, polyoxyethylene sorbitol derivative, polyoxyethylene alkyl ether sulfate and the like can be mentioned.

The emulsifiers of phospholipid and non-ionic surfactants can be used alone or as a mixture of two or more kinds thereof. Alternatively, commercially available phospholipids may be used.
The total amount of the emulsifier in emulsion composition A to be used is generally about 0.1 - about 10%(W/V), preferably about 0.2 - about 7%(W/V), more preferably about 0.5 - about 5%(W/V), relative to the whole composition. The anionic synthetic phospholipid is in a proportion of about 0.0001 - about 5%(W/V) relative to the whole composition.

In emulsion composition A, the proportion of the emulsifier relative to the oil component is, for example, about 0.1 - about 150 wt%, preferably about 0.5 - about 125 wt%, more preferably about 1 - about 100 wt%. The emulsifier is often used in a proportion of generally about 1 - about 15 wt%, particularly about 1 - about 10 wt%, relative to the oil component.

Water to be used emulsion composition A of the present invention is not particularly limited as long as it is acceptable as a pharmaceutical product and, for example, purified water, water for injection (distilled water for injection) and the like can be mentioned. For production of a product other than pharmaceutical products, water is not particularly limited.
The amount of water in emulsion composition A to be used is generally about 40 - about 99%(W/V), preferably about 55 - about 98.8%(W/V), relative to the whole composition.

The emulsion composition A can be prepared by mixing a dispersion phase component comprising the compound of the present invention (main drug), an oil component and an emulsifier with water and emulsifying the mixture and a buffer may be added to an aqueous phase before emulsification, or may be added to the emulsion composition after emulsification. Where necessary, additives such as a stabilizer to improve the stability of the aforementioned main drug, an isotonicity agent to control the osmotic pressure, an emulsion aid to improve the emulsifying power, an emulsion stabilizer to improve stability of emulsifier and the like may be added.

Examples of the stabilizer include antioxidants (e.g., ascorbic acid, tocopherol, sorbic acid, retinol etc.), chelating agents (e.g., edetic acid, citric acid, tartaric acid etc., and salts thereof) and the like. The amount of the stabilizer to be used is generally about 0.00001 - about 10%(W/V), preferably about 0.0001 - about 5%(W/V), relative to the whole emulsion composition A.

An isotonicity agent contains, for example, glycerol, sugar alcohol, monosaccharides, disaccharides, amino acid, dextran, albumin and the like. These isotonicity agents can be used in a mixture of one or more kinds thereof.

Examples of the emulsion aid include fatty acid having a carbon number of about 6 - 30, salts of such fatty acid, monoglycerides of the aforementioned fatty acid and the like. The aforementioned fatty acid includes, for example, caproic acid, capric acid, caprylic acid, lauric acid, myristic acid, palmitic acid, stearic acid, behenic acid, palmitoleic acid, oleic acid, linoleic acid, arachidonic acid, eicosapentanoic acid, docosahexaenoic acid and the like, and salts of fatty acid include, for example, alkali metal salts such as sodium salt, potassium salt and the like, calcium salt and the like.

As the emulsion stabilizer, for example, cholesterol, cholesteryl ester, tocopherol, albumin, fatty acid amide derivative, polysaccharides, fatty acid ester derivative of polysaccharides and the like can be used.

While the concentration of the compound of the present invention of emulsion composition A varies depending on the pharmacological activity or blood kinetics of the compound, it is generally about 0.001 - about 5%(W/V), preferably about 0.01 - about 2%(W/V), more preferably about 0.1 - about 1.5%(W/V). In addition, the content of the compound of the present invention in emulsion composition A can also be set to about 1 - about 5000 mg, preferably about 10 - about 2000 mg, more preferably about 100 - about 1500 mg, in 100 ml of the composition. In addition, the content of the compound of the present invention can also be adjusted to about 0.001 - about 95 wt%, preferably about 0.01 - about 30 wt%, more preferably about 0.1 - about 3 wt%, relative to the whole composition.

The proportion (wt%) of the compound of the present invention relative to the dispersion phase consisting of an oil component and an emulsifier is generally about 0.0047 - about 24%, preferably about 0.047 - about 9.4%.

The emulsion composition A is adjusted to pH about 3.7 - about 5.5, preferably about 3.7 - about 5.0, more preferably about 4.0 - about 5.0.
As a pH adjuster, for example, phosphoric acid, carbonic acid, citric acid, hydrochloric acid, sodium hydroxide and the like are used and hydrochloric acid, sodium hydroxide and the like are particularly preferable.

As the aforementioned buffer, any pharmaceutically acceptable buffer can be used. For example, a buffer containing acetic acid, glacial acetic acid, lactic acid, citric acid, phosphoric acid, carbonic acid, histidine, glycine, barbital, phthalic acid, adipic acid, ascorbic acid, maleic acid, succinic acid, tartaric acid, glutamic acid, benzoic acid, aspartic acid and a salt thereof (e.g., potassium, sodium and the like), specifically sodium acetate, sodium lactate, sodium citrate, disodium hydrogenphosphate, sodium dihydrogenphosphate, sodium carbonate, sodium hydrogen carbonate, hydrochloric acid, sodium hydroxide and the like as a constituent component is preferable. Moreover, respective buffers may be used in combination. Particularly, one or more buffers selected from acetate buffer, glacial acetate buffer, lactate buffer, citrate buffer and phosphate buffer is preferable.
As the buffer, a combination of (i) acetic acid or glacial acetic acid and sodium acetate (acetate buffer or glacial acetate buffer), or (ii) lactic acid and sodium lactate (lactate buffer) and the like is preferably used.
The concentration of the buffer is generally not more than about 100 mM, specifically about 0.1mM - about 100 mM, preferably about 0.2 - about 50 mM, more preferably about 5 mM - about 40 mM.

The pH adjuster is an acidic or alkaline compound to be added to adjust the pH of a solution to a desired pH.
The amount of the pH adjuster to be generally added to an injection is trace. The amount of sodium hydroxide as a pH adjuster in a fatty emulsion commercially available in Japan is often not more than about 0.5 mM. While the pH can be adjusted to a desired pH during adjusting the solution, the pH of the solution easily changes by the addition of an acid or alkali, and maintenance of pH is difficult.

The buffer is a compound having an action to reduce changes in pH on addition of acid or alkali, namely, a bufferizing action. In many cases, it is a mixed solution of a weak acid and a salt thereof, or a weak base and a salt thereof.
By addition of a buffer, emulsion composition A is not influenced by the development of free fatty acid, and can maintain a constant pH of an emulsion composition during high-pressure vapor sterilization and long-term preservation.
The amount of the buffer to be used for general injections aims at bufferizing action. For example, the amount of an acetate buffer in a solution injection commercially available in Japan is about 0.2 mM - about 100 mM.

The emulsion composition A is preferably used, for example, as a composition for injection.
The emulsion composition A can be basically produced by a known method or a method according thereto. Particularly, while a conventionally used emulsion technique can be utilized for the emulsion, the compound of the present invention is preferably dissolved or dispersed in advance in an oil component. To be precise, a composition containing an O/W or S/O/W emulsion can be produced by dispersing a mixture of dispersion phase (1) containing an oil component and an emulsifier, and the compound of the present invention (2) in water. The buffer may be added to an aqueous phase before emulsification, or added to an emulsion after emulsification during production.

The more preferable method includes, for example, a method of preparing an oil-in-water composition comprising homogenizing an unhomogeneous mixture of a mixture of the main drug, an oil component, an emulsifier and, where necessary, an additive such as isotonicity agent and the like, and water containing a buffer using an emulsifying machine to give a crude emulsion, adding water as necessary, further homogenizing the emulsion using the above-mentioned emulsifying machine, and removing large particles by a filtration means such as a filter and the like. The aforementioned mixture is often warmed to a temperature of, for example, about 30 - about 90°C, preferably about 40 - about 80°C, to dissolve or disperse the main drug. As the emulsifying machine to emulsify an unhomogeneous mixture of the aforementioned mixture and water, conventionally used apparatuses, for example, homogenizers such as a pressurization injection type homogenizer, ultrasonication homogenizer and the like, homomixers such as high-speed rotation type mixer and the like, and the like can be used. To remove large particles having a particle size of not less than about 5 µm, homogenized emulsion is often subjected to a filtration means such as a filter and the like.

In emulsion composition A, the particle size distribution of a dispersion phase, wherein the compound of the present invention is dissolved, is, for example, often about 0.01 - about 7 µm, preferably about 0.02 - about 5 µm. From the aspects of the stability of the emulsion and distribution in the body after administration, the average particle size of the dispersion phase particles, wherein the compound of the present invention is dissolved, is, for example, about 0.025 - about 0.7 µm, preferably about 0.05 - about 0.4 µm.

The average particle size used in the present specification means an average particle size based on the volume distribution and measured by a laser diffraction particle size distribution measurement apparatus, with the laser diffraction scattering method as a measurement principle.

Pyrogen can be removed from emulsion composition A by a method known per se.
Where necessary, after nitrogen gas substitution, emulsion composition A is sterilized and tightly sealed.
Since pH of emulsion composition A is adjusted to about 3.7 - about 5.5 by adding a buffer, pH of the composition and average particle size of the dispersion phase particles hardly change even after sterilization by an autoclave etc. or after long-term preservation, and the composition is stable. Therefore, the stability of the compound of the present invention and emulsion composition A is superior. Moreover, emulsion composition A is free of a visibly observed free oil drop even after sterilization by an autoclave etc. or after long-term preservation, and therefore, phase separation of dispersion phase particles and water wherein the dispersion phase particles are dispersed does not occur, and the composition is stable.

Furthermore, emulsion composition A can increase the concentration of the compound of the present invention, and control the particle size of the dispersion phase particles. Thus, it can enhance retentivity in blood, blood vessel permeability and transitivity into inflammation site. Therefore, in vivo kinetics ·distribution in the body of the compound of the present invention can be improved and targeting becomes possible, as a result of which administration of an effective drug with suppressed side effects becomes possible. Accordingly, emulsion composition A is particularly useful for the treatment of a target disease by an intravenous administration.

While the dose of a cycloalkene compound or compound A used as a TLR signaling inhibitory substance varies depending on the age, body weight, symptom, dosage form, administration method, dosing period and the like, it is, for example, generally about 0.01 to about 1000 mg/kg, preferably about 0.01 to about 100 mg/kg, more preferably about 0.1 to about 100 mg/kg, particularly about 0.1 to about 50 mg/kg, especially about 1.5 to about 30 mg/kg, a day in the amount of compound A etc. for a patient (adult, body weight about 60 kg) with an inflammatory disease, which is orally or parenterally administered one to several portions a day. As mentioned above, since the dose varies depending on various conditions, an amount less than the aforementioned dose may be sufficient or administration of an excess amount may be necessary.

While the dose of a combination drug in the present invention varies depending on the kind of compound, the age, body weight, symptom, dosage form, administration method, dosing period and the like, it is, for example, generally about 0.01 to about 1000 mg/kg, preferably about 0.01 to about 100 mg/kg, more preferably about 0.1 to about 100 mg/kg, particularly about 0.1 to about 50 mg/kg, especially about 1.5 to about 30 mg/kg, a day in the amount of each of a TLR signaling inhibitory substance and a concomitant drug for a patient (adult, body weight about 60 kg) with an inflammatory disease, which is intravenously administered one to several portions a day. Of course, as mentioned above, since the dose varies depending on various conditions, an amount less than the aforementioned dose may be sufficient or administration of an excess amount may be necessary.

The concomitant drug may be contained in any amount as long as a side effect does not pose a problem. While the daily dose of the combination drug may vary depending on the disease state, the age, sex, body weight and difference in sensitivity of the administration object, timing and interval of administration, characteristics, dispensing and kind of the pharmaceutical preparation, the kind of active ingredient and the like and is not particularly limited, the amount of the drug is generally about 0.001 - 2000 mg, preferably about 0.01 - 500 mg, more preferably about 0.1 - 100 mg, per 1 kg body weight of mammal by oral administration, which is generally administered in 1 to 4 portions during a day.

In administration of a combination drug of the present invention, a TLR signaling inhibitory substance may be administered after administration of the concomitant drug or the concomitant drug may be administered after administration of TLR signaling inhibitory substance, though they may be administered simultaneously. When administered at a time interval, the interval differs depending on the effective ingredient to be administered, drug form and administration method, and for example, when the concomitant drug is administered first, a method in which a TLR signaling inhibitory substance is administered within time range of from 1 min to 3 days, preferably from 10 min to 1 day, more preferably from 15 min to 1 hr after administration of the concomitant drug is exemplified. When a TLR signaling inhibitory substance is administered first, a method in which the concomitant drug is administered within time range of from 1 min to 1 day, preferably from 10 min to 6 hrs, more preferably from 15 min to 1 hr after administration of a TLR signaling inhibitory substance is exemplified.

### Examples

The present invention is further described in the following by referring to Reference Examples, Examples and Experimental Examples, which are not intended to restrict the invention.
The ¹H-NMR spectrum was determined by a VARIAN GEMINI 200 (200 MHz) spectrometer using tetramethylsilane as an internal standard and represented as the entire δ values in ppm. The number in a bracket when a solvent mixture was employed is the volume ratio of each solvent, wherein % means % by weight unless otherwise specified. The ratio of the solvents in silica gel chromatography shows the volume ratio of the solvents to be admixed.

A high polar diastereomer means a diastereomer having a smaller Rf value, and a low polar diastereomer means a diastereomer having a larger Rf value, when determined by normal phase thin layer chromatography under the same conditions (e.g., using ethyl acetate/hexane etc. as an solvent).
The melting point was measured using melting point measuring apparatus (manufactured by Yanagimoto Mfg. Co., Ltd). The data of the powder X-ray diffraction was measured using RINT2500 (Rigaku Industrial Corporation) using Cu-Kα₁ ray as a ray source.

Each symbol in examples means the following:
s: singlet d: doublet: t: triplet q: quartet
dd: double doublet tt: triple triplet m: multiplet
br: broad J: coupling constant
The following Reference Examples A can be produced according to Reference Examples of WO99/46242, Reference Example B can be produced according to Examples of WO99/46242, Reference Examples C can be produced according to Reference Examples of WO01/10826, and Reference Examples D can be produced according to Examples of WO01/10826.

[Reference Examples A]
Reference Example A1 ethyl 2-sulfo-1-cyclohexene-1-carboxylate
Reference Example A2 ethyl 2-chlorosulfonyl-1-cyclohexene-1-carboxylate
Reference Example A3 ethyl 2-chlorosulfonyl-1-cyclopentene-1-carboxylate
Reference Example A4 ethyl 2-chlorosulfonyl-1-cycloheptene-1-carboxylate
Reference Example A5 sodium 6-[N-(4-chloro-2-fluorophenyl)-sulfamoyl]-1-cyclohexene-1-carboxylate
Reference Example A6 1-(3-fluoro-4-nitrophenyl)-1H-1,2,4-triazole
Reference Example A7 1-(4-amino-3-fluorophenyl)-1H-1,2,4-triazole
Reference Example A8 methyl 4-(benzyloxycarbonylamino)-3-chlorobenzoate
Reference Example A9 4-(benzyloxycarbonylamino)-3-chlorobenzoic acid
Reference Example A10 tert-butyl N-(4-benzyloxycarbonylamino-3-chlorobenzoyl)glycinate
Reference Example A11 tert-butyl N-(4-amino-3-chlorobenzoyl)-glycinate
Reference Example A12 6-[N-(2,4-difluorophenyl)sulfamoyl]-1-cyclohexene-1-carboxylic acid
Reference Example A13 ethyl 2-mercapto-5-phenyl-1-cyclohexene-1-carboxylate
Reference Example A14 ethyl 2-chlorosulfonyl-5-phenyl-1-cyclohexene-1-carboxylate
Reference Example A15 ethyl 5-tert-butyl-2-mercapto-1-cyclohexene-1-carboxylate
Reference Example A16 ethyl 5-tert-butyl-2-chlorosulfonyl-1-cyclohexene-1-carboxylate
Reference Example A17 ethyl 5,5-dimethyl-2-mercapto-1-cyclohexene-1-carboxylate
Reference Example A18 ethyl 2-chlorosulfonyl-5,5-dimethyl-1-cyclohexene-1-carboxylate

[Reference Examples B]
Reference Example B1 ethyl 6-[N-(4-chloro-2-fluorophenyl)-sulfamoyl]-1-cyclohexene-1-carboxylate (compound 1)
Reference Example B2 ethyl 6-[N-(4-chloro-2-fluorophenyl)-N-methylsulfamoyl]-1-cyclohexene-1-carboxylate (compound 2)
Reference Example B3 ethyl 6-[N-(2,4-difluorophenyl)sulfamoyl]-1-cyclohexene-1-carboxylate (compound 3)
Reference Example B4 ethyl 6-[N-(2,6-diisopropylphenyl)-sulfamoyl]-1-cyclohexene-1-carboxylate (compound 4)
Reference Example B5 ethyl 6-[N-(4-nitrophenyl)sulfamoyl]-1-cyclohexene-1-carboxylate (compound 5)
Reference Example B6 ethyl 6-(N-phenylsulfamoyl)-1-cyclohexene-1-carboxylate (compound 6)
   ethyl 2-(N-phenylsulfamoyl)-1-cyclohexene-1-carboxylate (compound 7)
Reference Example B7 ethyl 2-[N-(4-chloro-2-fluorophenyl)-sulfamoyl]-1-cyclohexene-1-carboxylate (compound 9) Reference Example B8 2-(4-methoxyphenyl)-4,5,6,7-tetrahydro-1,2-benzisothiazol-3(2H)-one 1,1-dioxide (compound 67)
   ethyl 2-[N-(4-methoxyphenyl)sulfamoyl]-1-cyclohexene-1-carboxylate (compound 8)
Reference Example B9 ethyl 6-[N-(2-fluorophenyl)sulfamoyl]-1-cyclohexene-1-carboxylate (compound 10)
Reference Example B10 ethyl 6-[N-(3-fluorophenyl)sulfamoyl]-1-cyclohexene-1-carboxylate (compound 11)

Reference Example B11 2-(4-fluorophenyl)-4,5,6,7-tetrahydro-1,2-benzisothiazol-3(2H)-one 1,1-dioxide (compound 68)
   ethyl 6-[N-(4-fluorophenyl)sulfamoyl]-1-cyclohexene-1-carboxylate (compound 12)
   ethyl 2-[N-(4-fluorophenyl)sulfamoyl]-1-cyclohexene-1-carboxylate (compound 18)
Reference Example B12 ethyl 6-[N-(2,6-difluorophenyl)-sulfamoyl]-1-cyclohexene-1-carboxylate (compound 13)
Reference Example B13 ethyl 6-[N-(2,3-difluorophenyl)-sulfamoyl]-1-cyclohexene-1-carboxylate (compound 14)
Reference Example B14 ethyl 6-[N-(2,5-difluorophenyl)-sulfamoyl]-1-cyclohexene-1-carboxylate (compound 15)
Reference Example B15 ethyl 6-[N-(3,4-difluorophenyl)-sulfamoyl]-1-cyclohexene-1-carboxylate (compound 16)
Reference Example B16 ethyl 6-[N-(3,5-difluorophenyl)-sulfamoyl]-1-cyclohexene-1-carboxylate (compound 17)
Reference Example B17 1-ethyl 6-[N-(2,4-difluorophenyl)-sulfamoyl]-1-cyclohexene-1-carboxylate (compound 19)
   d-ethyl 6-[N-(2,4-difluorophenyl)sulfamoyl]-1-cyclohexene-1-carboxylate (compound 20)
Reference Example B18 ethyl 6-[N-(2-ethoxycarbonylphenyl)-sulfamoyl]-1-cyclohexene-1-carboxylate (compound 21)
Reference Example B19 methyl 6-[N-(2,4-difluorophenyl)-sulfamoyl]-1-cyclohexene-1-carboxylate (compound 22)
Reference Example B20 propyl 6-[N-(2,4-difluorophenyl)-sulfamoyl]-1-cyclohexene-1-carboxylate (compound 23)

Reference Example B21 methyl 6-[N-(4-chloro-2-fluorophenyl)-sulfamoyl]-1-cyclohexene-1-carboxylate (compound 24)
Reference Example B22 isopropyl 6-[N-(2,4-difluorophenyl)-sulfamoyl]-1-cyclohexene-1-carboxylate (compound 25)
Reference Example B23 ethyl 6-[N-(2-methoxycarbonylphenyl)-sulfamoyl]-1-cyclohexene-1-carboxylate (compound 26)
Reference Example B24 ethyl 6-[N-(2-fluoro-4-methylphenyl)-sulfamoyl]-1-cyclohexene-1-carboxylate (compound 27)
Reference Example B25 ethyl 6-[N-(2-chlorophenyl)sulfamoyl]-1-cyclohexene-1-carboxylate (compound 28)
Reference Example B26 ethyl 6-[N-(2-chloro-4-fluorophenyl)-sulfamoyl]-1-cyclohexene-1-carboxylate (compound 29)
Reference Example B27 ethyl 6-[N-(4-chlorophenyl)sulfamoyl]-1-cyclohexene-1-carboxylate (compound 30)
Reference Example B28 ethyl 6-[N-(2,3,4-trifluorophenyl)-sulfamoyl]-1-cyclohexene-1-carboxylate (compound 31)
Reference Example B29 isobutyl 6-[N-(2,4-difluorophenyl)-sulfamoyl]-1-cyclohexene-1-carboxylate (compound 32)
Reference Example B30 butyl 6-[N-(2,4-difluorophenyl)-sulfamoyl]-1-cyclohexene-1-carboxylate (compound 33)

Reference Example B31 ethyl 6-[N-(4-bromo-2-fluorophenyl)-sulfamoyl]-1-cyclohexene-1-carboxylate (compound 34)
Reference Example B32 ethyl 6-[N-(2,4-dichlorophenyl)-sulfamoyl]-1-cyclohexene-1-carboxylate (compound 35)
Reference Example B33 ethyl 6-[N-(2-acetoxyphenyl)sulfamoyl]-1-cyclohexene-1-carboxylate (compound 36)
Reference Example B34 ethyl 6-[N-(3-chlorophenyl)sulfamoyl]-1-cyclohexene-1-carboxylate (compound 37)
Reference Example B35 ethyl 6-[N-(2,3-dichlorophenyl)-sulfamoyl]-1-cyclohexene-1-carboxylate (compound 38)
Reference Example B36 ethyl 6-[N-(2-ethylphenyl)sulfamoyl]-1-cyclohexene-1-carboxylate (compound 39)
Reference Example B37 ethyl 6-[N-[4-(2H-1,2,3-triazol-2-yl)phenyl]sulfamoyl]-1-cyclohexene-1-carboxylate (compound 40)
Reference Example B38 ethyl 6-[N-(2,5-dichlorophenyl)-sulfamoyl]-1-cyclohexene-1-carboxylate (compound 41)
Reference Example B39 ethyl 6-[N-(2-trifluoromethoxyphenyl)-sulfamoyl]-1-cyclohexene-1-carboxylate (compound 42)
Reference Example B40 ethyl 6-[N-(2,4,5-trifluorophenyl)-sulfamoyl]-1-cyclohexene-1-carboxylate (compound 43)

Reference Example B41 ethyl 6-[N-[4-(2H-tetrazol-2-yl)phenyl]sulfamoyl]-1-cyclohexene-1-carboxylate (compound 44)
Reference Example B42 ethyl 6-[N-(2-chloro-4-methylphenyl)-sulfamoyl]-1-cyclohexene-1-carboxylate (compound 45)
Reference Example B43 ethyl 6-[N-(4-fluoro-2-methylphenyl)-sulfamoyl]-1-cyclohexene-1-carboxylate (compound 46)
Reference Example B44 ethyl 6-[N-(2,6-dichlorophenyl)-sulfamoyl]-1-cyclohexene-1-carboxylate (compound 47)
Reference Example B45 ethyl 6-[N-[4-(1H-tetrazol-1-yl)phenyl]sulfamoyl]-1-cyclohexene-1-carboxylate (compound 48)
Reference Example B46 ethyl 6-[N-(4-(1H-1,2,3-triazol-1-yl)phenyl]sulfamoyl]-1-cyclohexene-1-carboxylate (compound 49)
Reference Example B47 ethyl 6-[N-(2-trifluoromethylphenyl)-sulfamoyl]-1-cyclohexene-1-carboxylate (compound 50)
Reference Example B48 ethyl 6-[N-(4-methoxycarbonylphenyl)-sulfamoyl]-1-cyclohexene-1-carboxylate (compound 51)
Reference Example B49 benzyl 6-[N-(2,4-difluorophenyl)-sulfamoyl]-1-cyclohexene-1-carboxylate(compound 52)
Reference Example B50 ethyl 6-[N-[4-[2,3-bis(tert-butoxycarbonyl)guanidinomethyl]phenyl]sulfamoyl]-1-cyclohexene-1-carboxylate (compound 53)

Reference Example B51 ethyl 6-[N-(2-chloro-4-methoxycarbonylphenyl)sulfamoyl]-1-cyclohexene-1-carboxylate (compound 54)
Reference Example B52 ethyl 6-[N-(2-chloro-4-cyanophenyl)-sulfamoyl]-1-cyclohexene-1-carboxylate (compound 55)
Reference Example B53 2-hydroxyethyl 6-[N-(2,4-difluorophenyl)sulfamoyl]-1-cyclohexene-1-carboxylate (compound 56)
Reference Example B54 ethyl 6-[N-[2-fluoro-4-(1H-1,2,4-triazol-1-yl)phenyl]sulfamoyl]-1-cyclohexene-1-carboxylate (compound 57)
Reference Example B55 ethyl 2-[N-(2,4-difluorophenyl)-sulfamoyl]-1-cyclopentene-1-carboxylate (compound 66) ethyl 5-[N-(2,4-difluorophenyl)sulfamoyl]-1-cyclopentene-1-carboxylate (compound 58)
Reference Example B56 tert-butyl [6-[N-(2,4-difluorophenyl)-sulfamoyl]-1-cyclohexen-1-yl]carbonyloxyacetate (compound 59)
Reference Example B57 [6-[N-(2,4-difluorophenyl)sulfamoyl]-1-cyclohexen-1-yl]carbonyloxyacetic acid (compound 60)
Reference Example B58 ethyl 7-[N-(2,4-difluorophenyl)-sulfamoyl]-1-cycloheptene-1-carboxylate (compound 61)
Reference Example B59 ethyl 6-[N-[2-chloro-4-(N-tert-butoxycarbonylmethylcarbamoyl)phenyl]sulfamoyl]-1-cyclohexene-1-carboxylate (compound 62)
Reference Example B60 ethyl 6-[N-[2-chloro-4-(N-ethoxycarbonylmethylcarbamoyl)phenyl]sulfamoyl]-1-cyclohexene-1-carboxylate (compound 63)

Reference Example B61 ethyl 5-[N-(2-chloro-4-fluorophenyl)-sulfamoyl]-1-cyclopentene-1-carboxylate (compound 64)
Reference Example B62 2-[4-(2,2,3,3,3-pentafluoropropoxy)-phenyl]-4,5,6,7-tetrahydro-1,2-benzisothiazol-3(2H)-one 1,1-dioxide (compound 69)
Reference Example B63 ethyl 7-[N-(2-chloro-4-fluorophenyl)-sulfamoyl]-1-cycloheptene-1-carboxylate (compound 65)
Reference Example B64 2-(2,4-difluorophenyl)-5,6,7,7a-tetrahydro-1,2-benzisothiazol-3(2H)-one 1,1-dioxide (compound 70)
Reference Example B65 ethyl 6-[N-(2-chloro-4-fluorophenyl)-sulfamoyl]-1-cyclohexene-1-carboxylate (compound 29)
Reference Example B66 ethyl (6S)-6-[(2-chloro-4-fluoroanilino)sulfonyl]-1-cyclohexene-1-carboxylate (1-ethyl 6-[N-(2-chloro-4-fluorophenyl)sulfamoyl]-1-cyclohexene-1-carboxylate) (compound 71)
ethyl (6R)-6-[(2-chloro-4-fluoroanilino)sulfonyl]-1-cyclohexene-1-carboxylate (d-ethyl 6-[N-(2-chloro-4-fluorophenyl)sulfamoyl]-1-cyclohexene-1-carboxylate) (compound 72)
Reference Example B67 ethyl 6-[N-(2-bromo-4-fluorophenyl)-sulfamoyl]-1-cyclohexene-1-carboxylate (compound 73)
Reference Example B68 ethyl 6-[N-(4-bromo-2-chlorophenyl)-sulfamoyl]-1-cyclohexene-1-carboxylate (compound 74)
Reference Example B69 high polar diastereomer (compound 75) and low polar diastereomer (compound 76) of ethyl 6-[N-(2,4-difluorophenyl)sulfamoyl]-3-phenyl-1-cyclohexene-1-carboxylate
Reference Example B70 high polar diastereomer (compound 77) and low polar diastereomer (compound 78) of ethyl 6-[N-(2-chloro-4-fluorophenyl)sulfamoyl]-3-phenyl-1-cyclohexene-1-carboxylate

Reference Example B71 high polar diastereomer (compound 79) and low polar diastereomer (compound 80) of ethyl 6-[N-(2,4-difluorophenyl)sulfamoyl]-3-tert-butyl-1-cyclohexene-1-carboxylate
Reference Example B72 high polar diastereomer (compound 81) and low polar diastereomer (compound 82) of ethyl 6-[N-(2-chloro-4-fluorophenyl)sulfamoyl]-3-tert-butyl-1-cyclohexene-1-carboxylate
Reference Example B73 ethyl 6-[N-(2,4-difluorophenyl)-sulfamoyl]-3,3-dimethyl-1-cyclohexene-1-carboxylate (compound 83)
Reference Example B74 ethyl 6-[N-(2-chloro-4-fluorophenyl)-sulfamoyl]-3,3-dimethyl-1-cyclohexene-1-carboxylate (compound 84)
Reference Example B75 ethyl 3-bromo-6-[N-(2,4-difluorophenyl)-sulfamoyl]-1-cyclohexene-1-carboxylate (compound 85)
The chemical structures of compounds 1 - 85 are shown in Tables 1 - 12.

**Table 1**

| | | | | |
|---|---|---|---|---|
| 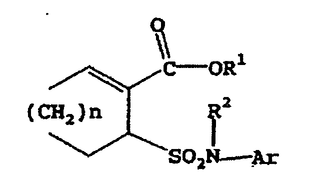 | | | | |

| Compound No. | **R¹** | **R²** | **Ar** | **n** |
|---|---|---|---|---|
| **1** | **C₂H₅** | **H** | 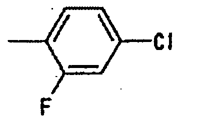 | **2** |
| **2** | **C₂H₅** | **CH₃** | 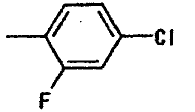 | **2** |
| **3** | **C₂H₅** | **H** | 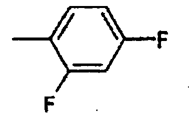 | **2** |
| **4** | **C₂H₅** | **H** | 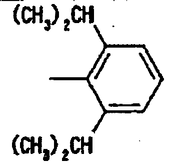 | **2** |
| **5** | **C₂H₅** | **H** | 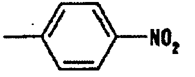 | **2** |
| **6** | **C₂H₅** | **H** | 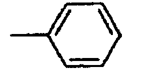 | **2** |
| **10** | **C₂H₅** | **H** | 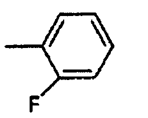 | **2** |

**Table 2**

| | | | | |
|---|---|---|---|---|
| **11** | **C₂H₅** | **H** | 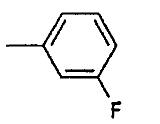 | **2** |
| **12** | **C₂H₅** | **H** | 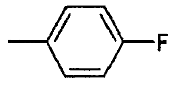 | **2** |
| **13** | **C₂H₅** | **H** | 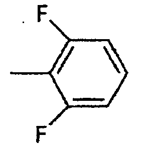 | **2** |
| **14** | **C₂H₅** | **H** | 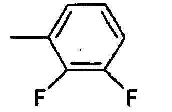 | **2** |
| **15** | **C₂H₅** | **H** | 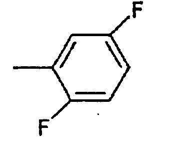 | **2** |
| **16** | **C₂H₅** | **H** | 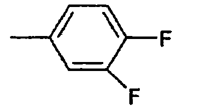 | **2** |
| **17** | **C₂H₅** | **H** | 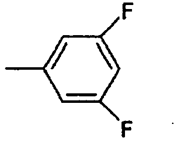 | **2** |
| **19** (*l*-form) | **C₂H₅** | **H** | 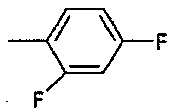 | **2** |
| **20** (d-form) | **C₂H₅** | **H** | 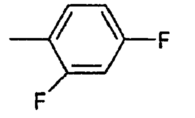 | **2** |

**Table 3**

| | | | | |
|---|---|---|---|---|
| **21** | **C₂H₅** | **H** | 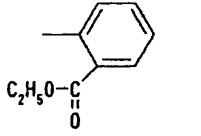 | **2** |
| **22** | **CH₃** | **H** | 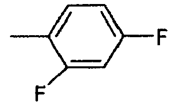 | **2** |
| **23** | **(CH₂)₂CH₃** | **H** | 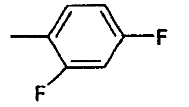 | **2** |
| **24** | **CH₃** | **H** | 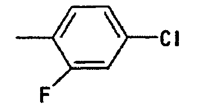 | **2** |
| **25** | **GH(CH₃)₂** | **H** | 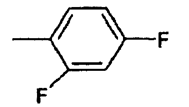 | **2** |
| **26** | **C₂H₅** | **H** | 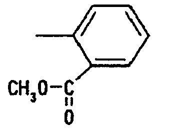 | **2** |
| **27** | **C₂H₅** | **H** | 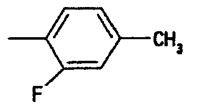 | **2** |
| **28** | **C₂H₅** | **H** | 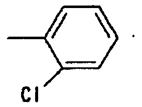 | **2** |
| **29** | **C₂H₅** | **H** | 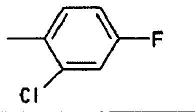 | **2** |
| **30** | **C₂H₅** | **H** | 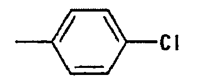 | **2** |

**Table 4**

| | | | | |
|---|---|---|---|---|
| **31** | **C₂H₅** | **H** | 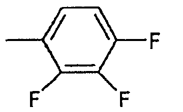 | **2** |
| **32** | **CH₂CH(CH₃)₂** | **H** | 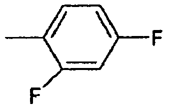 | **2** |
| **33** | **(CH₂)₃CH₃** | **H** | 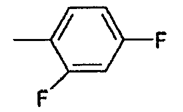 | **2** |
| **34** | **C₂H₅** | **H** | 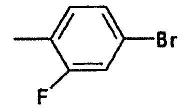 | **2** |
| **35** | **C₂H₅** | **H** | 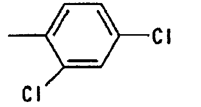 | **2** |
| **36** | **C₂H₅** | **H** | 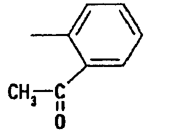 | **2** |
| **37** | **C₂H₅** | **H** | 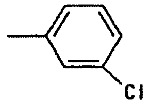 | **2** |
| **38** | **C₂H₅** | **H** | 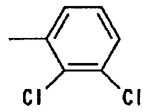 | **2** |
| **39** | **C₂H₅** | **H** | 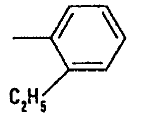 | **2** |
| **40** | **C₂H₅** | **H** | 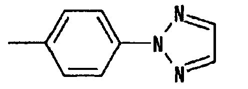 | **2** |

**Table 5**

| | | | | |
|---|---|---|---|---|
| **41** | **C₂H₅** | **H** | 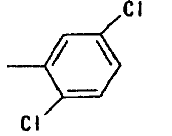 | **2** |
| **42** | **C₂H₅** | **H** | 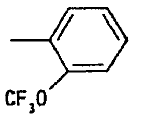 | **2** |
| **43** | **C₂H₅** | **H** | 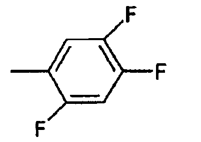 | **2** |
| **44** | **C₂H₅** | **H** | 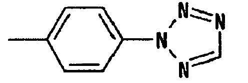 | **2** |
| **45** | **c₂H₅** | **H** | 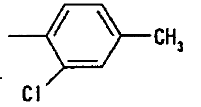 | **2** |
| **46** | **C₂H₅** | **H** | 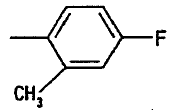 | **2** |
| **47** | **C₂H₅** | **H** | 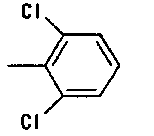 | **2** |
| **48** | **C₂H₅** | **H** | 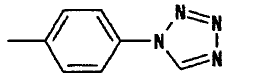 | **2** |
| **49** | **C₂H₅** | **H** | 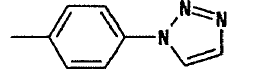 | **2** |
| **50** | **C₂H₅** | **H** | 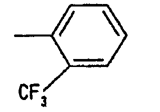 | **2** |

**Table 6**

| | | | | |
|---|---|---|---|---|
| **51** | **C₂H₅** | **H** | 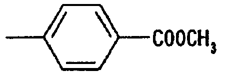 | **2** |
| **52** | 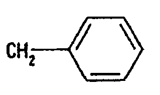 | **H** | 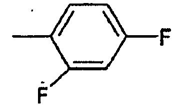 | **2** |
| **53** | **C₂H₅** | **H** | 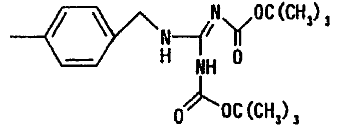 | **2** |
| **54** | **C₂H₅** | **H** | 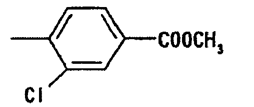 | **2** |
| **55** | **C₂H₅** | **H** | 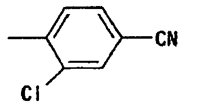 | **2** |
| **56** | **(CH₂)₂OH** | **H** | 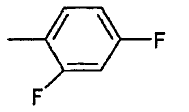 | **2** |
| **57** | **C₂H₅** | **H** | 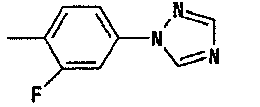 | **2** |
| **58** | **C₂H₅** | **H** | 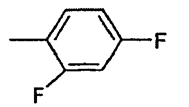 | **1** |
| **59** | **CH₂COOC(CH₃)₃** | **H** | 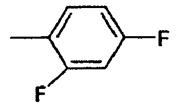 | **2** |
| **60** | **CH₂COOH** | **H** | 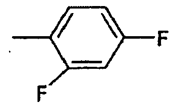 | **2** |

**Table 7**

| | | | | |
|---|---|---|---|---|
| **61** | **C₂H₅** | **H** | 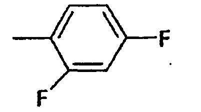 | **3** |
| **62** | **C₂H₅** | **H** | 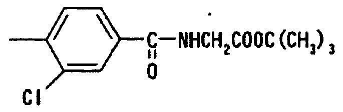 | **2** |
| **63** | **C₂H₅** | **H** | 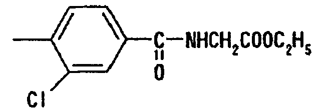 | **2** |
| **64** | **C₂H₅** | **H** | 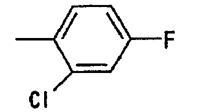 | **1** |
| **65** | **C₂H₅** | **H** | 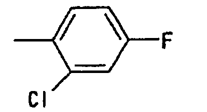 | **3** |
| **71** (S-form) | **C₂H₅** | **H** | 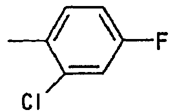 | **2** |
| **72** (R-form) | **C₂H₅** | **H** | 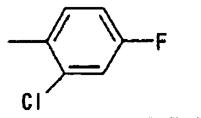 | **2** |
| **73** | **C₂H₅** | **H** | 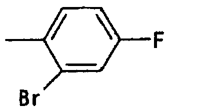 | **2** |
| **74** | **C₂H₅** | **H** | 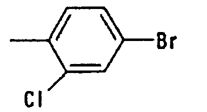 | **2** |

**Table 8**

| | | | |
|---|---|---|---|
| 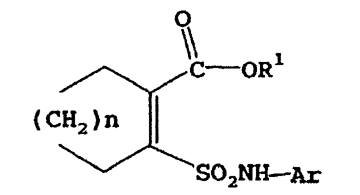 | | | |

| Compound No. | **R¹** | **Ar** | **n** |
|---|---|---|---|
| **7** | **C₂H₅** | 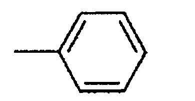 | **2** |
| **8** | **C₂H₅** | 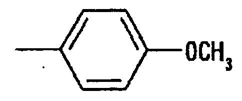 | **2** |
| **9** | **C₂H₅** | 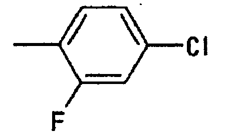 | **2** |
| **18** | **C₂H₅** | 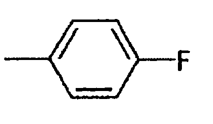 | **2** |
| **66** | **C₂H₅** | 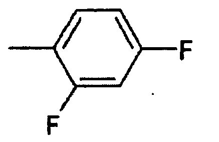 | **1** |

**Table 9**

| | | |
|---|---|---|
| 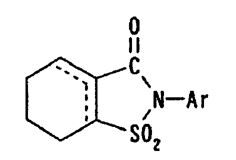 | | |

| Compound No. | 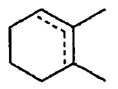 | **Ar** |
|---|---|---|
| **67** | 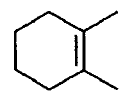 | 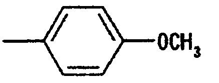 |
| **68** | 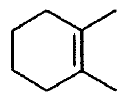 | 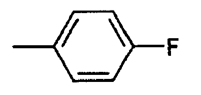 |
| **69** | 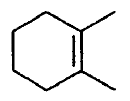 | 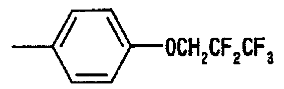 |
| **70** | 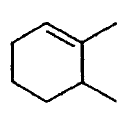 | 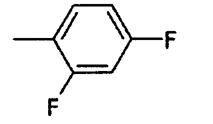 |

**Table 10**

| | | | | |
|---|---|---|---|---|
| 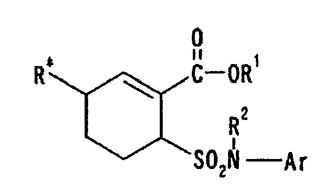 | | | | |

| Compound No. | **R¹** | **R²** | **R^{*}** | **Ar** |
|---|---|---|---|---|
| **75** (High polar diastereomer) | **C₂H₅** | **H** | 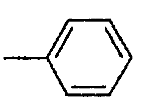 | 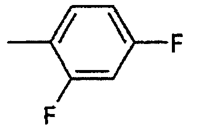 |
| **76** (Low polar diastereomer) | **C₂H₅** | **H** | 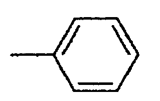 | 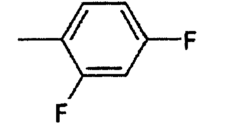 |
| **77** (High polar diastereomer) | **C₂H₅** | **H** | 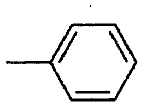 | 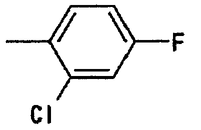 |
| **78** (Low polar diastereomer) | **C₂H₅** | **H** | 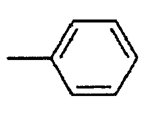 | 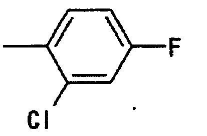 |
| **79** (High polar diastereomer) | **C₂H₅** | **H** | **C(CH₃)₃** | 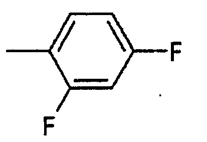 |
| **80** (Low polar diaStereomer) | **C₂H₅** | **H** | **C(CH₃)₃** | 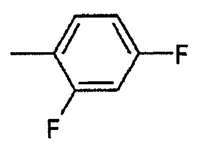 |
| **81** (High polar diastereomer) | **C₂H₅** | **H** | **C(CH₃)₃** | 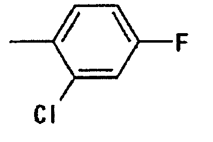 |

**Table 11**

| | | | | |
|---|---|---|---|---|
| **82** (Low polar diastereomer) | **C₂H₅** | **H** | **C(CH₃)₃** | 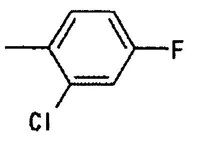 |
| **85** | **C₂H₅** | **H** | **Br** | 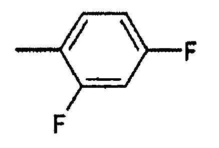 |

**Table 12**

| | |
|---|---|
| 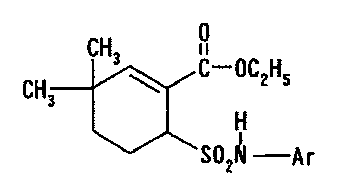 | |

| Compound No. | **Ar** |
|---|---|
| **83** | 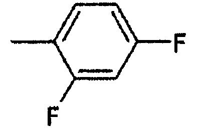 |
| **84** | 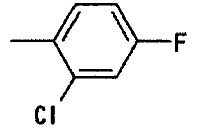 |

[Reference Examples C]
Reference Example C1 ethyl 6-(benzylsulfanyl)-1-cyclohexene-1-carboxylate
Reference Example C2 ethyl 6-[(4-methoxybenzyl)sulfanyl]-1-cyclohexene-1-carboxylate
Reference Example C3 ethyl 6-[(2,4-difluorobenzyl)sulfanyl]-1-cyclohexene-1-carboxylate
Reference Example C4 ethyl 6-[(2-chloro-4-fluorobenzyl)sulfanyl]-1-cyclohexene-1-carboxylate
Reference Example C5 ethyl 5-hydroxy-3,6-dihydro-2H-pyran-4-carboxylate
Reference Example C6 ethyl 5-sulfanyl-3,6-dihydro-2H-pyran-4-carboxylate
Reference Example C7 4-(ethoxycarbonyl)-5,6-dihydro-2H-pyran-3-sulfonic acid
Reference Example C8 ethyl 5-(chlorosulfonyl)-3,6-dihydro-2H-pyran-4-carboxylate

[Reference Examples D]
Reference Example D1 ethyl 6-(benzylsulfonyl)-1-cyclohexene-1-carboxylate (compound 1')
Reference Example D2 ethyl 6-[(4-methoxybenzyl)sulfonyl]-1-cyclohexene-1-carboxylate (compound 2')
Reference Example D3 ethyl 6-[(2,4-difluorobenzyl)sulfonyl]-1-cyclohexene-1-carboxylate (compound 3')
Reference Example D4 ethyl 6-[(2-chloro-4-fluorobenzyl)sulfonyl]-1-cyclohexene-1-carboxylate (compound 4')
Reference Example D5 ethyl (-)-6-[(2-chloro-4-fluorobenzyl)sulfonyl]-1-cyclohexene-1-carboxylate (compound 5')
   ethyl (+)-6-[(2-chloro-4-fluorobenzyl)sulfonyl]-1-cyclohexene-1-carboxylate (compound 6')
Reference Example D6 ethyl 3-[(2,4-difluorophenyl)sulfamoyl]-3,6-dihydro-2H-pyran-4-carboxylate (compound 7')
Reference Example D7 ethyl 3-[(2-chloro-4-fluorophenyl)sulfamoyl]-3,6-dihydro-2H-pyran-4-carboxylate (compound 8')

The chemical structures of the compounds 1' - 8' are shown in Table 13 and Table 14.

**Table 13**

| | |
|---|---|
| 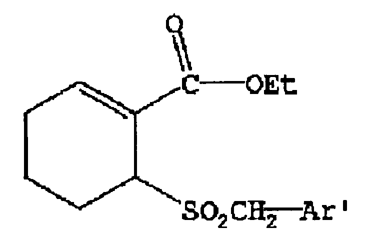 | |

| Compound No. | **Ar'** |
|---|---|
| **1'** | 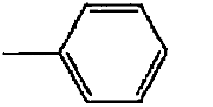 |
| **2'** | 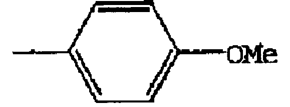 |
| **3'** | 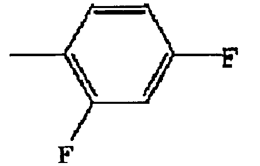 |
| **4'** | 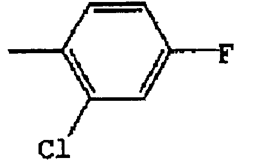 |
| **5'** (-)-form | 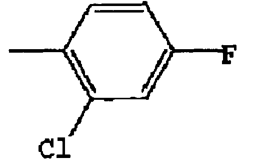 |
| **6'** (+)-form | 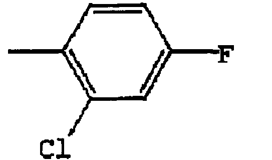 |

**Table 14**

| | |
|---|---|
| 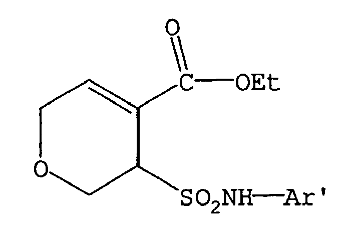 | |

| Compound No. | Ar' |
|---|---|
| 7' | 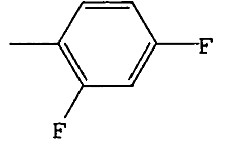 |
| 8' | 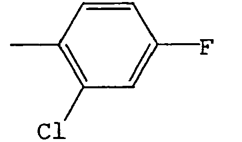 |

### Example 1

Compound 72 (11 g) of Reference Example B66 was dissolved in soybean oil (220 g), and egg-yolk lecithin (13.2 g) and dimyristoylphosphatidylglycerol (2.2 g) were dissolved therein. Glycerol (24.7 g) was dissolved in distilled water, and acetic acid buffer was added and dissolved to a final concentration of acetic acid 11.2 mM, sodium acetate 8.8 mM. They were mixed, crudely emulsified and finely emulsified by a high-pressure homogenizer (RANNIE bench top homogenizer) at a pressure of 8000 psi. The obtained emulsion composition (20 mL) was filled in a 20 mL vial, purged with nitrogen, tightly sealed with a rubber plug and a plastic cap, and subjected to a sterilization treatment in an autoclave at 121°C or above for 15 min to give an emulsion composition having the composition of the above-mentioned formulation 1.

Then, as a control, compound 72 (17 g) was dissolved in soybean oil (340 g), and egg-yolk lecithin (20.4 g) and dimyristoylphosphatidylglycerol (3.4 g) were dissolved therein. Glycerol (38.25 g) was dissolved in distilled water. They were mixed, crudely emulsified and finely emulsified by a high-pressure homogenizer (RANNIE bench top homogenizer) at a pressure of 8000 psi. The obtained emulsion composition (20 mL) was filled in a 20 mL vial, purged with nitrogen, tightly sealed with a rubber plug and a plastic cap, and subjected to a sterilization treatment in an autoclave at 121°C or above for 15 min to give an emulsion composition having the composition of the above-mentioned control formulation 2. The respective formulations are shown in Table 15.

**Table 15**

| | formulation 1 | control formulation 2 |
|---|---|---|
| compound 72 | 11 g | 17 g |
| soybean oil | 220 g | 340 g |
| egg-yolk lecithin | 13.2 g | 20.4 g |
| glycerol | 24.7 g | 38.25 g |
| dimyristoylphosphatidylglycerol | 2.2 g | 3.4 g |
| acetic acid | 11.2 mM | - |
| sodium acetate·anhydride | 8.8 mM | - |
| distilled water total amount | 1.1 L | 1.7 L |

### Example 2

Emulsion compositions of Formulation 1 and control formulation 2 obtained in Example 1 were preserved at 25°C, and the property, pH and average particle size were measured over time. The particle size was measured by Malvern Mastersizer S. The results are shown in Table 16.

**Table 16**

| | | property | pH | average particle size (µm) |
|---|---|---|---|---|
| formulation 1 | before sterilization | Adequate | 4.4 | 0.3 |
| | Initial | Adequate | 4.4 | 0.3 |
| | 25°C, 3 months | Adequate | 4.4 | 0.3 |
| | 25°C, 6 months | Adequate | 4.5 | 0.3 |
| control formulation 2 | before sterilization | Adequate | 4.6 | 0.3 |
| | Initial | Adequate | 4.0 | 0.3 |
| | 25°C, 3 months | Adequate | 3.6 | 0.3 |
| | 25°C, 6 months | inadequate¹⁾ | 3.6 | 0.8 |

| | | | | |
|---|---|---|---|---|
| 1) Dispersion phase particles and water wherein the dispersion phase particles are dispersed showed phase separation. | | | | |

In formulation 1 containing acetate buffer, pH did not change before and after sterilization, and hardly changed even by a long-term preservation at 25°C, and formulation 1 was stable in the property, pH and average particle size. In control formulation 2, pH decreased from 4.6 to 4.0 before and after sterilization, and further decreased to 3.6 by preservation at 25°C for 3 months. Due to the decrease in pH, the emulsion composition became unstable, and dispersion phase particles and water wherein the dispersion phase particles are dispersed showed phase separation by preservation at 25°C for 6 months (inadequate property), and the average particle size increased to 0.8 µm. Thus, by the addition of acetate buffer to an emulsion composition containing compound 72, pH decrease during sterilization and pH decrease during long-term preservation could be improved.

### Example 3

Compound 72 (600 g) was dissolved in soybean oil (12 kg), and purified egg-yolk lecithin (720 g) and dimyristoylphosphatidylglycerol (120 g) were dissolved therein at 50 - 60°C. Glycerol (1350 g) was dissolved in distilled water (20 kg), and glacial acetic acid (40.35 g) and sodium acetate·3 hydrate (71.85 g) were added and dissolved at 50 - 60°C. They were mixed and crudely emulsified by homogenizer Polytron (ULTRA TURRAX) for 5 min. Then, the emulsion was finely emulsified by high-pressure homogenizer MicronLab40 (APV Gaulin) at a pressure of 8000 psi, 8 passes. Compound 72 was dissolved and emulsified under a nitrogen stream. The obtained emulsion composition was filtered through a membrane filter having a pore size of 4.5 µm. The obtained emulsion composition (20 mL) was filled in a 20 mL vial, purged with nitrogen, tightly sealed with a rubber plug and a plastic cap, and subjected to a sterilization treatment in an autoclave at 121°C or above for 15 min to give an emulsion composition having the composition of the above-mentioned formulation 3.

Then, as a control, compound 72 (600 g) was dissolved in soybean oil (12 kg), and egg-yolk lecithin (720 g) and dimyristoylphosphatidylglycerol (120 g) were dissolved therein at 50 - 60°C. Glycerol (1350 g) was dissolved in distilled water (35 kg) and mixed to dissolve the mixture at 50 - 60°C. They were mixed, and crudely emulsified by homogenizer Polytron (ULTRA TURRAX) for 5 min, and crudely emulsified.
Then, the emulsion was finely emulsified by high-pressure homogenizer MicronLab40 (APV Gaulin) at a pressure of 8000 psi, 8 passes. Compound 72 was dissolved and emulsified under a nitrogen stream. The obtained emulsion composition was filtered through a membrane filter having a pore size of 5 µm. The obtained emulsion composition (20 mL) was filled in a 20 mL vial, purged with nitrogen, tightly sealed with a rubber plug and a plastic cap, and subjected to a sterilization treatment in an autoclave at 121°C or above for 15 min to give an emulsion composition having the composition of the above-mentioned control formulation 4. Respective formulations are shown in Table 17.

**Table 17**

| | formulation 3 | control formulation 4 |
|---|---|---|
| compound 72 | 600 g | 600 g |
| soybean oil | 12000 g | 12000 g |
| egg-yolk lecithin | 720 g | 720 g |
| glycerol | 1350 g | 1350 g |
| dimyristoylphosphatidylglycerol | 120 g | 120 g |
| glacial acetic acid | 40.35 g | - |
| sodium acetate·3 hydrate | 71.85 g | - |
| distilled water total amount | 60 L | 60 L |

### Example 4

Emulsion compositions of formulation 3 and control formulation 4 obtained in Example 3 were preserved at 25°C, and the property, pH and average particle size were measured over time. The average particle size of formulation 3 was measured by Malvern Mastersizer 2000, and that of control formulation 4 was measured by Malvern Mastersizer S. The results are shown in Table 18.

**Table 18**

| | | property | pH | average particle size (µm) |
|---|---|---|---|---|
| formulation 3 | before emulsification - sterilization | adequate | 4.5 | - |
| | initial | adequate | 4.4 | 0.2 |
| | 25°C, 3 months | adequate | 4.5 | 0.2 |
| | 25°C, 6 months | adequate | 4.5 | 0.2 |
| | 25°C, 15 months | adequate | 4.5 | 0.2 |
| | 25°C, 18 months | adequate | 4.5 | 0.2 |
| | 25°C, 24 months | adequate | 4.5 | 0.2 |
| control formulation 4 | before emulsification - sterilization | adequate | 5.2 | - |
| | initial | adequate | 4.0 | 0.3 |
| | 25°C, 3 months | adequate | 3.5 | 0.3 |
| | 25°C, 6 months | inadequate¹⁾ | 3.5 | 3.6 |

| | | | | |
|---|---|---|---|---|
| 1) visibly observed free oil drop was observed. | | | | |

In formulation 3 containing acetate buffer, pH was constant before and after emulsification-sterilization, and pH did not decrease even by a long-term preservation at 25°C for 18 months, and formulation 3 was highly stable with no change in the property and average particle size.
In control formulation 4, pH decreased from after preservation at 25°C for 3 months. After preservation for 6 months, free oil drop was observed on the surface of the emulsion composition (inadequate property), and the average particle size increased to 3.6 µm. Thus, by the addition of 20 mM acetate buffer to an emulsion composition containing compound 72, emulsion composition free of pH decrease during preparation, sterilization and long-term preservation could be prepared. Thus, preparation of a markedly stable emulsion composition became possible.

### Example 5

To prepare formulations (total amount 25 mL) having the same ratio as in control formulation 2 and containing various buffers, various concentrations of buffer was added and dissolved in an aqueous phase comprising glycerol and distilled water. As the composition of each buffer, acetate buffer contains acetic acid and sodium acetate, lactate buffer contains lactic acid and sodium lactate, citrate buffer contains citric acid and sodium citrate, phosphate-citrate buffer contains disodium hydrogenphosphate and citric acid, phosphate buffer contains sodium dihydrogenphosphate and disodium hydrogenphosphate, carbonate buffer contains sodium carbonate and sodium hydrogen carbonate, and their concentrations are as described in Tables 19-22. Then, compound 72, egg-yolk lecithin and dimyristoylphosphatidylglycerol were dissolved in soybean oil. They were mixed and crudely emulsified by a homogenizer (Ultraturrax T25 S1-Janke & Kunkel blade homogenizer), and an emulsion composition was prepared by a sonicator (Vibracell Ultrasonicator). The obtained emulsion composition was filtered through a membrane filter having a pore size of 5 µm. The obtained emulsion composition (20 mL) was filled in a 20 mL vial, purged with nitrogen, tightly sealed with a rubber plug and a plastic cap, and subjected to a sterilization treatment in an autoclave at 121°C or above for 15 min to give emulsion compositions containing various buffers. The pH of the emulsion compositions before and after autoclave treatment was examined.

The results are shown in Tables 19 - 22. Emulsion compositions containing compound 72 after addition of acetate buffer, lactate buffer, citrate buffer and phosphate - citrate buffer at 5 mM to 32 mM afforded emulsion compositions of pH 4 - 5 which hardly show pH change even by a high-pressure vapor sterilization treatment.
When the pH of an emulsion composition was adjusted to 6 with a buffer, pH decreased markedly by a high-pressure vapor sterilization treatment, even when phosphate buffer, carbonate buffer or citrate buffer was used. With carbonate buffer, pH changed to about 7 before high-pressure vapor sterilization.

**Table 19**

| pH of emulsion composition | kind of buffer | buffer concentration | high-pressure vapor sterilization | pH |
|---|---|---|---|---|
| 4 | acetate buffer | 5 mM | before treatment | 4.1 |
| | | | after treatment | 4.1 |
| | | 10 mM | before treatment | 4.0 |
| | | | after treatment | 4.0 |
| | | 15 mM | before treatment | 4.0 |
| | | | after treatment | 4.0 |
| | | 20 mM | before treatment | 4.0 |
| | | | after treatment | 4.0 |
| | lactate buffer | 5 mM | before treatment | 4.2 |
| | | | after treatment | 4.1 |
| | | 12.5 mM | before treatment | 4.2 |
| | | | after treatment | 4.1 |
| | | 25 mM | before treatment | 4.2 |
| | | | after treatment | 4.1 |
| | citrate buffer | 5 mM | before treatment | 4.1 |
| | | | after treatment | 4.1 |
| | | 10 mM | before treatment | 4.0 |
| | | | after treatment | 4.1 |
| | | 15 mM | before treatment | 4.0 |
| | | | after treatment | 4.1 |
| | | 20 mM | before treatment | 4.1 |
| | | | after treatment | 4.2 |

**Table 20**

| pH of emulsion composition | kind of buffer | buffer concentration | high-pressure vapor sterilization | pH |
|---|---|---|---|---|
| 4.5 | acetate buffer | 5 mM | before treatment after treatment | 4.6 4.5 |
| | | 10 mM | before treatment after treatment | 4.6 4.4 |
| | | 15 mM | before treatment after treatment | 4.5 4.4 |
| | | 20 mM | before treatment after treatment | 4.5 4.4 |
| | lactate buffer | 5 mM | before treatment after treatment | 4.5 4.4 |
| | | 12.5 mM | before treatment after treatment | 4.5 4.4 |
| | | 25 mM | before treatment after treatment | 4.5 4.4 |
| | phosphate - citrate buffer | 8 mM | before treatment after treatment | 4.7 4.3 |
| | | 16 mM | before treatment after treatment | 4.6 4.3 |
| | | 32 mM | before treatment after treatment | 4.6 4.3 |
| | citrate buffer | 5 mM | before treatment after treatment | 4.6 4.7 |
| | | 10 mM | before treatment after treatment | 4.6 4.7 |
| | | 15 mM | before treatment after treatment | 4.5 4.7 |
| | | 20 mM | before treatment after treatment | 4.5 4.7 |

**Table 21**

| pH of emulsion composition | kind of buffer | buffer concentration | high-pressure vapor sterilization | pH |
|---|---|---|---|---|
| 5 | acetate buffer | 5 mM | before treatment after treatment | 5.0 4.8 |
| | | 10 mM | before treatment after treatment | 5.0 4.8 |
| | | 15 mM | before treatment after treatment | 4.9 4.8 |
| | | 20 mM | before treatment after treatment | 5.0 4.9 |
| | citrate buffer | 5 mM | before treatment after treatment | 5.1 5.1 |
| | | 10 mM | before treatment after treatment | 5.1 5.2 |
| | | 15 mM | before treatment after treatment | 5.1 5.1 |
| | | 20 mM | before treatment after treatment | 5.0 5.0 |

**Table 22**

| pH of emulsion composition | kind of buffer | buffer concentration | high-pressure vapor sterilization | pH |
|---|---|---|---|---|
| 6 | citrate buffer | 5 mM | before treatment after treatment | 6.3 5.6 |
| | | 10 mM | before treatment after treatment | 6.3 5.7 |
| | | 15 mM | before treatment after treatment | 6.3 5.7 |
| | | 20 mM | before treatment after treatment | 6.3 5.6 |
| | phosphate buffer | 5 mM | before treatment after treatment | 6.1 4.6 |
| | | 10 mM | before treatment after treatment | 6.1 4.7 |
| | | 15 mM | before treatment after treatment | 6.0 5.0 |
| | | 20 mM | before treatment after treatment | 6.0 5.3 |
| | carbonate buffer | 5 mM | before treatment after treatment | 7.0 4.3 |
| | | 10 mM | before treatment after treatment | 7.0 4.3 |
| | | 15 mM | before treatment after treatment | 7.0 5.0 |
| | | 20 mM | before treatment after treatment | 7.1 5.6 |

### Comparative Example 1

To prepare formulations (total amount 25 mL) having the same ratio as in control formulation 2 and containing various concentrations of sodium hydroxide as a pH adjuster, sodium hydroxide was dissolved in an aqueous phase comprising glycerol and distilled water to final concentrations of 0, 0.5, 0.75, 1, 1.5 and 2.0 mM. Then, compound 72, egg-yolk lecithin and dimyristoylphosphatidylglycerol were dissolved in soybean oil. They were mixed and crudely emulsified by a homogenizer (Ultraturrax T25 S1-Janke & Kunkel blade homogenizer), and an emulsion composition was prepared by a sonicator (Vibracell Ultrasonicator). The obtained emulsion composition was filtered through a membrane filter having a pore size of 5 µm. The obtained emulsion composition (20 mL) was filled in a 20 mL vial, purged with nitrogen, tightly sealed with a rubber plug and a plastic cap, and subjected to a sterilization treatment in an autoclave at 121°C or above for 15 min to give emulsion compositions containing various concentrations of sodium hydroxide as a pH adjuster. The pH of the emulsion compositions before and after autoclave treatment was examined.
The results are shown in Table 23. The emulsion compositions containing compound 72, including those free of sodium hydroxide, showed remarkable pH decrease after sterilization when any concentration of sodium hydroxide was added.

**Table 23**

| sodium hydroxide concentration | high-pressure vapor sterilization | Ph |
|---|---|---|
| 0 mM | before treatment | 5.4 |
| | after treatment | 4.6 |
| 0.5 mM | before treatment | 6.5 |
| | after treatment | 4.5 |
| 0.75 mM | before treatment | 6.7 |
| | after treatment | 4.5 |
| 1 mM | before treatment | 6.7 |
| | after treatment | 4.5 |
| 1.5 mM | before treatment | 6.7 |
| | after treatment | 4.6 |
| 2.0 mM | before treatment | 6.9 |
| | after treatment | 4.7 |

### Experimental Example 1 Suppressive action on expression caused by gene induction by LPS stimulation

A suppressive effect of test compound 1 (compound 72 of Reference Example B66) on the production of various mediators by lipopolysaccharide (LPS), which is a TLR4 agonist, in mouse macrophage cell line RAW264.7 cells was examined. The day before the experiment, the cells were suspended to 5x10⁵ cells/mL in RPMI1640 medium supplemented with 10% inactivated fetal bovine serum, plated on a plate and incubated at 37°C, 5% CO₂/95% air overnight. The medium was changed to RPMI1640 medium supplemented with 1% inactivated fetal bovine serum, and test compound 1 (final concentration 100 nM) and LPS (final concentration 10 ng/mL) were added. After culture for 6 hr, RNA was prepared by a conventional method and gene was analyzed using DNA Chip (Mu73A, Affimetrix) and Gene Spring ver.4. The test substance was dissolved in N,N-dimethylformamide to 10 mM and diluted with a medium. The results of a representative example of the analyzed gene are shown in Table 24. Test compound 1 suppressed almost all the gene variation analyzed for the genes that showed variation by LPS stimulation.

**Table 24-1**

| | expression of mRNA (fluorescence intensity) | | |
|---|---|---|---|
| gene | control | LPS | LPS+ test compound 1 |
| A20 | 143.9 | 1044.3 | 204.0 |
| Adenosine A2b receptor | 2.7 | 2092.2 | 141.9 |
| argininosuccinate synthetase | 376.7 | 8623.2 | 478.9 |
| bcl-3 | 0 | 7512.6 | 158.7 |
| Caspase-11 | 436.0 | 6975.7 | 763.5 |
| CCR1 | 26.5 | 1877.4 | 137.9 |
| COX-2 | 24.4 | 13981.9 | 71.9 |
| Cytokine inducible SH2-containing protein | 0 | 22669.3 | 2092.5 |
| Fas antigen | 132.7 | 2507.5 | 202.7 |
| Fc receptor | 1244.4 | 20683.4 | 982.5 |
| Galectin-9 | 1904.3 | 19639.3 | 3639.2 |
| G-CSF | 698.4 | 18440.5 | 844.9 |
| Guanylate binding protein-2 | 0 | 8931.2 | 19.4 |
| Histidine decarboxylase | 44.3 | 4472.2 | 21.1 |
| IL-1 beta | 0 | 18712.2 | 424.9 |
| IL-1 receptor antagonist | 156.9 | 21942.3 | 538.2 |
| IL-10 | 221.8 | 6617.9 | 128.1 |
| IL-18 | 708.7 | 4881.4 | 429.4 |
| IL-4 receptor | 84.4 | 7015.8 | 160.6 |

**Table 24-2**

| | expression of mRNA (fluorescence intensity) | | |
|---|---|---|---|
| gene | control | LPS | LPS+ test compound 1 |
| IL-6 | 26.7 | 5345.6 | 0 |
| inducible nitric oxide synthase | 9.3 | 1251.6 | 125.9 |
| Interferon regulatory factor | 1491.7 | 7554.9 | 1356.3 |
| IP-10 | 40.1 | 12954.7 | 30.7 |
| Janus kinase 2 | 228.3 | 2175.1 | 310.9 |
| Major histocompatibility complex | 378.5 | 3399.6 | 353.0 |
| MCP-1 | 115.6 | 24184.7 | 336.8 |
| MCP-3 | 2.4 | 3159.0 | 46.5 |
| MIP-2 | 819.2 | 12542.9 | 3055.0 |
| MMP-9 | 0 | 3571.5 | 352.6 |
| P2X4 | 193.0 | 1239.3 | 227.0 |
| Phosphatase PAC1 | 653.7 | 12520.7 | 765.9 |
| plasminogen activator inhibitor | 199.1 | 15207.5 | 67.5 |
| RANTES | 0 | 12030.1 | 34.8 |
| serum amyloid A | 393.1 | 24253.2 | 1041.0 |
| TNF receptor | 906.2 | 24864.6 | 3467.0 |
| TNF receptor associated factor | 213.6 | 2457.3 | 311.4 |

### Experimental Example 2 Suppressive action on production of mediator from LPS stimulated macrophage

A suppressive effect of test compound 1 (compound 72 of Reference Example B66) on the production of various mediators by lipopolysaccharide (LPS), which is a TLR4 agonist, using mouse macrophage cell line RAW264.7 cells was examined. The day before the experiment, the cells were suspended to 5x10⁵ cells/mL in RPMI1640 medium supplemented with 10% inactivated fetal bovine serum, plated on a 96 well microplate at 0.2 mL/well and incubated at 37°C, 5% CO₂/95% air overnight. The medium was changed to RPMI1640 medium supplemented with 1% inactivated fetal bovine serum, and test compound 1 and LPS (final concentration 10 ng/ml) was added. After overnight culture, cytokine, chemokine, prostaglandin E2 in the culture supernatant was quantified by ELISA, and nitrite ion (stable metabolite of NO) concentration was quantified by a fluorescence method using 2,3-diaminonaphthalene. Test compound 1 was dissolved in N,N-dimethylformamide to 10 mM, diluted with RPMI1640 medium supplemented with 1% inactivated fetal bovine serum to 0.1 mM, further diluted with the medium to 10-fold concentration of the final concentration and 1/10 thereof was added. The results are shown in Table 25. Test compound 1 suppressed almost all the examined mediator production at an equivalent IC₅₀.

**Table 25**

| | IC₅₀ (nmol/L) |
|---|---|
| NO | 1.8 |
| TNF-α | 3.1 |
| IL-1α | 1.1 |
| IL-6 | 2.0 |
| IL-10 | 1.6 |
| MIP-2 | 7.1 |
| PGE₂ | 4.0 |

### Experimental Example 3 Suppressive action on production of mediator from human peripheral blood mononuclear cell

Peripheral blood mononuclear cells were separated from three healthy subjects by a conventional method, suspended in RPMI1640 medium supplemented with 10% inactivated fetal bovine serum, and plated on a 96 well microplate at 8x10⁴ cells/well. Test compound 2 (compound 8' of Reference Example D7), LPS (final concentration 10 ng/mL) and INFα (final concentration 1U/mL) were added, cultured overnight, and TNF-α, IL-6 and IL-8 in the culture supernatant was quantified using a commercially available ELISA kit. Test substance was dissolved in N,N-dimethylformamide to 10 mM, diluted with the medium to 10-fold concentration of the final concentration and 1/10 thereof was added. The results are shown in Table 26. Test compound 2 suppressed production of TNFα, IL-6 and IL-8 at an equivalent IC₅₀. In addition, no difference in the suppressive activity was found in the peripheral blood mononuclear cells of the three subjects.

**Table 26**

| | IC₅₀ (nmol/L) | | |
|---|---|---|---|
| | A | B | C |
| TNF-α | 43 | 39 | 32 |
| IL-6 | 36 | 51 | 35 |
| IL-8 | 83 | 130 | 81 |

### Experimental Example 4 Suppressive action on production of cytokine from LPS stimulated macrophage

A suppressive effect of test compound 1 (compound 72 of Reference Example B66) on the production of various cytokines by lipopolysaccharide (LPS), which is a TLR4 agonist, using mouse macrophage cell line RAW264.7 cells was examined. The day before the experiment, the cells were suspended to 1x10⁶ cells/mL in RPMI1640 medium supplemented with 10% inactivated fetal bovine serum, plated on a 96 well microplate at 0.1 mL/well and incubated at 37°C, 5% CO₂/95% air overnight. The medium was changed, and test compound 1 (final concentration 100 nM) and LPS (final concentration 10 ng/mL) were added. After overnight culture, the content of cytokine in the culture supernatant was quantified by BioPlex cytokine assay (Bio-Rad). Test compound 1 was dissolved in dimethyl sulfoxide (DMSO) to 10 mM, diluted with RPMI1640 medium supplemented with 1% inactivated fetal bovine serum to 0.1 mM, further diluted with the medium to 10-fold concentration of the final concentration and 1/10 thereof was added. The results are shown in Table 27. Test compound 1 suppressed production of all cytokines that showed increased expression by LPS addition.

**Table 27**

| expression amount (pg/mL) of cytokines | | | |
|---|---|---|---|
| Cytokines | control | LPS | LPS+test compound 1 |
| ILlbeta | 0 | 97 | 2 |
| IL1alpha | 21 | 74 | 47 |
| IL2 | 3 | 20 | 8 |
| IL3 | 3 | 22 | 3 |
| IL6 | 82 | 233 | 152 |
| IL10 | 25 | 80 | 20 |
| IL12(p40) | 3 | 18 | 4 |
| IL12(p70) | 12 | 48 | 13 |
| IL17 | 9 | 50 | 13 |
| KC | 10 | 79 | 22 |
| TNFalpha | 48 | 10483 | 125 |
| GM-CSF | 53 | 135 | 85 |
| IFNgamma | 9 | 31 | 8 |
| RANTES | 31 | 1520 | 48 |

### Experimental Example 5 Inhibitory action on intracellular signal transduction after LPS stimulation

A suppressive effect of test compound 1 (compound 72 of Reference Example B66) on the production of various cytokines by lipopolysaccharide (LPS), which is a TLR4 agonist, using mouse macrophage cell line RAW264.7 cells was examined. The day before the experiment, the cells were suspended to 1x10⁶ cells/mL in RPMI1640 medium supplemented with 10% inactivated fetal bovine serum, plated on a 96 well microplate at 0.1 mL/well and incubated at 37°C, 5% CO₂/95% air overnight. The medium was changed, and test compound 1 and LPS (final concentration 10 ng/mL) were added. After LPS addition, ice-cooled washing buffer (Bio-Rad) was added by 0.1 mL/well at 1, 3, 10, 20, 30 min later, ice-cooled cell lysis buffer (0.075 mL/well) was added, and the mixture was preserved overnight in a freezer at -80°C. After thawing at 4°C, the solution (1.5 mL) in the well was placed in an Eppendorf tube, and centrifuged at 4500 rpm, 10 min, 4°C. The supernatant was recovered, and phosphorylated protein was quantified using Bioplex phosphorylated protein assay (Bio-Rad). Test compound 1 was dissolved in dimethyl sulfoxide (DMSO) to 10 mM, diluted with RPMI1640 medium supplemented with 1% inactivated fetal bovine serum to 0.1 mM, further diluted with the medium to 10-fold concentration of the final concentration and 1/10 thereof was added. The results are shown in Fig. 1. Test compound 1 suppressed phosphorylation of all examined proteins involved in LPS signal transduction.

### Experimental Example 6 Influence on binding of LPS to PBMC (peripheral blood mononuclear cells)

Peripheral blood mononuclear cells were separated by a conventional method, and reacted with test compound 1 (compound 72 of Reference Example B66), anti-human CD14 monoclonal antibody MEM-18 and anti-human CCR5 monoclonal antibody 2D7 (negative control) at 4°C for 30 min. The cells were reacted with fluorescence label LPS (E. coli O55:B5, Alexa Fluor^{(R)} 488-labeled, 50 ng/mL) in the presence of 1% human serum at 37°C for 45 min. The cells (10,000 cells) were washed and the binding of fluorescence label LPS was examined by flow cytometry. The results are shown in Fig. 2. The binding of fluorescence label LPS to PBMC was remarkably suppressed by pretreatment of PBMC with anti-human CD14 monoclonal antibody. In contrast, addition of test compound 1 (10 µM) completely failed to inhibit binding of fluorescence label LPS (Fig. 2).

### Industrial Applicability

A pharmaceutical composition containing the TLR signal inhibitory substance of the present invention has a suppressive action on the production of various cytokines such as IL-8 and the like and inflammatory mediators, a suppressive action on the expression of COX-II and the like, and the like, and is useful as an agent for the prophylaxis or treatment of various diseases such as infectious disease, cardiac disease, autoimmune diseases and the like.
This application is based on a patent application No. 2006-117270 filed in Japan, the contents of which are incorporated in full herein by this reference.
Although the present invention have been presented or described by referring to preferred embodiments of this invention, it will, however, be understood by those of ordinary skill in the art that various modifications may be made to the forms and details without departing from the scope of the invention as set forth in the appended claims. All patents, patent publications and other publications indicated or cited in the Specification are hereby incorporated in their entireties by reference.

## Claims

1. An agent for suppressing the production of at least one factor selected from IL-2, IL-3, IL-8, IL-10, IL-12, IL-17, MIP-2, KC, GM-CSF, IFN-γ and prostaglandin E2, which comprises a TLR signaling inhibitory substance.

2. The agent of claim 1, which suppresses the production of at least one kind of factor selected from IL-2, IL-3 and prostaglandin E2.

3. An agent for suppressing the expression of at least one kind selected from COX-II, IL-10, IL-18, MCP-1/3, MIP-2, RANTES, P2X4, plasminogen activator inhibitor, G-CSF, Fas antigen, TNF receptor, Fc receptor, galectin-9, histidine decarboxylase, IL-1 receptor antagonist and MMP-9, which comprises a TLR signaling inhibitory substance.

4. The agent of claim 3, which suppresses the expression of at least one kind selected from COX-II, IL-18, MCP-1/3, RANTES, P2X4, plasminogen activator inhibitor, G-CSF, Fas antigen, TNF receptor, Fc receptor, galectin-9, histidine decarboxylase, IL-1 receptor antagonist and MMP-9.

5. An inhibitor of at least one kind of phosphorylation enzyme selected from ATF2, JNK, p38MAP kinase, IκBα, ERK1/2, p90RSK, STAT2 and p70 S6 kinase, which comprises a TLR signaling inhibitory substance.

6. The agent of claim 5, which inhibits at least one kind of phosphorylation enzyme selected from ATF2, p90RSK, STAT2 and p70 S6 kinase.

7. The agent of any one of claims 1 to 6, wherein the TLR signaling inhibitory substance is a compound represented by the formula (I): 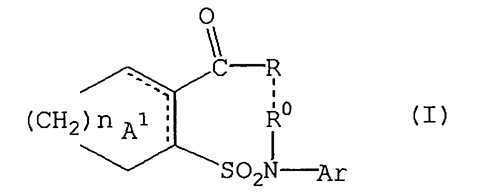 wherein R is an aliphatic hydrocarbon group optionally having substituent(s), an aromatic hydrocarbon group optionally having substituent(s), a heterocyclic group optionally having substituent(s), a group represented by the formula: -OR¹ wherein R¹ is a hydrogen atom or an aliphatic hydrocarbon group optionally having substituent(s), or a group represented by the formula: 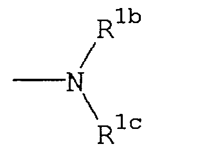 wherein R^{1b} and R^{1c} are the same or different and each is a hydrogen atom or an aliphatic hydrocarbon group optionally having substituent(s),
R0 is a hydrogen atom or an aliphatic hydrocarbon group, or R and R⁰ in combination show a bond,
ring A¹ is a cycloalkene optionally substituted by 1 to 4 substituents selected from the group consisting of (1) an aliphatic hydrocarbon group optionally having substituent(s), (2) an aromatic hydrocarbon group optionally having substituent(s), (3) a group represented by the formula: -OR¹¹ wherein R¹¹ is a hydrogen atom or an aliphatic hydrocarbon group optionally having substituent(s), and (4) a halogen atom, Ar is an aromatic hydrocarbon group optionally having substituent(s),
a group represented by the formula: 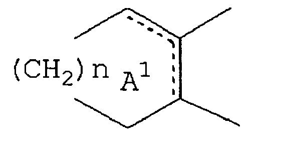 is a group represented by the formula: 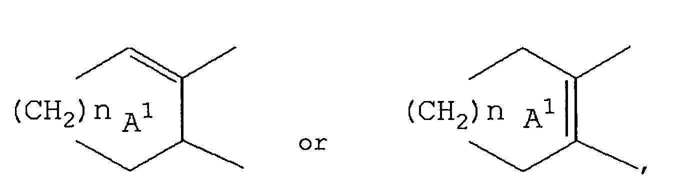 wherein n is an integer of 1 - 4, or a salt thereof or a prodrug thereof, or,
a compound represented by the formula (II): 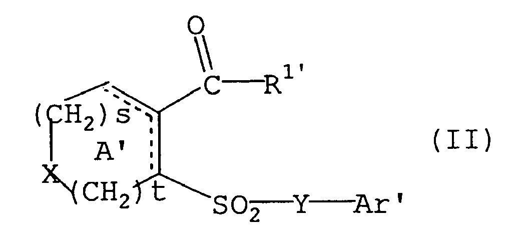 wherein R^{1'} is an aliphatic hydrocarbon group optionally having substituent(s), an aromatic hydrocarbon group optionally having substituent(s), a heterocyclic group optionally having substituent(s), a group represented by the formula: -OR^{1a'} wherein R^{1a'} is a hydrogen atom or an aliphatic hydrocarbon group optionally having substituent(s), or a group represented by the formula: 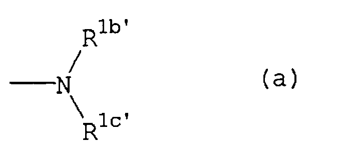 wherein R^{1b'} and R^{1c'} are the same or different and each is a hydrogen atom or an aliphatic hydrocarbon group optionally having substituent(s),
X is a methylene group, NH, a sulfur atom or an oxygen atom, Y is a methylene group optionally having substituent(s) or NH optionally having substituent(s), ring A' is a 5- to 8-membered ring optionally having 1 to 4 substituents selected from the group consisting of (1) an aliphatic hydrocarbon group optionally having substituent(s), (2) an aromatic hydrocarbon group optionally having substituent(s), (3) a group represented by the formula: -OR^{2'} wherein R^{2'} is a hydrogen atom or an aliphatic hydrocarbon group optionally having substituent(s) and (4) a halogen atom,
Ar' is an aromatic hydrocarbon group optionally having substituent(s),
a group represented by the formula: 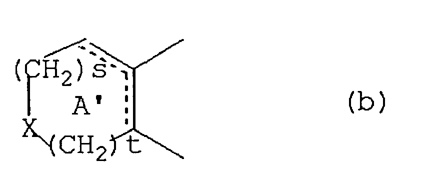 is a group represented by the formula: 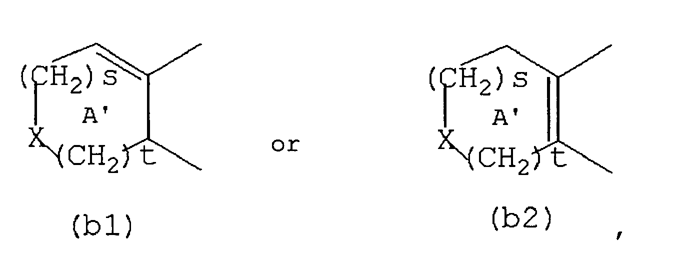 wherein s is an integer of 0 to 2,
t is an integer of 1 to 3,
the total of s and t is not more than 4;
provided that when X is a methylene group, then Y is a methylene group optionally having substituent(s), or a salt thereof or a prodrug thereof.

8. The agent of claim 7, wherein the formula (I) is the formula (Ia): 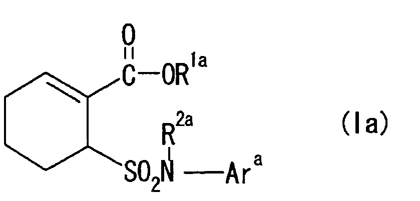 wherein R^{1a} is C₁₋₆ alkyl, R^{2a} is a hydrogen atom or C₁₋₆ alkyl, Ar^{a} is a phenyl group substituted by 1 or 2 halogen atoms, the formula (II) is the formula (IIa): 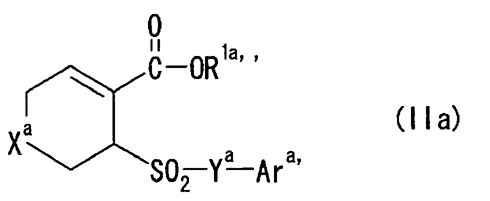 wherein R^{1a''} is C₁₋₆ alkyl, X^{a} is a methylene group or an oxygen atom, Y^{a} is a methylene group or -NH-, Ar^{a'} is a phenyl group optionally having 1 or 2 substituents selected from a halogen atom and a C₁₋₆ alkoxy group.

9. The agent of any one of claims 1 to 6, which is used in combination with at least one kind of drug selected from the group consisting of antibacterial agents, antifungal agents, non-steroidal anti-inflammatory agent, steroid drugs, anticoagulant drugs and antisepsis agents.

10. A method of suppressing the production of at least one kind of factor selected from IL-2, IL-3, IL-8, IL-10, IL-12, IL-17, MIP-2, KC, GM-CSF, IFN-γ and prostaglandin E2, comprising administering an effective amount of a TLR signaling inhibitory substance to a mammal.

11. The method of claim 10, which suppresses at least one kind of factor selected from IL-2, IL-3 and prostaglandin E2.

12. Use of a TLR signaling inhibitory substance for the production of an agent for suppressing the production of at least one kind of factor selected from IL-2, IL-3, IL-8, IL-10, IL-12, IL-17, MIP-2, KC, GM-CSF, IFN-γ and prostaglandin E2.

13. The use of claim 12, wherein the agent suppresses the production of at least one kind of factor selected from IL-2, IL-3 and prostaglandin E2.

14. A method of suppressing the expression of at least one kind selected from COX-II, IL-10, IL-18, MCP-1/3, MIP-2, RANTES, P2X4, plasminogen activator inhibitor, G-CSF, Fas antigen, TNF receptor, Fc receptor, galectin-9, histidine decarboxylase, IL-1 receptor antagonist and MMP-9, comprising administering an effective of a TLR signaling inhibitory substance to a mammal.

15. The method of claim 14, wherein the expression of at least one kind selected from COX-II, IL-18, MCP-1/3, RANTES, P2X4, plasminogen activator inhibitor, G-CSF, Fas antigen, TNF receptor, Fc receptor, galectin-9, histidine decarboxylase, IL-1 receptor antagonist and MMP-9 is suppressed.

16. Use of a TLR signaling inhibitory substance for the production of an agent for suppressing the expression of at least one kind selected from COX-II, IL-10, IL-18, MCP-1/3, MIP-2, RANTES, P2X4, plasminogen activator inhibitor, G-CSF, Fas antigen, TNF receptor, Fc receptor, galectin-9, histidine decarboxylase, IL-1 receptor antagonist and MMP-9.

17. The use of claim 16, wherein the agent suppresses the expression of at least one kind selected from COX-II, IL-18, MCP-1/3, RANTES, P2X4, plasminogen activator inhibitor, G-CSF, Fas antigen, TNF receptor, Fc receptor, galectin-9, histidine decarboxylase, IL-1 receptor antagonist and MMP-9.

18. A method of inhibiting at least one kind of phosphorylation enzyme selected from ATF2, JNK, p38MAP kinase, IκBα, ERK1/2, p90RSK, STAT2 and p70 S6 kinase, comprising administering an effective amount of a TLR signaling inhibitory substance to a mammal.

19. The method of claim 18, wherein at least one kind of phosphorylation enzyme selected from ATF2, p90RSK, STAT2 and p70 S6 kinase is inhibited.

20. Use of a TLR signaling inhibitory substance for the production of an inhibitor of at least one kind of phosphorylation enzyme selected from ATF2, JNK, p38MAP kinase, IκBα, ERK1/2, p90RSK, STAT2 and p70 S6 kinase.

21. The use of claim 20, wherein at least one kind of phosphorylation enzyme selected from ATF2, p90RSK, STAT2 and p70 S6 kinase is inhibited.
